# EUROPEAN PATENT SPECIFICATION

(11) **EP 3 529 246 B1**
(45) Date of publication and mention of the grant of the patent: **18.10.2023**
(21) Application number: 17794827.0
(22) Date of filing: 18.10.2017
(51) Int. Cl.: C07D 413/04, C07D 413/12, C07D 417/04, C07D 271/06, A01N 43/82

(54) **OXADIAZOLES HAVING FUNGICIDAL ACTIVITY**
OXADIAZOLE MIT FUNGIZIDER WIRKUNG
OXADIAZOLES À ACTIVITÉ FONGICIDE

(30) Priority: 24.10.2016 US 201662411912 P
(43) Date of publication of application: 28.08.2019
(73) Proprietor: FMC Corporation, Philadelphia, PA 19104 (US)
(72) Inventor: PASTERIS, Robert James, Newark Delaware 19713 (US); BEREZNAK, James Francis, Newtown Square Pennsylvania 19073 (US); CHITTABOINA, Srinivas, Bibipet Telangana AP 503125 (IN)
(74) Representative: Dehns
(86) International application number: PCT/US2017/057081
(87) International publication number: WO 2018/080859

(56) References cited:
- EP-A1- 3 165 094
- WO-A1-01/98284
- WO-A1-94/05153
- WO-A1-97/15576
- WO-A1-98/56789
- WO-A1-2013/008162
- WO-A1-2017/072247
- WO-A1-2017/111152
- WO-A1-2017/191000
- US-A- 4 871 753

## Description

### FIELD OF THE INVENTION

This invention relates to certain oxadiazoles, their *N*-oxides, salts and compositions, and methods of their use as fungicides.

### BACKGROUND OF THE INVENTION

The control of plant diseases caused by fungal plant pathogens is extremely important in achieving high crop efficiency. Plant disease damage to ornamental, vegetable, field, cereal, and fruit crops can cause significant reduction in productivity and thereby result in increased costs to the consumer. Many products are commercially available for these purposes, but the need continues for new compounds which are more effective, less costly, less toxic, environmentally safer or have different sites of action.

PCT Patent Publication WO 2013/008162 discloses trifluoromethyl-oxadiazole derivatives and their use as pharmaceuticals.

PCT Patent Publications WO 1994/05153, WO 1997/15576 and WO 1998/56789 disclose herbicides including oxadiazoles.

U.S. 4,871,753 discloses 3-phenyl-5-trifluoromethyl-1,2,4-oxadiazoles and their use as fungicides.

### SUMMARY OF THE INVENTION

This invention is defined in the appended claims. Hereinafter are disclosed compounds of Formula 1 (including all stereoisomers), N oxides, and salts thereof, agricultural compositions containing them and their use as fungicides: 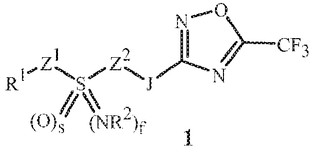 wherein
R¹ is C₁-C₆ alkyl, C₂-C₆ alkenyl, C₂-C₆ alkynyl, C₂-C₅ alkylcarbonyl or C₂-C₅ alkoxycarbonyl, each optionally substituted with up to 3 substituents independently selected from R³; or phenyl, benzyl, phenethyl or naphthalenyl, each optionally substituted with up to 5 substituents independently selected from R^{3a}; or a 3- to 7-membered carbocyclic ring, wherein up to 3 carbon atom ring members are independently selected from C(=O) and C(=S), each ring optionally substituted with up to 5 substituents independently selected from R^{3a}; or
R¹ is a 5- to 6-membered heterocyclic ring, each ring containing ring members selected from carbon atoms and 1 to 4 heteroatoms independently selected from up to 2 O, up to 2 S and up to 4 N atoms, wherein up to 3 carbon atom ring members are independently selected from C(=O) and C(=S), and the sulfur atom ring members are independently selected from S(=O)ᵤ(=NR¹²)ᵥ, each ring optionally substituted with up to 5 substituents independently selected from R^{3a} on carbon atom ring members and R^{3b} on nitrogen atom ring members;
Z¹ is a direct bond, O, NR^{4a} or NR^{4a}NR^{4b};
s and f are each 0, 1 or 2, provided that the sum of s and f is 0, 1 or 2;
each R² is independently H, cyano, C₁-C₃ alkyl or C₁-C₃ haloalkyl;
Z² is a direct bond, O, S, NR⁵ or NR⁵CH₂; or CH₂, CH₂CH₂ or CH₂CH₂CH₂, each optionally substituted with up to 4 substituents independently selected from R⁶ or up to 1 substituent selected from R¹³;
J is phenyl optionally substituted with up to 2 substituents independently selected from R⁷; or a 3- to 7-membered carbocyclic ring, wherein up to 3 carbon atom ring members are independently selected from C(=O) and C(=S), each ring optionally substituted with up to 2 substituents independently selected from R⁷; or
J is a 5- to 6-membered heterocyclic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and S(=O)₂, each ring optionally substituted with up to 2 substituents independently selected from R⁷ on carbon atom ring members and R⁸ on nitrogen atom ring members;
each R³ is independently halogen, hydroxy, cyano, -S-C≡N, -SH, amino, nitro, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₁-C₄ alkylthio, C₁-C₄ alkylsulfinyl, C₁-C₄ alkylsulfonyl, C₁-C₄ haloalkylsulfonyl, C₁-C₄ alkylamino, C₂-C₄ dialkylamino, C₂-C₄ alkylcarbonyl, C₂-C₄ haloalkylcarbonyl, C₂-C₅ alkoxycarbonyl, C₂-C₅ haloalkoxycarbonyl, C₂-C₅ alkylaminocarbonyl or C₃-C₅ dialkylaminocarbonyl; or pyridinyl, pyrimidinyl, pyrazolyl, imidazolyl, triazolyl, thiazolyl, oxazolyl, isoxazolyl, oxetanyl, 1,3-dioxolanyl, tetrahydropyranyl, thienyl, furanyl, pyrrolidinyl, isoxazolinyl, tetrahydrofuranyl, piperidinyl, morpholinyl or piperazinyl, each optionally substituted with up to 3 substituents independently selected from R^{3c} on carbon atom ring members and R^{3d} on nitrogen atom ring members;
each R^{3a} and R^{3c} is independently halogen, hydroxy, cyano, amino, nitro, C₁-C₄ alkyl, C₁-C₄ haloalkyl, C₂-C₄ alkenyl, C₂-C₄ alkynyl, C₁-C₄ hydroxyalkyl, C₃-C₆ cycloalkyl, C₄-C₇ cycloalkylalkyl, C₁-C₄ alkoxy, C₁-C₄ haloalkoxy, C₂-C₄ alkenyloxy, C₂-C₄ alkynyloxy, C₂-C₄ alkoxyalkyl, C₂-C₆ alkylcarbonyloxy, C₁-C₄ alkylthio, C₁-C₄ haloalkylthio, C₂-C₆ alkylcarbonylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfonyloxy, C₁-C₄ alkylamino, C₂-C₈ dialkylamino, C₃-C₆ cycloalkylamino, C₂-C₄ alkylcarbonyl, C₂-C₆ alkoxycarbonyl, C₂-C₆ alkylaminocarbonyl, C₃-C₈ dialkylaminocarbonyl or C₃-C₆ trialkylsilyl;
each R^{3b} and R^{3d} is independently C₁-C₃ alkyl, C₁-C₃ alkoxy, C₂-C₃ alkylcarbonyl or C₂-C₃ alkoxycarbonyl;
R^{4a} and R^{4b} are each independently H, hydroxy, cyano, C₁-C₄ alkyl, C₁-C₄ haloalkyl, C₂-C₄ alkenyl, C₂-C₄ haloalkenyl, C₂-C₄ alkynyl, C₂-C₄ haloalkynyl, C₁-C₄ alkoxy, C₁-C₄ haloalkoxy, C₂-C₄ alkoxyalkyl, C₂-C₄ alkylthioalkyl, C₁-C₄ alkylsulfonyl, C₁-C₄ haloalkylsulfonyl, C₂-C₄ alkylsulfinylalkyl, C₂-C₄ alkylsulfonylalkyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl, C₃-C₅ dialkylaminocarbonyl, C₃-C₇ alkylaminocarbonylalky or C₄-C₇ dialkylaminocarbonylalkyl; or
one pair of R¹ and R^{4a} substituents or one pair of R¹ and R^{4b} substituents are taken together with the atoms to which they are attached to form a 5- to 7-membered ring containing ring members selected from carbon atoms and optionally up to 3 heteroatoms independently selected from up to 1 O, up to 1 S and up to 2 N atoms, wherein up to 3 ring members are independently selected from C(=O), C(=S), S(=O) and S(=O)₂, the ring optionally substituted with up to 4 substituents independently selected from R⁹; or
one pair of R^{3a} and R^{4a} substituents or one pair of R^{3a} and R^{4b} substituents are taken together with the atoms to which they are attached to form a 5- to 7-membered ring containing ring members selected from carbon atoms and optionally up to 3 heteroatoms independently selected from up to 1 O, up to 1 S and up to 2 N atoms, wherein up to 3 ring members are independently selected from C(=O), C(=S), S(=O) and S(=O)₂, the ring optionally substituted with up to 4 substituents independently selected from R¹⁰; or
one pair of R^{3b} and R^{4a} substituents or one pair of R^{3b} and R^{4b} substituents are taken together with the nitrogen atoms to which they are attached to form a 5- to 7-membered ring containing ring members selected from carbon atoms and optionally up to 3 heteroatoms independently selected from up to 1 O, up to 1 S and up to 2 N atoms, wherein up to 3 ring members are independently selected from C(=O), C(=S), S(=O) and S(=O)₂, the ring optionally substituted with up to 4 substituents independently selected from R¹¹;
R⁵ is H, hydroxy, cyano, C₁-C₄ alkyl, C₁-C₄ haloalkyl, C₂-C₄ alkenyl, C₂-C₄ haloalkenyl, C₂-C₄ alkynyl, C₂-C₄ haloalkynyl, C₁-C₄ alkoxy, C₁-C₄ haloalkoxy, C₂-C₄ alkoxyalkyl, C₂-C₄ alkylthioalkyl, C₁-C₄ alkylsulfonyl, C₁-C₄ haloalkylsulfonyl, C₂-C₄ alkylsulfinylalkyl, C₂-C₄ alkylsulfonylalkyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl or C₃-C₅ dialkylaminocarbonyl;
each R⁶ is independently halogen, hydroxy, cyano, nitro, C₁-C₂ alkyl, C₁-C₂ haloalkyl, C₁-C₂ alkoxy or C₁-C₂ haloalkoxy;
each R⁷ is independently halogen, hydroxy, cyano, amino, nitro, C₁-C₄ alkyl, C₁-C₄ haloalkyl, C₂-C₄ alkenyl, C₂-C₄ alkynyl, C₁-C₄ hydroxyalkyl, C₃-C₆ cycloalkyl, C₄-C₇ cycloalkylalkyl, C₁-C₄ alkoxy, C₁-C₄ haloalkoxy, C₂-C₄ alkenyloxy, C₂-C₄ alkynyloxy, C₂-C₄ alkoxyalkyl, C₂-C₄ alkylcarbonyloxy, C₁-C₄ alkylthio, C₁-C₄ haloalkylthio, C₂-C₄ alkylcarbonylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfinyloxy, C₁-C₄ alkylsulfonyloxy, C₁-C₄ alkylamino, C₂-C₆ dialkylamino, C₃-C₆ cycloalkylamino, C₂-C₄ alkylcarbonyl, C₂-C₆ alkoxycarbonyl, C₂-C₆ alkylaminocarbonyl, C₃-C₆ dialkylaminocarbonyl or C₃-C₆ trialkylsilyl;
each R⁸ is independently C₁-C₃ alkyl;
each R⁹, R¹⁰ and R¹¹ is independently halogen, hydroxy, cyano, nitro, C₁-C₄ alkyl, C₁-C₄ haloalkyl, C₂-C₄ alkenyl, C₂-C₄ haloalkenyl, C₂-C₄ alkynyl, C₂-C₄ haloalkynyl, C₁-C₄ alkoxy, C₁-C₄ haloalkoxy, C₂-C₄ alkoxyalkyl, C₂-C₄ alkylthioalkyl, C₁-C₄ alkylsulfonyl, C₁-C₄ haloalkylsulfonyl, C₂-C₄ alkylsulfinylalkyl, C₂-C₄ alkylsulfonylalkyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl or C₃-C₅ dialkylaminocarbonyl;
each R¹² is independently H, cyano, C₁-C₃ alkyl or C₁-C₃ haloalkyl;
each u and v are independently 0, 1 or 2 in each instance of S(=O)ᵤ(=NR¹²)ᵥ, provided that the sum of u and v is 0, 1 or 2;
R¹³ is phenyl optionally substituted with up to 3 substituents independently selected from R¹⁴; and
each R¹⁴ is independently halogen, hydroxy, cyano, nitro, C₁-C₂ alkyl, C₁-C₂ haloalkyl, C₁-C₂ alkoxy or C₁-C₂ haloalkoxy;

More particularly, herein is disclosed a compound of Formula **1** (including all geometric and stereoisomers), tautomers, an *N*-oxide, or a salt thereof.

This invention also relates to a fungicidal composition comprising (a) a compound of Formula **1** according to claim 1; and (b) at least one additional component selected from the group consisting of surfactants, solid diluents and liquid diluents.

This invention also relates to a fungicidal composition comprising (a) a compound of Formula **1** according to claim 1; and (b) at least one other fungicide (e.g., at least one other fungicide having a different site of action).

This invention further relates to a method for controlling plant diseases caused by fungal plant pathogens comprising applying to the plant or portion thereof, or to the plant seed, a fungicidally effective amount of a compound of the invention (e.g., as a composition described herein).

### DETAILS OF THE INVENTION

As used herein, the terms "comprises," "comprising," "includes," "including," "has," "having," "contains," "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components, or elements, in addition to those literally disclosed, provided that these additional materials, steps, features, components, or elements do not materially affect the basic and novel characteristics) of the claimed invention. The term "consisting essentially of" occupies a middle ground between "comprising" and "consisting of".

Where applicants have defined an invention or a portion thereof with an open-ended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As referred to in the present disclosure and claims, "plant" includes members of Kingdom Plantae, particularly seed plants (Spermatopsida), at all life stages, including young plants (e.g., germinating seeds developing into seedlings) and mature, reproductive stages (e.g., plants producing flowers and seeds). Portions of plants include geotropic members typically growing beneath the surface of the growing medium (e.g., soil), such as roots, tubers, bulbs and corms, and also members growing above the growing medium, such as foliage (including stems and leaves), flowers, fruits and seeds.

As referred to herein, the term "seedling", used either alone or in a combination of words means a young plant developing from the embryo of a seed or bud of a vegetative propagation unit such as tuber, corm or rhizome.

In the above recitations, the term "alkyl", used either alone or in compound words such as "alkylthio" or "haloalkyl" includes straight-chain and branched alkyl, such as, methyl, ethyl, n-propyl, i-propyl, and the different butyl, pentyl and hexyl isomers. "Alkenyl" includes straight-chain and branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain and branched alkynes such as ethynyl, 1-propynyl, 2-propynyl, and the different butynyl, pentynyl and hexynyl isomers. "Alkynyl" can also include moieties comprised of multiple triple bonds such as 2,5-hexadiynyl.

"Alkylamino" includes an NH radical substituted with a straight-chain or branched alkyl group. Examples of "alkylamino" include CH₃CH₂NH, CH₃CH₂CH₂NH, and (CH₃)₂CHCH₂NH. Examples of "dialkylamino" include (CH₃)₂N, (CH₃CH₂CH₂)₂N and CH₃CH₂(CH₃)N.

"Alkoxy" includes, for example, methoxy, ethoxy, n-propyloxy, i-propyloxy, and the different butoxy, pentoxy and hexyloxy isomers. "Alkenyloxy" includes straight-chain and branched alkenyl attached to and linked through an oxygen atom Examples of "alkenyloxy" include H₂C=CHCH₂O and CH₃CH=CHCH₂O. "Alkynyloxy" includes straight-chain and branched alkynyloxy moieties. Examples of "alkynyloxy" include HC≡CCH₂O and CH₃C≡CCH₂O. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂OCH₂ and CH₃CH₂OCH₂CH₂.

The term "alkylthio" includes straight-chain and branched alkylthio moieties such as methylthio, ethylthio, and the different propylthio and butylthio isomers. "Alkylsulfinyl" includes both enantiomers of an alkylsulfinyl group. Examples of "alkylsulfinyl" include CH₃S(=O), CH₃CH₂S(=O), CH₃CH₂CH₂S(=O), (CH₃)₂CHS(=O), and the different butylsulfinyl isomers. Examples of "alkylsulfonyl" include CH₃S(=O)₂, CH₃CH₂S(=O)₂, CH₃CH₂CH₂S(=O)₂, (CH₃)₂CHS(=O)₂, and the different butylsulfonyl isomers.

"Alkylthioalkyl" denotes alkylthio substitution on alkyl. Examples of "alkylthioalkyl" include CH₃SCH₂, CH₃SCH₂CH₂, CH₃CH₂SCH₂, CH₃CH₂CH₂SCH₂ and CH₃CH₂SCH₂CH₂; "alkylsulfinylalkyl" and "alkylsulfonylalkyl" include the corresponding sulfoxides and sulfones, respectively.

"Alkylcarbonyl" denotes a straight-chain or branched alkyl group bonded to a C(=O) moiety. Examples of "alkylcarbonyl" include CH₃C(=O), CH₃CH₂CH₂C(=O) and (CH₃)₂CHC(=O). Examples of "alkoxycarbonyl" include CH₃OC(=O), CH₃CH₂OC(=O), CH₃CH₂CH₂OC(=O), (CH₃)₂CHOC(=O), and the different butoxy- and pentoxycarbonyl isomers. Examples of "alkylaminocarbonyl" include CH₃NHC(=O), CH₃CH₂NHC(=O), CH₃CH₂CH₂NHC(=O), (CH₃)₂CHNHC(=O), and the different butylamino- and pentylaminocarbonyl isomers. Examples of "dialkylaminocarbonyl" include (CH₃)₂NC(=O), (CH₃CH₂)₂NC(=O), CH₃CH₂(CH₃)NC(=O) and (CH₃)₂CH(CH₃)NC(=O).

The term "alkylcarbonyloxy" denotes a straight-chain or branched alkyl bonded to a C(=O)O moiety. Examples of "alkylcarbonyloxy" include CH₃CH₂C(=O)O and (CH₃)₂CHC(=O)O. The term "alkylcarbonylalkoxy" denotes alkylcarbonyl bonded to an alkoxy moiety. Examples of "alkylcarbonylalkoxy" include CH₃C(=O)CH₂CH₂O and CH₃CH₂C(=O)CH₂O. Examples of "alkoxycarbonyloxy" include CH₃CH₂CH₂OC(=O)O and (CH₃)₂CHOC(=O)O. "Alkylcarbonylthio" denotes a straight-chain or branched alkylcarbonyl attached to and linked through a sulfur atom. Examples of "alkylcarbonylthio" include CH₃C(=O)S, CH₃CH₂CH₂C(=O)S and (CH₃)₂CHC(=O)S.

The term "alkylaminocarbonylalkyl" denotes a straight-chain or branched alkylaminocarbonyl attached to alkyl. Examples of "alkylaminocarbonylalkyl" include (CH₃)₂CHCH₂NHC(=O)CH₂ and CH₃CH₂NHC(=O)CH₂. Examples of "dialkylaminocarbonylalkyl" include CH₃CH₂CH₂(CH₃)NC(=O)CH₂ and (CH₃)₂NC(=O)CH₂.

"Cycloalkyl" includes, for example, cyclopropyl, cyclobutyl, cyclopentyl and cyclohexyl. The term "cycloalkylalkyl" denotes cycloalkyl substitution on an alkyl moiety. Examples of "cycloalkylalkyl" include cyclopropylmethyl, cyclopentylethyl, and other cycloalkyl moieties bonded to a straight-chain or branched alkyl group.

"Cycloalkylamino" denotes an NH radical substituted with cycloalkyl. Examples of "cycloalkylamino" include cyclopropylamino and cyclohexylamino.

The term "halogen", either alone or in compound words such as "haloalkyl", or when used in descriptions such as "alkyl substituted with halogen" includes fluorine, chlorine, bromine or iodine. Further, when used in compound words such as "haloalkyl", or when used in descriptions such as "alkyl substituted with halogen" said alkyl may be partially or fully substituted with halogen atoms which may be the same or different. Examples of "haloalkyl" or "alkyl substituted with halogen" include F₃C, ClCH₂, CF₃CH₂ and CF₃CCl₂. The terms "haloalkenyl", "haloalkynyl" "haloalkoxy", "haloalkylthio", "haloalkylsulfinyl", "haloalkylsulfonyl", "halocycloalkyl", and the like, are defined analogously to the term "haloalkyl". Examples of "haloalkenyl" include Cl₂C=CHCH₂ and CF₃CH=CHCH₂. Examples of "haloalkynyl" include HC≡CCHCl, CF₃C≡C, CCl₃C≡C and FCH₂C≡CCH₂. Examples of "haloalkoxy" include CF₃O, CCl₃CH₂O, F₂CHCH₂CH₂O and CF₃CH₂O. Examples of "haloalkylthio" include CCl₃S, CF₃S, CCl₃CH₂S and ClCH₂CH₂CH₂S. Examples of "haloalkylsulfinyl" include CF₃S(=O), CCl₃S(=O), CF₃CH₂S(=O) and CF₃CF₂S(=O). Examples of "haloalkylsulfonyl" include CF₃S(=O)₂, CCl₃S(=O)₂, CF₃CH₂S(=O)₂ and CF₃CF₂S(=O)₂. Examples of "halocycloalkyl" include 2-chlorocyclopropyl, 2-fluorocyclobutyl, 3-bromocyclopentyl and 4-chorocyclohexyl. The term "halodialkyl", either alone or in compound words such as "halodialkylamino", means at least one of the two alkyl groups is substituted with at least one halogen atom, and independently each halogenated alkyl group may be partially or fully substituted with halogen atoms which may be the same or different. Examples of "halodialkylamino" include (BrCH₂CH₂)₂N and BrCH₂CH₂(ClCH₂CH₂)N.

"Hydroxyalkyl" denotes an alkyl group substituted with one hydroxy group. Examples of "hydroxyalkyl" include HOCH₂CH₂, CH₃CH₂(OH)CH and HOCH₂CH₂CH₂CH₂.

"Trialkylsilyl" includes 3 branched and/or straight-chain alkyl radicals attached to and linked through a silicon atom, such as trimethylsilyl, triethylsilyl and *tert*-butyldimethylsilyl.

The total number of carbon atoms in a substituent group is indicated by the "Cᵢ-Cⱼ" prefix where i and j are numbers from 1 to 8. For example, C₁-C₄ alkylsulfonyl designates methylsulfonyl through butylsulfonyl; C₂ alkoxyalkyl designates CH₃OCH₂; C₃ alkoxyalkyl designates, for example, CH₃CH(OCH₃), CH₃OCH₂CH₂ or CH₃CH₂OCH₂; and C₄ alkoxyalkyl designates the various isomers of an alkyl group substituted with an alkoxy group containing a total of four carbon atoms, examples including CH₃CH₂CH₂OCH₂ and CH₃CH₂OCH₂CH₂.

The term "unsubstituted" in connection with a group such as a ring or ring system means the group does not have any substituents other than its one or more attachments to the remainder of Formula 1. The term "optionally substituted" means that the number of substituents can be zero. Unless otherwise indicated, optionally substituted groups may be substituted with as many optional substituents as can be accommodated by replacing a hydrogen atom with a non-hydrogen substituent on any available carbon or nitrogen atom Commonly, the number of optional substituents (when present) ranges from 1 to 4. As used herein, the term "optionally substituted" is used interchangeably with the phrase "substituted or unsubstituted" or with the term "(un)substituted."

The number of optional substituents may be restricted by an expressed limitation. For example, in the definition of Z² the phrase "optionally substituted with up to 4 substituents independently selected from R⁶" means that 0, 1, 2, 3 or 4 substituents can be present (if the number of potential connection points allows). Similarly, the phrase "optionally substituted with up to 5 substituents independently selected from R^{3a}" means that 0, 1, 2, 3, 4 or 5 substituents can be present if the number of available connection points allows. When a range specified for the number of substituents (e.g., k being an integer from 1 to 2 in Exhibit A) exceeds the number of positions available for substituents (e.g., 1 position available for (R^{Y})ₖ on U-17 in Exhibit A), the actual higher end of the range is recognized to be the number of available positions.

When a compound is substituted with a substituent bearing a subscript that indicates the number of said substituents can vary (e.g., (R^{Y})ₖ in Exhibit A wherein k is 1 to 2), then said substituents are independently selected from the group of defined substituents, unless otherwise indicated. When a variable group is shown to be optionally attached to a position, for example (R^{Y})ₖ in Exhibit A wherein k may be 0, then hydrogen may be at the position even if not recited in the definition of the variable group. When a position on a group is said to be "not substituted" or "unsubstituted", then hydrogen atoms are attached to take up any free valency.

Naming of substituents in the present disclosure uses recognized terminology providing conciseness in precisely conveying to those skilled in the art the chemical structure. For sake of conciseness, locant descriptors may be omitted.

Unless otherwise indicated, a "ring" or "ring system" as a component of Formula 1 (e.g., substituent J) is carbocyclic or heterocyclic. The term "ring system" denotes two or more connected rings.

The term "ring member" refers to an atom (e.g., C, O, N or S) or other moiety (e.g., C(=O), C(=S) or S(=O)ᵤ(=NR¹²)ᵥ) forming the backbone of a ring or ring system The term "aromatic" indicates that each of ring atom is essentially in the same plane and has a *p-*orbital perpendicular to the ring plane, and that (4n + 2) π electrons, where n is a positive integer, are associated with the ring to comply with Hückel's rule

The term "carbocyclic ring" denotes a ring wherein the atoms forming the ring backbone are selected only from carbon. Unless otherwise indicated, a carbocyclic ring can be a saturated, partially unsaturated, or fully unsaturated ring. When a fully unsaturated carbocyclic ring satisfies Hückel's rule, then said ring is also called an "aromatic ring". "Saturated carbocyclic" refers to a ring having a backbone consisting of carbon atoms linked to one another by single bonds; unless otherwise specified, the remaining carbon valences are occupied by hydrogen atoms.

As used herein, the term "partially unsaturated ring" or "partially unsaturated heterocycle" refers to a ring which contains unsaturated ring atoms and one or more double bonds but which is not aromatic.

The terms "heterocyclic ring" or "heterocycle" denotes a ring wherein at least one of the atoms forming the ring backbone is other than carbon. Unless otherwise indicated, a heterocyclic ring can be a saturated, partially unsaturated, or fully unsaturated ring. When a fully unsaturated heterocyclic ring satisfies Hückel's rule, then said ring is also called a "heteroaromatic ring" or aromatic heterocyclic ring. "Saturated heterocyclic ring" refers to a heterocyclic ring containing only single bonds between ring members.

Unless otherwise indicated, heterocyclic rings and ring systems are attached to the remainder of Formula 1 through any available carbon or nitrogen atom by replacement of a hydrogen on said carbon or nitrogen atom.

As noted above, a pair of R¹ and R^{4a} substituents or a pair of R¹ and R^{4b} substituents may be taken together with the atoms to which they are attached to form an optionally substituted 5- to 7-membered ring. This 5- to 7-membered ring includes as ring members the two atoms to which R¹ and R^{4a} are directly attached or the two atoms to which R¹ and R^{4b} are directly attached. The other ring members (i.e. intervening linking atoms) are selected from carbon atoms and up to 3 heteroatoms independently selected from up to 1 O, up to 1 S and up to 2 N atoms, wherein up to 3 ring members are independently selected from C(=O), C(=S), S(=O) and S(=O)₂. The ring is optionally substituted with up to 4 substituents independently selected from R⁹. The nitrogen atom ring members may be oxidized as *N-*oxides, because compounds relating to Formula 1 also include *N*-oxide derivatives.

Compounds of this invention can exist as one or more stereoisomers. Stereoisomers are isomers of identical constitution but differing in the arrangement of their atoms in space and include enantiomers, diastereomers, *cis-* and *trans*-isomers (also known as geometric isomers) and atropisomers. Atropisomers result from restricted rotation about single bonds where the rotational barrier is high enough to permit isolation of the isomeric species. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. For a comprehensive discussion of all aspects of stereoisomerism, see Ernest L. Eliel and Samuel H. Wilen, Stereochemistry of Organic Compounds, John Wiley & Sons, 1994.

Compounds of this invention can exist as one or more conformational isomers due to restricted rotation about an amide bond (e.g., C(=O)-N) in Formula 1. This invention comprises mixtures of conformational isomers. In addition, this invention includes compounds that are enriched in one conformer relative to others.

This invention comprises all stereoisomers, conformational isomers and mixtures thereof in all proportions as well as isotopic forms such as deuterated compounds.

One skilled in the art will appreciate that not all nitrogen containing heterocycles can form *N*-oxides since the nitrogen requires an available lone pair for oxidation to the oxide; one skilled in the art will recognize those nitrogen-containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R Katritzky and A. J. Boulton, Eds., Academic Press.

One skilled in the art recognizes that because in the environment and under physiological conditions salts of chemical compounds are in equilibrium with their corresponding nonsalt forms, salts share the biological utility of the nonsalt forms. Thus a wide variety of salts of the compounds of Formula **1** are useful for control of plant diseases caused by fungal plant pathogens (i.e. are agriculturally suitable). The salts of the compounds of Formula **1** include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. When a compound of Formula **1** contains an acidic moiety such as a carboxylic acid, salts also include those formed with organic or inorganic bases such as pyridine, triethylamine or ammonia, or amides, hydrides, hydroxides or carbonates of sodium, potassium, lithium, calcium, magnesium or barium. Accordingly, herein are disclosed the *N-*oxides and agriculturally suitable salts of the compounds of formula **1**.

Compounds selected from Formula 1, stereoisomers, *N-*oxides, and salts thereof, typically exist in more than one form, therefore Formula 1 includes all crystalline and non-crystalline forms of the compounds that Formula 1 represents. Non-crystalline forms include embodiments which are solids such as waxes and gums as well as embodiments which are liquids such as solutions and melts. Crystalline forms include embodiments which represent essentially a single crystal type and embodiments which represent a mixture of polymorphs (i.e. different crystalline types). The term "polymorph" refers to a particular crystalline form of a chemical compound that can crystallize in different crystalline forms, these forms having different arrangements and/or conformations of the molecules in the crystal lattice. Although polymorphs can have the same chemical composition, they can also differ in composition due to the presence or absence of co-crystallized water or other molecules, which can be weakly or strongly bound in the lattice. Polymorphs can differ in such chemical, physical and biological properties as crystal shape, density, hardness, color, chemical stability, melting point, hygroscopicity, suspensibility, dissolution rate and biological availability. One skilled in the art will appreciate that a polymorph of a compound represented by Formula **1** can exhibit beneficial effects (e.g., suitability for preparation of useful formulations, improved biological performance) relative to another polymorph or a mixture of polymorphs of the same compound represented by Formula **1.** Preparation and isolation of a particular polymorph of a compound represented by Formula **1** can be achieved by methods known to those skilled in the art including, for example, crystallization using selected solvents and temperatures.

Embodiments as described in the Summary of the Invention include those described below. They do not belong to the invention per se. In the following Embodiments, Formula **1** includes stereoisomers, *N*-oxides and salts thereof, and reference to "a compound of Formula **1"** includes the definitions of substituents specified in the Summary of the Invention unless further defined in the Embodiments.

Embodiment 1. A compound of Formula 1 wherein when R¹ is separate (i.e. not taken together with R^{4a} or R^{4b} to form a ring), then R¹ is C₁-C₆ alkyl, C₂-C₆ alkenyl, C₂-C₆ alkynyl, C₂-C₅ alkylcarbonyl or C₂-C₅ alkoxycarbonyl, each optionally substituted with up to 2 substituents independently selected from R³; or phenyl, benzyl or naphthalenyl, each optionally substituted with up to 4 substituents independently selected from R^{3a}; or a 3- to 7-membered carbocyclic ring, wherein up to 2 carbon atom ring members are independently selected from C(=O) and C(=S), each ring optionally substituted with up to 4 substituents independently selected from R^{3a}; or a 5- to 6-membered heterocyclic ring, each ring containing ring members selected from carbon atoms and 1 to 4 heteroatoms independently selected from up to 2 O, up to 2 S and up to 4 N atoms, wherein up to 2 carbon atom ring members are independently selected from C(=O) and C(=S), each ring optionally substituted with up to 4 substituents independently selected from R^{3a} on carbon atom ring members and R^{3b} on nitrogen atom ring members.

Embodiment 2. A compound of Embodiment 1 wherein R¹ is C₁-C₆ alkyl, C₂-C₆ alkenyl, C₂-C₅ alkylcarbonyl or C₂-C₅ alkoxycarbonyl, each optionally substituted with up to 2 substituents independently selected from R³.

Embodiment 3. A compound of Embodiments 1 or 2 wherein R¹ is C₁-C₆ alkyl, C₂-C₆ alkenyl, C₂-C₅ alkylcarbonyl or C₂-C₅ alkoxycarbonyl, each optionally substituted with up to 1 substituent selected from R³.

Embodiment 4. A compound of any one of Embodiments 1 through 3 wherein R¹ is C₁-C₄ alkyl, C₂-C₄ alkylcarbonyl or C₂-C₄ alkoxycarbonyl, each optionally substituted with up to 1 substituent selected from R³.

Embodiment 5. A compound of Formula **1** or Embodiment 1 wherein when R¹ is separate (i.e. not taken together with R^{4a} or R^{4b} to form a ring), then R¹ is C₁-C₄ alkyl, C₂-C₄ alkenyl, C₂-C₄ alkynyl, C₂-C₅ alkylcarbonyl or C₂-C₄ alkoxycarbonyl, each optionally substituted with up to 2 substituents independently selected from R³; or phenyl optionally substituted with up to 4 substituents independently selected from R^{3a}; or a 3- to 7-membered carbocyclic ring, wherein up to 2 carbon atom ring members are independently selected from C(=O) and C(=S), each ring optionally substituted with up to 4 substituents independently selected from R^{3a}; or a 5- to 6-membered heterocyclic ring, each ring containing ring members selected from carbon atoms and 1 to 4 heteroatoms independently selected from up to 2 O, up to 2 S and up to 4 N atoms, wherein up to 2 carbon atom ring members are independently selected from C(=O) and C(=S), each ring optionally substituted with up to 4 substituents independently selected from R^{3a} on carbon atom ring members and R^{3b} on nitrogen atom ring members.

Embodiment 6. A compound of Formula **1** or Embodiment 5 wherein when R¹ is separate (i.e. not taken together with R^{4a} or R^{4b} to form a ring), then R¹ is phenyl optionally substituted with up to 4 substituents independently selected from R^{3a}; or a 3- to 7-membered carbocyclic ring, wherein up to 2 carbon atom ring members are independently selected from C(=O) and C(=S), each ring optionally substituted with up to 4 substituents independently selected from R^{3a}; or a 5- to 6-membered heterocyclic ring, each ring containing ring members selected from carbon atoms and 1 to 4 heteroatoms independently selected from up to 2 O, up to 2 S and up to 4 N atoms, wherein up to 2 carbon atom ring members are independently selected from C(=O) and C(=S), each ring optionally substituted with up to 4 substituents independently selected from R^{3a} on carbon atom ring members and R^{3b} on nitrogen atom ring members.

Embodiment 7. A compound of Formula **1** or Embodiment 1 wherein when R¹ is separate (i.e. not taken together with R^{4a} or R^{4b} to form a ring), then R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 2 substituents independently selected from R³; or R¹ is selected from U-1 through U-79 as shown in Exhibit A

### Exhibit A

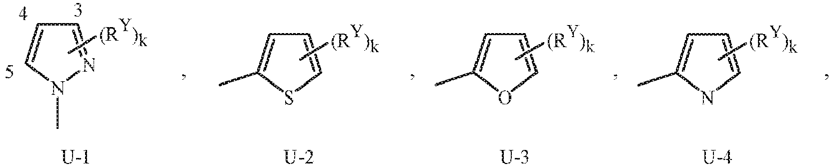

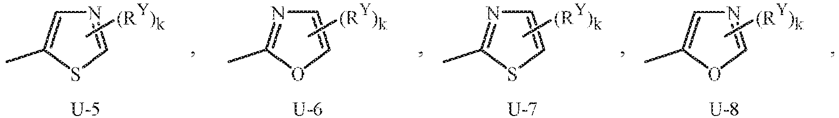

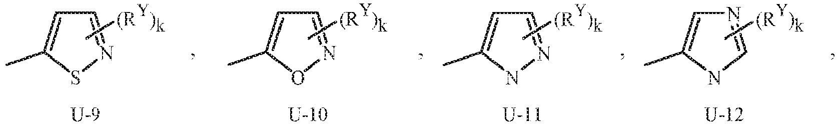

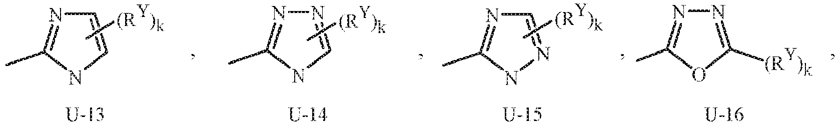

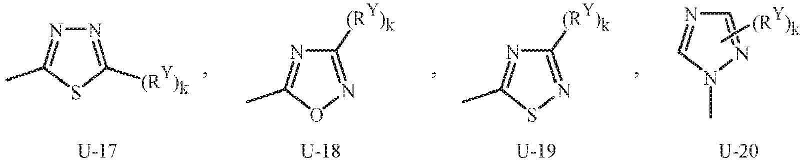

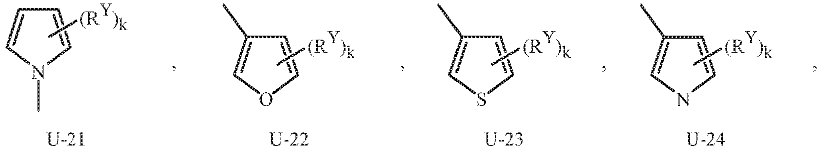

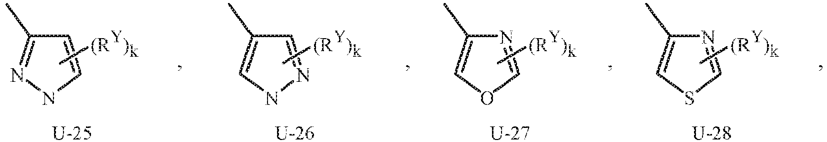

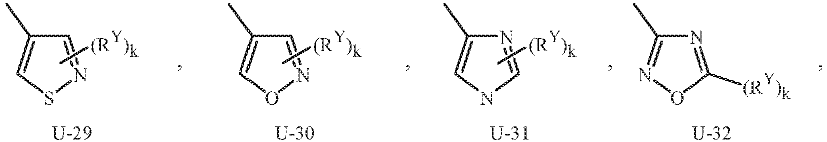

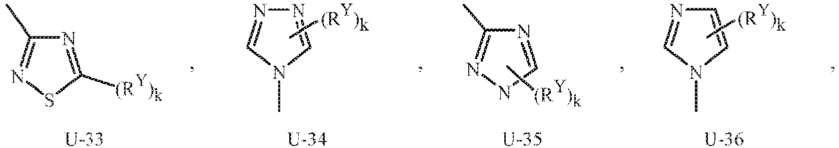

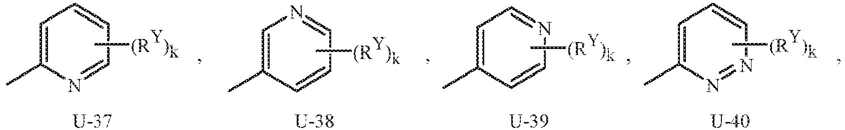

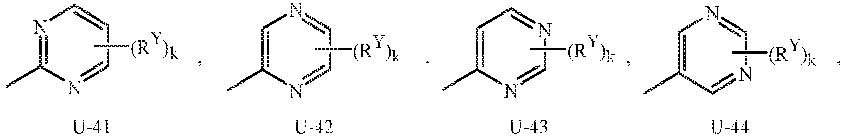

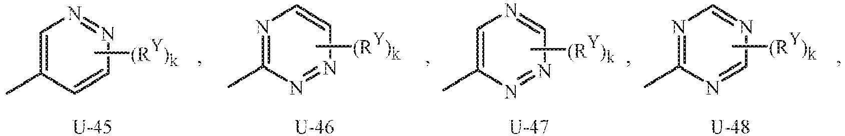

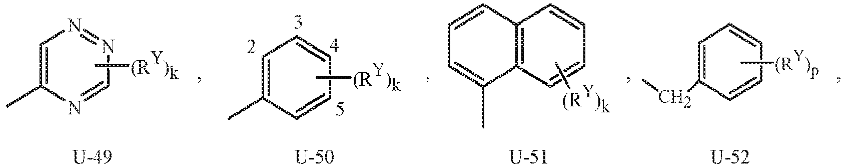

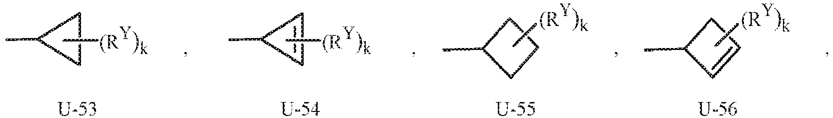

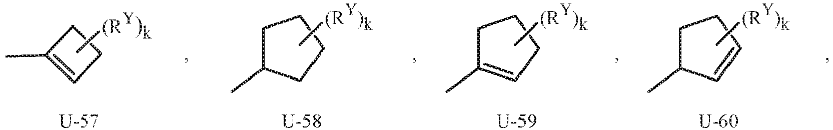

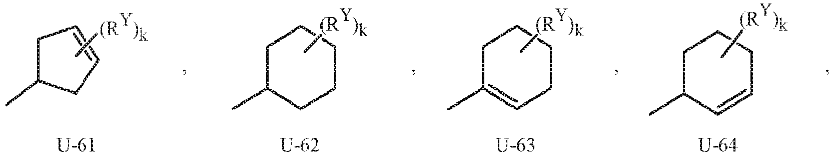

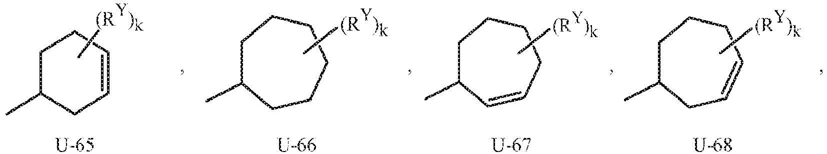

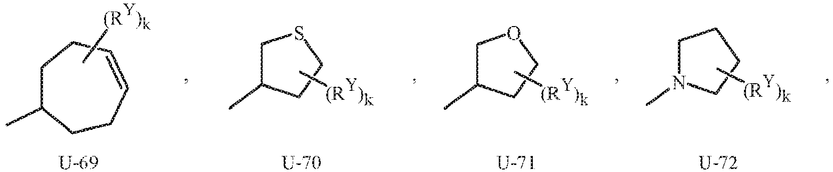

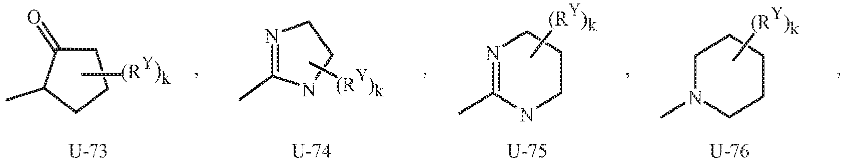

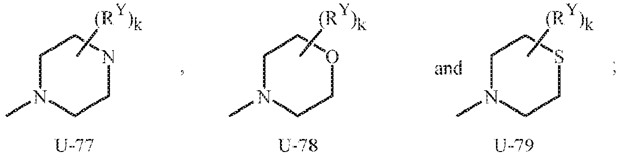

wherein when R^{Y} is attached to a carbon ring member, said R^{Y} is selected from R^{3a}, and when R^{Y} is attached to a nitrogen ring member (e.g., in U-4, U-11 through U-15, U-24 through U-26, U-31, U-35, U-74, U-75 and U-77), said R^{Y} is selected from R^{3b}; and k is 0, 1 or 2.

Embodiment 7a. A compound of Embodiment 7 wherein R¹ is selected from U-1 through U-79.

Embodiment 8. A compound of Embodiment 7 wherein R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 2 substituents independently selected from R³; or U-1 through U-53, U-55, U-58, U-62, U-66 or U-74 through U-79.

Embodiment 8a. A compound of Embodiment 8 wherein R¹ is U-1 through U-53, U-55, U-58, U-62, U-66 or U-74 through U-79.

Embodiment 9. A compound of Embodiment 8 wherein R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 2 substituents independently selected from R³; or U-1, U-2, U-3, U-5, U-20, U-21, U-28, U-36, U-37, U-38, U-39, U-41, U-43, U-50, U-53, U-55, U-58, U-62 or U-74 through U-78.

Embodiment 9a. A compound of Embodiment 9 wherein R¹ is U-1, U-2, U-3, U-5, U-20, U-21, U-28, U-36, U-37, U-38, U-39, U-41, U-43, U-50, U-53, U-55, U-58, U-62 or U-74 through U-78.

Embodiment 10. A compound of Embodiment 9 wherein R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 1 substituent selected from R³; or U-37, U-38, U-39, U-41, U-50, U-53, U-55 or U-58.

Embodiment 10a. A compound of Embodiment 10 wherein R¹ is U-37, U-38, U-39, U-41, U-50, U-53, U-55 or U-58.

Embodiment 11. A compound of Embodiment 10 wherein R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 1 substituent selected from R³; or U-50, U-53 or U-55.

Embodiment 12. A compound of Embodiment 11 wherein R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 1 substituent selected from R³.

Embodiment 13. A compound of Embodiment 11 wherein R¹ is C₁-C₄ alkyl, C₂-C₄ alkenyl or C₂-C₄ alkynyl, each optionally substituted with up to 1 substituent selected from R³; or U-50.

Embodiment 14. A compound of Embodiment 13 wherein R¹ is C₁-C₄ alkyl, C₂-C₄ alkenyl or C₂-C₄ alkynyl, each optionally substituted with up to 1 substituent selected from R³.

Embodiment 15. A compound of Embodiment 13 wherein R¹ is C₁-C₄ alkyl, optionally substituted with up to 1 substituent selected from R³ or U-50.

Embodiment 16. A compound of Embodiment 15 wherein R¹ is C₁-C₄ alkyl optionally substituted with up to 1 substituent selected from R³.

Embodiment 17. A compound of Formula **1** or any one of Embodiments 1 through 15 wherein R¹ is other than phenyl.

Embodiment 18. A compound of Formula **1** or any one of Embodiments 1 through 17 wherein Z¹ is a direct bond, NR^{4a} or NR^{4a}NR^{4b}.

Embodiment 19. A compound of Embodiment 18 wherein Z¹ is a direct bond or NR^{4a}.

Embodiment 20. A compound of Embodiment 19 wherein Z¹ is a direct bond.

Embodiment 21. A compound of Embodiment 19 wherein Z¹ is NR^{4a}.

Embodiment 22. A compound of Formula **1 or** any one of Embodiments 1 through 21 wherein s is 0, 1 or 2 and f is 0 or 1, provided that the sum of s and f is 0, 1 or 2.

Embodiment 23. A compound of Embodiment 22 wherein s is 1 and f is 1.

Embodiment 24. A compound of Embodiment 22 wherein s is 0, 1 or 2 and f is 0.

Embodiment 25. A compound of Embodiment 24 wherein s is 2.

Embodiment 26. A compound of Formula **1** or any one of Embodiments 1 through 25 wherein R² is H, cyano, methyl, ethyl or halomethyl.

Embodiment 27. A compound of Embodiment 26 wherein R² is H or methyl. Embodiment 28. A compound of Embodiment 27 wherein R² is cyano.

Embodiment 29. A compound of Formula **1** or any one of Embodiments 1 through 28 wherein Z² is a direct bond, O, S, NR⁵ or NR⁵CH₂; or CH₂, CH₂CH₂ or CH₂CH₂CH₂, each optionally substituted with up to 4 substituents independently selected from R⁶.

Embodiment 30. A compound of Formula **1** or any one of Embodiments 1 through 29 wherein Z² is CH₂, CH₂CH₂ or CH₂CH₂CH₂, each optionally optionally substituted with up to 4 substituents independently selected from R⁶ or up to 1 substituent selected from R¹³.

Embodiment 31. A compound of Embodiment 30 wherein Z² is CH₂CH₂CH₂ optionally substituted with up to 2 substituents independently selected from R⁶ or up to 1 substituent selected from R¹³.

Embodiment 32. A compound of Embodiment 31 wherein Z² is CH₂CH₂CH₂ optionally substituted with up to 2 substituents independently selected from R⁶.

Embodiment 33. A compound of Embodiment 32 wherein Z² is CH₂CH₂CH₂ optionally substituted with up to 1 substituent selected from R⁶.

Embodiment 34. A compound of Embodiment 33 wherein Z² is CH₂CH₂CH₂.

Embodiment 35. A compound of Embodiment 31 wherein Z² is CH₂CH₂CH₂ optionally with up to 1 substituent selected from R¹³.

Embodiment 36. A compound of Embodiment 35 wherein Z² is CH₂CH₂CH(R¹³).

Embodiment 37. A compound of Embodiment 30 wherein Z² is a direct bond, O, NR⁵ or NR⁵CH₂; or CH₂ or CH₂CH₂, each optionally substituted with up to 4 substituents independently selected from R⁶.

Embodiment 38. A compound of Embodiment 37 wherein Z² is a direct bond, NR⁵ or NR⁵CH₂; or CH₂, optionally substituted with up to 2 substituents independently selected from R⁶.

Embodiment 39. A compound of Embodiment 38 wherein Z² is a direct bond, NR⁵, NR⁵CH₂ or CH₂.

Embodiment 40. A compound of Embodiment 39 wherein Z² is a direct bond.

Embodiment 41. A compound of Embodiment 39 wherein Z² is NR⁵.

Embodiment 42. A compound of Embodiment 39 wherein Z² is NR⁵CH₂.

Embodiment 43. A compound of Embodiment 39 wherein Z² is CH₂.

Embodiment 44. A compound of Embodiment 30 wherein Z² is is CH(R¹³).

Embodiment 45. A compound of Embodiment 30 wherein Z² is is CH₂CH(R¹³).

Embodiment 46. A compound of Formula 1 or any one of Embodiments 1 through 45 wherein J is phenyl optionally substituted with up to 2 substituents independently selected from R⁷; or a 3- to 7-membered carbocyclic ring, wherein up to 2 carbon atom ring members are independently selected from C(=O) and C(=S), each ring optionally substituted with up to 2 substituents independently selected from R⁷; or a 5- to 6-membered heterocyclic ring, each ring containing ring members selected from carbon atoms and 1 to 4 heteroatoms independently selected from up to 2 O, up to 2 S and up to 4 N atoms, wherein up to 2 carbon ring members are independently selected from C(=O) and C(=S), each ring optionally substituted with up to 2 substituents independently selected from R⁷ on carbon atom ring members and R⁸ on nitrogen atom ring members.

Embodiment 47. A compound of Formula **1** or any one of Embodiments 1 through 46 wherein J is selected from J-1 through J-92 as shown in Exhibit B

### Exhibit B

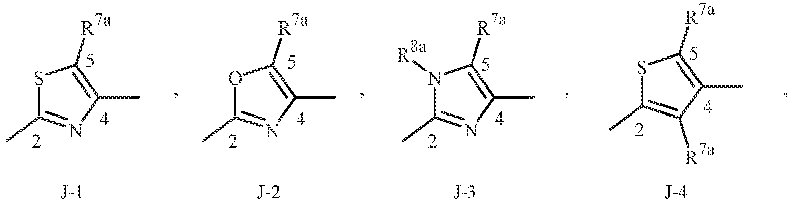

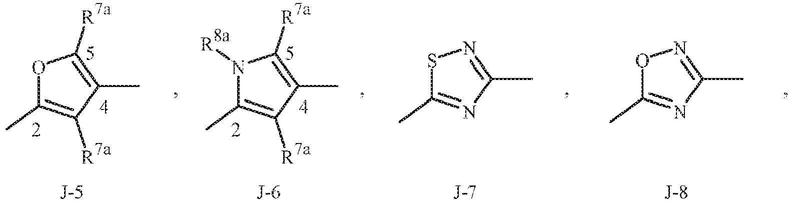

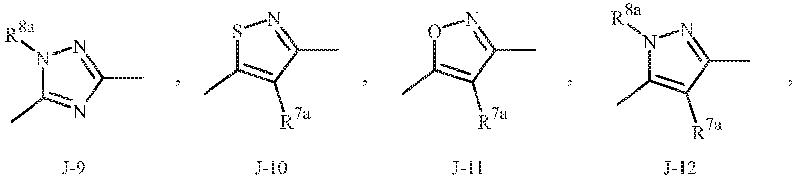

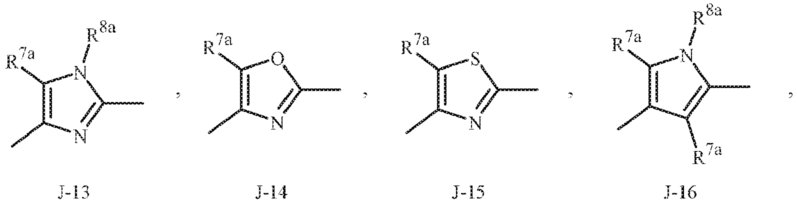

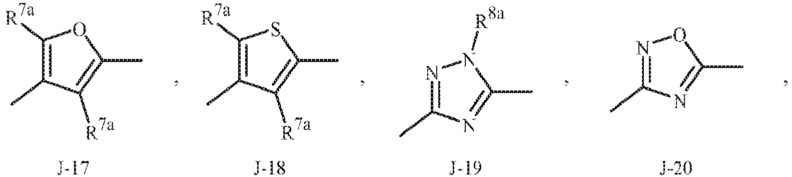

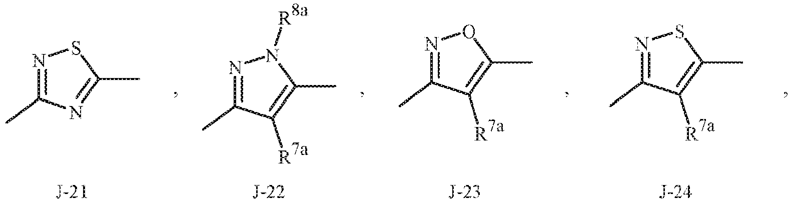

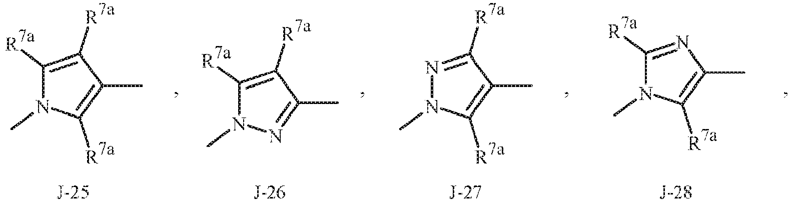

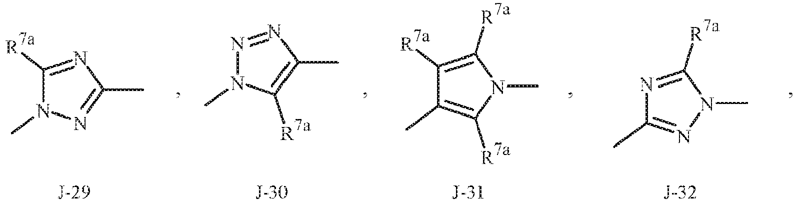

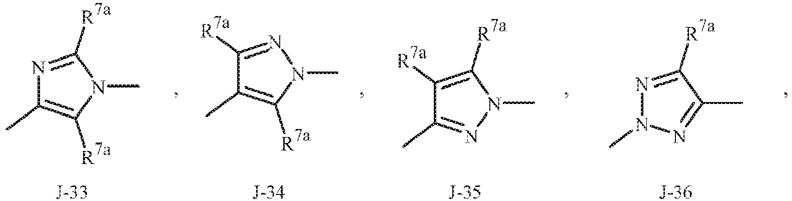

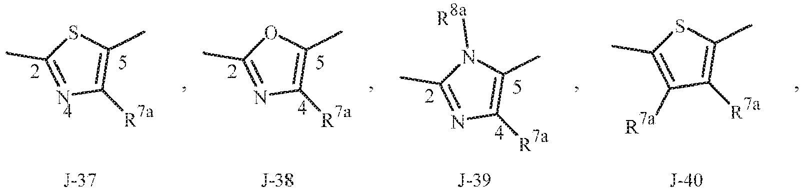

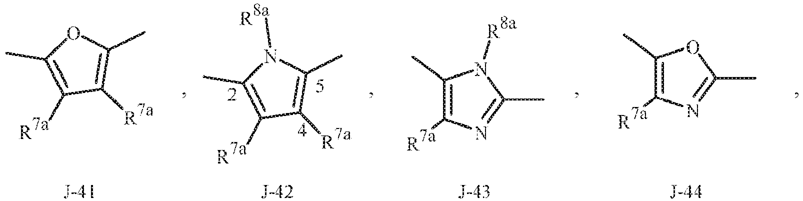

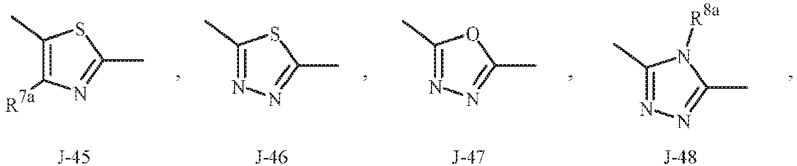

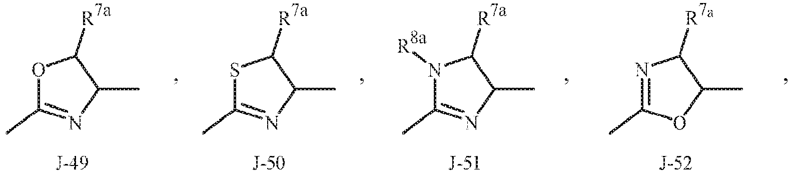

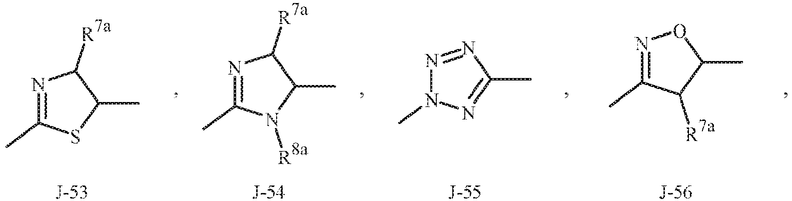

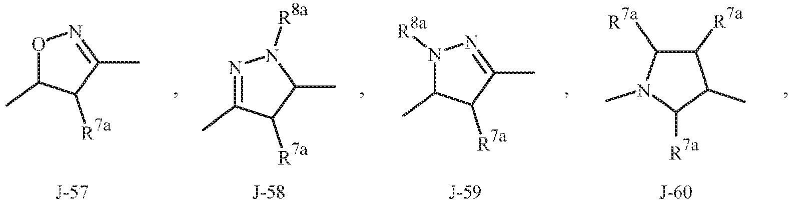

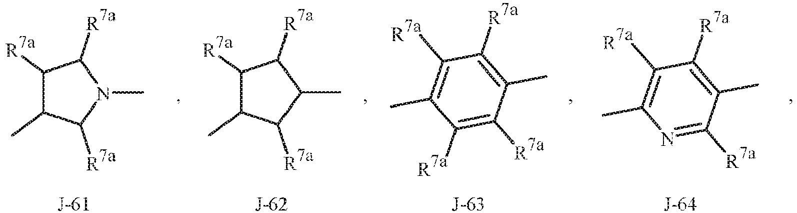

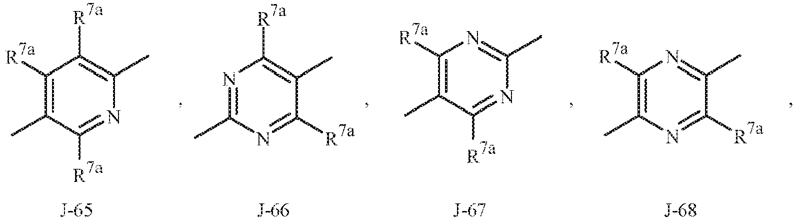

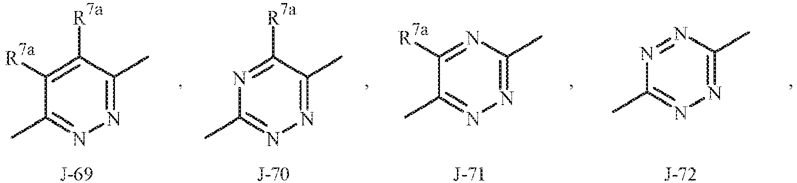

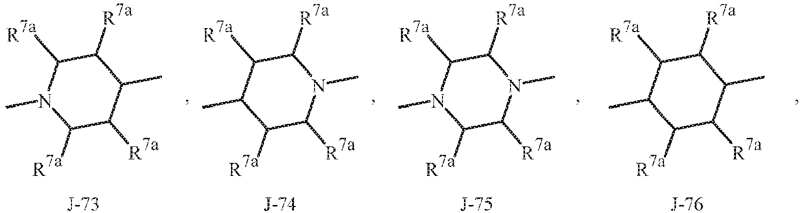

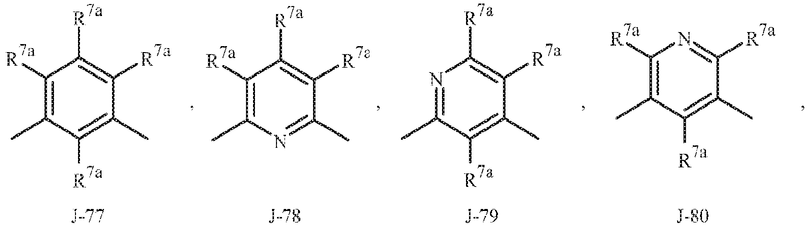

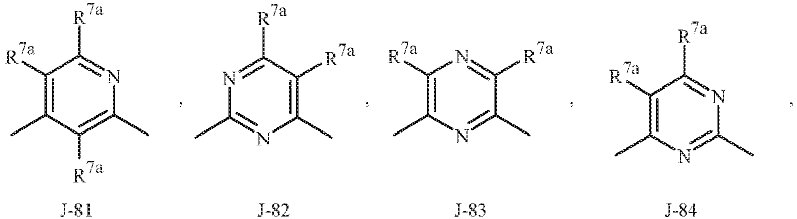

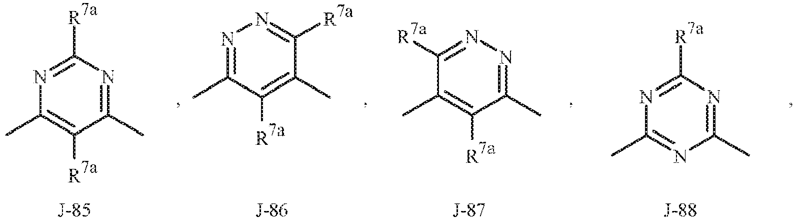

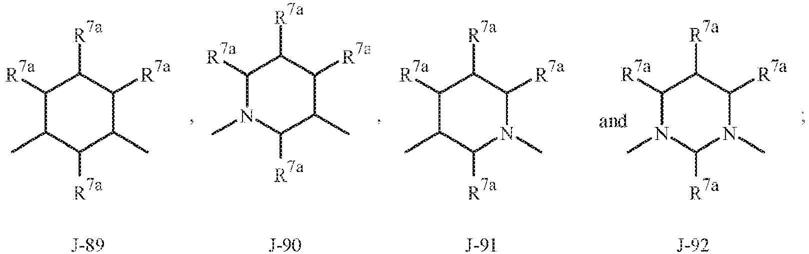

wherein the bond projecting to the left is bonded to Z², and the bond projecting to the right is bonded to the oxadiazole ring in Formula **1;** each R^{7a} is independently H or R⁷; and R^{8b} is H; provided that at most only two of R^{7a} are other than H.

Embodiment 48. A compound of Embodiment 47 wherein J is J-1 through J-5, J-17, J-18, J-37 through J-41, J-60, J-63 through J-71, J-73, J-74, J-75 or J-77 through J-85.

Embodiment 49. A compound of Embodiment 48 wherein J is J-4, J-5, J-40, J-41, J-63 through J-69, J-73 or J-77 through J-85.

Embodiment 50. A compound of Embodiment 49 wherein J is J-63 through J-69 or J-73.

Embodiment 50a. A compound of Embodiment 49 wherein J is J-40.

Embodiment 51. A compound of Embodiment 50 wherein J is J-63 through J-69.

Embodiment 52. A compound of Embodiment 51 wherein J is J-63 through J-65.

Embodiment 53. A compound of Embodiment 52 wherein J is J-63.

Embodiment 54. A compound of Embodiment 52 wherein J is J-64.

Embodiment 55. A compound of Formula 1 or any one of Embodiments 1 through 54 wherein each R³ is independently halogen, cyano, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₂-C₄ alkylcarbonyl, C₂-C₄ haloalkylcarbonyl or C₂-C₅ alkoxycarbonyl; or pyridinyl, pyrazolyl, imidazolyl, triazolyl, oxazolyl, isoxazolyl, thienyl, furanyl, pyrrolidinyl, isoxazolinyl, tetrahydrofuranyl, piperidinyl, morpholinyl or piperazinyl, each optionally substituted with up to 3 substituents independently selected from R^{3c} on carbon atom ring members and R^{3d} on nitrogen atom ring members.

Embodiment 56. A compound of Embodiment 55 wherein each R³ is independently halogen, cyano, C₁-C₃ alkyl, C₁-C₃ haloalkyl, C₃-C₆ cycloalkyl, C₃-C₆ halocycloalkyl, C₁-C₃ alkoxy or C₁-C₃ haloalkoxy; or pyridinyl, pyrazolyl, imidazolyl, triazolyl, pyrrolidinyl, isoxazolinyl, tetrahydrofuranyl, piperidinyl, morpholinyl or piperazinyl, each optionally substituted with up to 2 substituents independently selected from R^{3c} on carbon atom ring members and R^{2b} on nitrogen atom ring members.

Embodiment 57. A compound of Embodiment 56 wherein each R³ is independently halogen, cyano, C₁-C₃ alkyl, C₁-C₃ haloalkyl, C₃-C₆ cycloalkyl, C₃-C₆ halocycloalkyl, C₁-C₃ alkoxy or C₁-C₃ haloalkoxy; or pyridinyl, imidazolyl, triazolyl, pyrrolidinyl, piperidinyl, morpholinyl or piperazinyl, each optionally substituted with up to 2 substituents independently selected from R^{3c} on carbon atom ring members and R^{3d} on nitrogen atom ring members.

Embodiment 57a. A compound of Embodiment 57 wherein each R³ is independently halogen, cyano, C₁-C₃ alkyl, C₁-C₃ haloalkyl, C₃-C₆ halocycloalkyl or C₁-C₃ alkoxy; or pyridinyl, imidazolyl, triazolyl, pyrrolidinyl, piperidinyl, morpholinyl or piperazinyl, each optionally substituted with up to 1 substituent selected from R^{3c} on carbon atom ring members and R^{3d} on nitrogen atom ring members;

Embodiment 58. A compound of Formula 1 or any one of Embodiments 1 through 55 wherein each R³ is independently halogen, cyano, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₂-C₄ alkylcarbonyl, C₂-C₄ haloalkylcarbonyl or C₂-C₅ alkoxycarbonyl.

Embodiment 59. A compound of Embodiment 58 wherein each R³ is independently halogen, cyano, C₁-C₃ alkyl, C₁-C₃ haloalkyl, C₃-C₆ cycloalkyl, C₃-C₆ halocycloalkyl, C₁-C₃ alkoxy or C₁-C₃ haloalkoxy.

Embodiment 60. A compound of Embodiment 59 wherein each R³ is independently halogen, C₁-C₃ alkyl, C₁-C₃ haloalkyl, C₁-C₃ alkoxy or C₁-C₃ haloalkoxy. Embodiment 61. A compound of Formula 1 or any one of Embodiments 1 through 55 wherein each R³ is independently pyridinyl, pyrazolyl, imidazolyl, triazolyl, oxazolyl, isoxazolyl, thienyl, furanyl, pyrrolidinyl, isoxazolinyl, tetrahydrofuranyl, piperidinyl, morpholinyl or piperazinyl, each optionally substituted with up to 3 substituents independently selected from R^{3c} on carbon atom ring members and R^{3d} on nitrogen atom ring members.

Embodiment 62. A compound of Embodiment 61 wherein each R³ is independently pyridinyl, imidazolyl, triazolyl, pyrrolidinyl, piperidinyl, morpholinyl or piperazinyl, each optionally substituted with up to 2 substituents independently selected from R^{3c} on carbon atom ring members and R^{3d} on nitrogen atom ring members.

Embodiment 62a. A compound of Formula 1 or any one of Embodiments 1 through 55 wherein each R³ is independently C₁-C₄ alkyl, C₁-C₄ haloalkyl, C₁-C₄ alkoxy, C₁-C₄ haloalkoxy, C₂-C₄ alkylcarbonyl, C₂-C₄ haloalkylcarbonyl or C₂-C₅ alkoxycarbonyl.

Embodiment 62b. A compound of Embodiment 62a wherein each R³ is independently C₁-C₄ alkyl, C₁-C₄ alkoxy, C₁-C₄ haloalkoxy, C₂-C₄ alkylcarbonyl, C₂-C₄ haloalkylcarbonyl or C₂-C₄ alkoxycarbonyl.

Embodiment 62c. A compound of Embodiment 62b wherein each R³ is independently C₁-C₂ alkyl, C₁-C₂ alkoxy, C₂-C₄ alkylcarbonyl or C₂-C₄ alkoxycarbonyl.

Embodiment 63. A compound of Formula 1 or any one of Embodiments 1 through 62c wherein each R^{3a} and R^{3c} when taken alone (i.e. not taken together with R^{4a} or R^{4b}) is independently halogen, cyano, nitro, C₁-C₄ alkyl, C₁-C₄ haloalkyl, C₂-C₄ alkenyl, C₂-C₄ alkynyl, C₃-C₆ cycloalkyl, C₄-C₇ cycloalkylalkyl, C₁-C₄ alkoxy, C₁-C₄ haloalkoxy, C₂-C₄ alkenyloxy, C₂-C₄ alkynyloxy, C₁-C₄ alkylthio, C₁-C₄ haloalkylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfonyloxy, C₁-C₄ alkylamino, C₂-C₈ dialkylamino, C₃-C₆ cycloalkylamino, C₂-C₄ alkylcarbonyl, C₂-C₆ alkoxycarbonyl, C₂-C₆ alkylaminocarbonyl or C₃-C₈ dialkylaminocarbonyl.

Embodiment 64. A compound of Embodiment 63 wherein each R^{3a} and R^{3c} is independently halogen, cyano, nitro, C₁-C₄ alkyl, C₁-C₄ haloalkyl, C₂-C₄ alkenyl, C₂-C₄ alkynyl, C₃-C₆ cycloalkyl, C₁-C₄ alkoxy, C₁-C₄ haloalkoxy, C₂-C₄ alkenyloxy, C₂-C₄ alkynyloxy, C₁-C₄ alkylthio, C₁-C₄ haloalkylthio, C₁-C₄ alkylsulfonyl, C₁-C₄ haloalkylsulfonyl, C₁-C₄ alkylsulfonyloxy, C₁-C₄ alkylamino, C₂-C₈ dialkylamino, C₃-C₆ cycloalkylamino, C₂-C₄ alkylcarbonyl, C₂-C₅ alkoxycarbonyl, C₂-C₅ alkylaminocarbonyl or C₃-C₇ dialkylaminocarbonyl.

Embodiment 65. A compound of Embodiment 64 wherein each R^{3a} and R^{3c} is independently halogen, cyano, nitro, C₁-C₃ alkyl, C₁-C₃ haloalkyl, C₂-C₃ alkenyl, C₂-C₃ alkynyl, C₃-C₆ cycloalkyl, C₁-C₃ alkoxy, C₁-C₃ haloalkoxy, C₂-C₃ alkenyloxy, C₂-C₃ alkynyloxy, C₁-C₄ alkylsulfonyl, C₁-C₄ haloalkylsulfonyl, C₁-C₄ alkylsulfonyloxy, C₂-C₄ alkylcarbonyl, C₂-C₅ alkoxycarbonyl, C₂-C₅ alkylaminocarbonyl or C₃-C₇ dialkylaminocarbonyl.

Embodiment 66. A compound of Embodiment 65 wherein each R^{3a} and R^{3c} is independently halogen, C₁-C₃ alkyl, C₁-C₃ haloalkyl, C₁-C₃ alkoxy or C₁-C₃ haloalkoxy.

Embodiment 67. A compound of Embodiment 66 wherein each R^{3a} and R^{3c} is independently halogen, methyl, ethyl, methoxy or C₁-C₂ haloalkoxy.

Embodiment 68. A compound of Formula 1 or any one of Embodiments 1 through 47 wherein each R^{3b} and R^{3d} when taken alone (i.e. not taken together with R^{4a} or R^{4b}) is independently C₁-C₂ alkyl, C₁-C₂ alkoxy, C₂-C₃ alkylcarbonyl or C₂-C₃ alkoxycarbonyl.

Embodiment 69. A compound of Embodiment 68 wherein each R^{3b} and R^{3d} is independently methyl, methylcarbonyl or methoxycarbonyl.

Embodiment 70. A compound of Embodiment 69 wherein each R^{3b} and R^{3d} is methyl.

Embodiment 71. A compound of Formula 1 or any one of Embodiments 1 through 70 wherein R^{4a} and R^{4b} when taken alone (i.e. not taken together with R¹, R^{3a} or R^{3b}) are each independently H, hydroxy, C₁-C₄ alkyl, C₁-C₄ haloalkyl, C₂-C₄ alkenyl, C₂-C₄ alkynyl, C₁-C₄ alkoxy, C₁-C₄ haloalkoxy, C₂-C₄ alkoxyalkyl, C₁-C₄ alkylsulfonyl, C₁-C₄ haloalkylsulfonyl, C₂-C₄ alkylcarbonyl, C₂-C₄ haloalkylcarbonyl, C₂-C₅ alkoxycarbonyl, C₂-C₅ alkylaminocarbonyl or C₃-C₅ dialkylaminocarbonyl.

Embodiment 71a. A compound of Embodiment 71 wherein R^{4a} and R^{4b} are each independently H, C₁-C₄ alkyl, C₂-C₄ alkenyl, C₁-C₄ alkoxy or C₂-C₄ alkylcarbonyl.

Embodiment 71b. A compound of Embodiment 71a wherein R^{4a} and R^{4b} are each independently H, C₁-C₂ alkyl, C₂-C₄ alkenyl, C₁-C₂ alkoxy or C₂-C₄ alkylcarbonyl.

Embodiment 72. A compound of Embodiment 51 wherein R^{4a} and R^{4b} are each independently H, C₁-C₃ alkyl, C₁-C₃ haloalkyl, C₂-C₃ alkenyl, C₂-C₃ alkynyl, C₂-C₃ alkoxyalkyl, C₁-C₃ alkylsulfonyl, C₁-C₃ haloalkylsulfonyl, C₂-C₃ alkylcarbonyl, C₂-C₃ haloalkylcarbonyl, C₂-C₃ alkoxycarbonyl, C₂-C₃ alkylaminocarbonyl or C₃-C₅ dialkylaminocarbonyl.

Embodiment 73. A compound of Embodiment 72 wherein R^{4a} and R^{4b} are each independently H, methyl, C₁-C₂ haloalkyl, methoxymethyl, methylsulfonyl, methylcarbonyl or methoxycarbonyl.

Embodiment 74. A compound of Embodiment 73 wherein R^{4a} and R^{4b} are each independently H or methyl.

Embodiment 75. A compound of Formula **1** or any one of Embodiments 1 through 74 wherein when one pair of R¹ and R^{4a} substituents or one pair of R¹ and R^{4b} substituents are taken together with the atoms to which they are attached to form a ring, said ring is a 5- to 7-membered ring containing ring members selected from carbon atoms and optionally up to 3 heteroatoms independently selected from up to 1 O, up to 1 S and up to 2 N atoms, the ring optionally substituted with up to 3 substituents independently selected from R⁹.

Embodiment 76. A compound of Embodiment 75 wherein one pair of R¹ and R^{4a} substituents or one pair of R¹ and R^{4b} substituents are taken together with the atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 3 substituents independently selected from R⁹.

Embodiment 77. A compound of Embodiment 76 wherein one pair of R¹ and R^{4a} substituents or one pair of R¹ and R^{4b} substituents are taken together with the atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 2 substituents independently selected from R⁹.

Embodiment 78. A compound of Formula **1** or any one of Embodiments 1 through 77 wherein when one pair of R^{3a} and R^{4a} substituents or one pair of R^{3a} and R^{4b} substituents are taken together with the atoms to which they are attached to form a ring, said ring is a 5- to 7-membered ring containing ring members selected from carbon atoms and optionally up to 3 heteroatoms independently selected from up to 1 O, up to 1 S and up to 2 N atoms, the ring optionally substituted with up to 3 substituents independently selected from R¹⁰.

Embodiment 79. A compound of Embodiment 78 wherein one pair of R^{3a} and R^{4a} substituents or one pair of R^{3a} and R^{4b} substituents are taken together with the atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 3 substituents independently selected from R¹⁰.

Embodiment 80. A compound of Embodiment 79 wherein one pair of R^{3a} and R^{4a} substituents or one pair of R^{3a} and R^{4b} substituents are taken together with the atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 2 substituents independently selected from R¹⁰.

Embodiment 81. A compound of Formula **1** or any one of Embodiments 1 through 80 wherein when one pair of R^{3b} and R^{4a} substituents or one pair of R^{3b} and R^{4b} substituents are taken together with the nitrogen atoms to which they are attached to form a ring, said ring is a 5- to 7-membered ring containing ring members selected from carbon atoms and optionally up to 3 heteroatoms independently selected from up to 1 O, up to 1 S and up to 2 N atoms, the ring optionally substituted with up to 3 substituents independently selected from R¹¹.

Embodiment 82. A compound of Embodiment 81 wherein one pair of R^{3b} and R^{4a} substituents or one pair of R^{3b} and R^{4b} substituents are taken together with the nitrogen atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 3 substituents independently selected from R¹¹.

Embodiment 83. A compound of Embodiment 82 wherein one pair of R^{3b} and R^{4a} substituents or one pair of R^{3b} and R^{4b} substituents are taken together with the nitrogen atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 2 substituents independently selected from R¹¹.

Embodiment 84. A compound of Formula **1** or any one of Embodiments 1 through 83 wherein R⁵ is H, hydroxy, cyano, C₁-C₄ alkyl, C₁-C₄ haloalkyl, C₂-C₄ alkenyl, C₂-C₄ alkynyl, C₁-C₄ alkoxy, C₁-C₄ haloalkoxy, C₂-C₄ alkoxyalkyl, C₁-C₄ alkylsulfonyl, C₁-C₄ haloalkylsulfonyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl or C₃-C₅ dialkylaminocarbonyl.

Embodiment 85. A compound of Embodiment 84 wherein R⁵ is H, hydroxy, cyano, C₁-C₄ alkyl, C₁-C₄ haloalkyl, C₂-C₄ alkenyl, C₂-C₄ alkynyl, C₁-C₄ alkoxy, C₁-C₄ haloalkoxy, C₂-C₄ alkoxyalkyl, C₁-C₄ alkylsulfonyl, C₁-C₄ haloalkylsulfonyl C₂-C₄ alkylcarbonyl, C₂-C₄ haloalkylcarbonyl, C₂-C₅ alkoxycarbonyl, C₂-C₅ alkylaminocarbonyl or C₃-C₅ dialkylaminocarbonyl.

Embodiment 86. A compound of Embodiment 85 wherein R⁵ is H, C₁-C₃ alkyl, C₁-C₃ haloalkyl, C₂-C₃ alkenyl, C₂-C₃ alkynyl, C₂-C₃ alkoxyalkyl, C₁-C₃ alkylsulfonyl, C₁-C₃ haloalkylsulfonyl C₂-C₃ alkylcarbonyl, C₂-C₃ haloalkylcarbonyl, C₂-C₃ alkoxycarbonyl, C₂-C₅ alkylaminocarbonyl or C₃-C₅ dialkylaminocarbonyl.

Embodiment 87. A compound of Embodiment 86 wherein R⁵ is H, methyl, methoxymethyl, methylcarbonyl or methoxycarbonyl.

Embodiment 88. A compound of Embodiment 87 wherein R⁵ is H or methyl.

Embodiment 89. A compound of Formula 1 or any one of Embodiments 1 through 88 wherein each R⁶ is independently halogen, cyano, nitro, C₁-C₂ alkyl, C₁-C₂ haloalkyl, C₁-C₂ alkoxy or C₁-C₂ haloalkoxy.

Embodiment 90. A compound of Embodiment 89 wherein each R⁶ is independently halogen, cyano, nitro, methyl, trifluoromethyl or methoxy.

Embodiment 91. A compound of Formula **1** or any one of Embodiments 1 through 90 wherein each R⁷ is independently halogen, hydroxy, cyano, amino, nitro, C₁-C₄ alkyl, C₁-C₄ haloalkyl, C₂-C₄ alkenyl, C₂-C₄ alkynyl, C₁-C₄ hydroxyalkyl, C₃-C₆ cycloalkyl, C₁-C₄ alkoxy, C₁-C₄ haloalkoxy, C₂-C₄ alkenyloxy, C₂-C₄ alkynyloxy, C₂-C₄ alkoxyalkyl, C₁-C₄ alkylthio, C₁-C₄ haloalkylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfonyloxy, C₁-C₄ alkylamino, C₂-C₆ dialkylamino, C₃-C₆ cycloalkylamino, C₂-C₄ alkylcarbonyl, C₂-C₆ alkoxycarbonyl, C₂-C₆ alkylaminocarbonyl or C₃-C₆ dialkylaminocarbonyl.

Embodiment 92. A compound of Embodiment 91 wherein each R⁷ is independently halogen, cyano, nitro, C₁-C₄ alkyl, C₁-C₄ haloalkyl, C₂-C₄ alkenyl, C₂-C₄ alkynyl, C₃-C₆ cycloalkyl, C₁-C₄ alkoxy, C₁-C₄ haloalkoxy, C₂-C₄ alkenyloxy, C₂-C₄ alkynyloxy, C₁-C₄ alkylthio, C₁-C₄ haloalkylthio, C₁-C₄ alkylsulfonyl, C₁-C₄ haloalkylsulfonyl, C₁-C₄ alkylsulfonyloxy, C₁-C₄ alkylamino, C₂-C₆ dialkylamino, C₃-C₆ cycloalkylamino, C₂-C₄ alkylcarbonyl, C₂-C₆ alkoxycarbonyl, C₂-C₆ alkylaminocarbonyl or C₃-C₆ dialkylaminocarbonyl.

Embodiment 93. A compound of Embodiment 92 wherein each R⁷ is independently halogen, cyano, nitro, C₁-C₃ alkyl, C₁-C₃ haloalkyl, C₂-C₃ alkenyl, C₂-C₃ alkynyl, C₃-C₆ cycloalkyl, C₁-C₃ alkoxy, C₁-C₃ haloalkoxy, C₂-C₃ alkenyloxy, C₂-C₃ alkynyloxy, C₁-C₄ alkylsulfonyl, C₁-C₄ haloalkylsulfonyl, C₁-C₄ alkylsulfonyloxy, C₂-C₄ alkylcarbonyl, C₂-C₆ alkoxycarbonyl, C₂-C₆ alkylaminocarbonyl or C₃-C₆ dialkylaminocarbonyl.

Embodiment 94. A compound of Embodiment 93 wherein each R⁷ is independently halogen, C₁-C₃ alkyl, C₁-C₃ haloalkyl, C₂-C₃ alkenyl, C₁-C₃ alkoxy, C₁-C₃ haloalkoxy, C₂-C₄ alkylcarbonyl or C₂-C₆ alkoxycarbonyl.

Embodiment 95. A compound of Embodiment 94 wherein each R⁷ is independently halogen, methyl, trifluoromethyl or methoxy.

Embodiment 96. A compound of Formula 1 or any one of Embodiments 1 through 95 wherein each R⁸ is independently methyl or ethyl.

Embodiment 97. A compound of Embodiment 96 wherein R⁸ is methyl.

Embodiment 98. A compound of Formula 1 or any one of Embodiments 1 through 97 wherein each R⁹, R¹⁰ and R¹¹ is independently halogen, hydroxy, cyano, nitro, C₁-C₄ alkyl, C₁-C₄ haloalkyl, C₂-C₄ alkenyl, C₂-C₄ alkynyl, C₁-C₄ alkoxy, C₁-C₄ haloalkoxy, C₁-C₄ alkylsulfonyl, C₁-C₄ haloalkylsulfonyl, C₂-C₄ alkylcarbonyl, C₂-C₄ haloalkylcarbonyl, C₂-C₅ alkoxycarbonyl, C₂-C₅ alkylaminocarbonyl or C₃-C₅ dialkylaminocarbonyl.

Embodiment 99. A compound of Embodiment 98 wherein each R⁹, R¹⁰ and R¹¹ is independently halogen, hydroxy, cyano, nitro, C₁-C₃ alkyl, C₁-C₃ haloalkyl, C₂-C₃ alkenyl, C₂-C₃ alkynyl, C₁-C₃ alkoxy, C₁-C₃ haloalkoxy, C₁-C₃ alkylsulfonyl, C₁-C₃ haloalkylsulfonyl, C₂-C₃ alkylcarbonyl, C₂-C₃ haloalkylcarbonyl, C₂-C₄ alkoxycarbonyl, C₂-C₄ alkylaminocarbonyl or C₃-C₅ dialkylaminocarbonyl.

Embodiment 100. A compound of Embodiment 99 wherein each R⁹, R¹⁰ and R¹¹ is independently halogen, hydroxy, cyano, nitro, C₁-C₃ alkyl, C₁-C₃ haloalkyl, C₁-C₃ alkoxy or C₁-C₃ haloalkoxy.

Embodiment 101. A compound of Formula 1 or any one of Embodiments 1 through 100 wherein R¹³ is phenyl optionally substituted with up to 2 substituents independently selected from R¹⁴.

Embodiment 102. A compound of Embodiment 101 wherein R¹³ is phenyl optionally substituted with up to 1 substituent selected from R¹⁴.

Embodiment 103. A compound of Embodiment 102 wherein each R¹³ is phenyl.

Embodiment 104. A compound of Formula 1 or any one of Embodiments 1 through 102 wherein each R¹⁴ is independently halogen, cyano, C₁-C₂ alkyl, C₁-C₂ haloalkyl or C₁-C₂ alkoxy.

Embodiment 105. A compound of Embodiment 104 wherein each R¹⁴ is independently halogen, cyano, methyl, halomethyl or methoxy.

Embodiment 106. A compound of Embodiment 105 wherein each R¹⁴ is independently halogen, methyl or methoxy.

Embodiment 107. A compound of Embodiment 106 wherein each R¹⁴ is independently halogen or methyl.

Embodiments disclosed herein, but not claimed, including Embodiments 1-107 above as well as any other embodiments described herein, can be combined in any manner, and the descriptions of variables in the embodiments pertain not only to the compounds of Formula 1 but also to the starting compounds and intermediate compounds useful for preparing the compounds of Formula **1**.

Combinations of Embodiments 1-107 are illustrated by:
Embodiment A. A compound of Formula 1 wherein
   R¹ is C₁-C₆ alkyl, C₂-C₆ alkenyl, C₂-C₆ alkynyl, C₂-C₅ alkylcarbonyl or C₂-C₅ alkoxycarbonyl, each optionally substituted with up to 2 substituents independently selected from R³; or phenyl, benzyl or naphthalenyl, each optionally substituted with up to 4 substituents independently selected from R^{3a}; or a 3- to 7-membered carbocyclic ring, wherein up to 2 carbon atom ring members are independently selected from C(=O) and C(=S), each ring optionally substituted with up to 4 substituents independently selected from R^{3a}; or a 5- to 6-membered heterocyclic ring, each ring containing ring members selected from carbon atoms and 1 to 4 heteroatoms independently selected from up to 2 O, up to 2 S and up to 4 N atoms, wherein up to 2 carbon atom ring members are independently selected from C(=O) and C(=S), each ring optionally substituted with up to 4 substituents independently selected from R^{3a} on carbon atom ring members and R^{3b} on nitrogen atom ring members;
   Z¹ is a direct bond, NR^{4a} or NR^{4a}NR^{4b};
   s is 0, 1 or 2 and f is 0,
   Z² is a direct bond, O, NR⁵ or NR⁵CH₂; or CH₂ or CH₂CH₂, each optionally substituted with up to 4 substituents independently selected from R⁶;
   J is phenyl optionally substituted with up to 2 substituents independently selected from R⁷; or a 3- to 7-membered carbocyclic ring, wherein up to 2 carbon atom ring members are independently selected from C(=O) and C(=S), each ring optionally substituted with up to 2 substituents independently selected from R⁷; or a 5- to 6-membered heterocyclic ring, each ring containing ring members selected from carbon atoms and 1 to 4 heteroatoms independently selected from up to 2 O, up to 2 S and up to 4 N atoms, wherein up to 2 carbon ring members are independently selected from C(=O) and C(=S), each ring optionally substituted with up to 2 substituents independently selected from R⁷ on carbon atom ring members and R⁸ on nitrogen atom ring members;
   each R³ is independently halogen, cyano, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₂-C₄ alkylcarbonyl, C₂-C₄ haloalkylcarbonyl or C₂-C₅ alkoxycarbonyl; or pyridinyl, pyrazolyl, imidazolyl, triazolyl, oxazolyl, isoxazolyl, thienyl, furanyl, pyrrolidinyl, isoxazolinyl, tetrahydrofuranyl, piperidinyl, morpholinyl or piperazinyl, each optionally substituted with up to 3 substituents independently selected from R^{3c} on carbon atom ring members and R^{3d} on nitrogen atom ring members;
   each R^{3a} and R^{3c} is independently halogen, cyano, nitro, C₁-C₄ alkyl, C₁-C₄ haloalkyl, C₂-C₄ alkenyl, C₂-C₄ alkynyl, C₃-C₆ cycloalkyl, C₄-C₇ cycloalkylalkyl, C₁-C₄ alkoxy, C₁-C₄ haloalkoxy, C₂-C₄ alkenyloxy, C₂-C₄ alkynyloxy, C₁-C₄ alkylthio, C₁-C₄ haloalkylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfonyloxy, C₁-C₄ alkylamino, C₂-C₈ dialkylamino, C₃-C₆ cycloalkylamino, C₂-C₄ alkylcarbonyl, C₂-C₆ alkoxycarbonyl, C₂-C₆ alkylaminocarbonyl or C₃-C₈ dialkylaminocarbonyl;
   each R^{3b} and R^{3d} is independently C₁-C₂ alkyl, C₁-C₂ alkoxy, C₂-C₃ alkylcarbonyl or C₂-C₃ alkoxycarbonyl;
   R^{4a} and R^{4b} are each independently H, hydroxy, C₁-C₄ alkyl, C₁-C₄ haloalkyl, C₂-C₄ alkenyl, C₂-C₄ alkynyl, C₁-C₄ alkoxy, C₁-C₄ haloalkoxy, C₂-C₄ alkoxyalkyl, C₁-C₄ alkylsulfonyl, C₁-C₄ haloalkylsulfonyl, C₂-C₄ alkylcarbonyl, C₂-C₄ haloalkylcarbonyl, C₂-C₅ alkoxycarbonyl, C₂-C₅ alkylaminocarbonyl or C₃-C₅ dialkylaminocarbonyl; or
   one pair of R¹ and R^{4a} substituents or one pair of R¹ and R^{4b} substituents are taken together with the atoms to which they are attached to form a 5- to 7-membered ring containing ring members selected from carbon atoms and optionally up to 3 heteroatoms independently selected from up to 1 O, up to 1 S and up to 2 N atoms, the ring optionally substituted with up to 3 substituents independently selected from R⁹; or
   one pair of R^{3a} and R^{4a} substituents or one pair of R^{3a} and R^{4b} substituents are taken together with the atoms to which they are attached to form a 5-to 7-membered ring containing ring members selected from carbon atoms and optionally up to 3 heteroatoms independently selected from up to 1 O, up to 1 S and up to 2 N atoms, the ring optionally substituted with up to 3 substituents independently selected from R¹⁰; or
   one pair of R^{3b} and R^{4a} substituents or one pair of R^{3b} and R^{4b} substituents are taken together with the nitrogen atoms to which they are attached to form a 5- to 7-membered ring containing ring members selected from carbon atoms and optionally up to 3 heteroatoms independently selected from up to 1 O, up to 1 S and up to 2 N atoms, the ring optionally substituted with up to 3 substituents independently selected from R¹¹;
   R⁵ is H, hydroxy, cyano, C₁-C₄ alkyl, C₁-C₄ haloalkyl, C₂-C₄ alkenyl, C₂-C₄ alkynyl, C₁-C₄ alkoxy, C₁-C₄ haloalkoxy, C₂-C₄ alkoxyalkyl, C₁-C₄ alkylsulfonyl, C₁-C₄ haloalkylsulfonyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl or C₃-C₅ dialkylaminocarbonyl;
   each R⁶ is independently halogen, cyano, nitro, C₁-C₂ alkyl, C₁-C₂ haloalkyl, C₁-C₂ alkoxy or C₁-C₂ haloalkoxy;
   each R⁷ is independently halogen, hydroxy, cyano, amino, nitro, C₁-C₄ alkyl, C₁-C₄ haloalkyl, C₂-C₄ alkenyl, C₂-C₄ alkynyl, C₁-C₄ hydroxyalkyl, C₃-C₆ cycloalkyl, C₁-C₄ alkoxy, C₁-C₄ haloalkoxy, C₂-C₄ alkenyloxy, C₂-C₄ alkynyloxy, C₂-C₄ alkoxyalkyl, C₁-C₄ alkylthio, C₁-C₄ haloalkylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfonyloxy, C₁-C₄ alkylamino, C₂-C₆ dialkylamino, C₃-C₆ cycloalkylamino, C₂-C₄ alkylcarbonyl, C₂-C₆ alkoxycarbonyl, C₂-C₆ alkylaminocarbonyl or C₃-C₆ dialkylaminocarbonyl;
   each R⁸ is independently methyl or ethyl; and
   each R⁹, R¹⁰ and R¹¹ is independently halogen, hydroxy, cyano, nitro, C₁-C₄ alkyl, C₁-C₄ haloalkyl, C₂-C₄ alkenyl, C₂-C₄ alkynyl, C₁-C₄ alkoxy, C₁-C₄ haloalkoxy, C₁-C₄ alkylsulfonyl, C₁-C₄ haloalkylsulfonyl, C₂-C₄ alkylcarbonyl, C₂-C₄ haloalkylcarbonyl, C₂-C₅ alkoxycarbonyl, C₂-C₅ alkylaminocarbonyl or C₃-C₅ dialkylaminocarbonyl.
Embodiment B. A compound of Embodiment A wherein
   R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 2 substituents independently selected from R³; or R¹ is selected from U-1 through U-79 as shown in Exhibit A

### Exhibit A

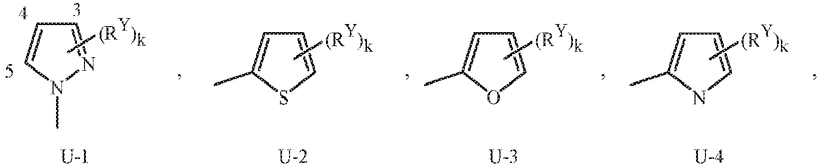

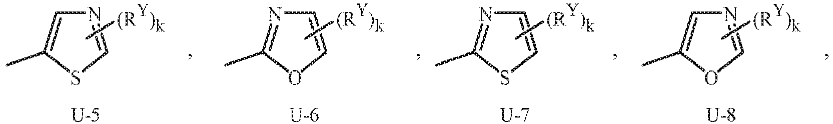

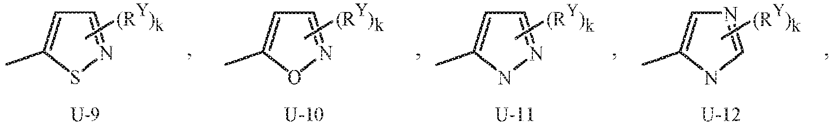

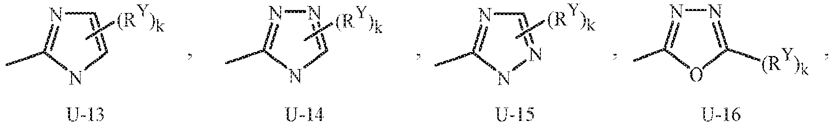

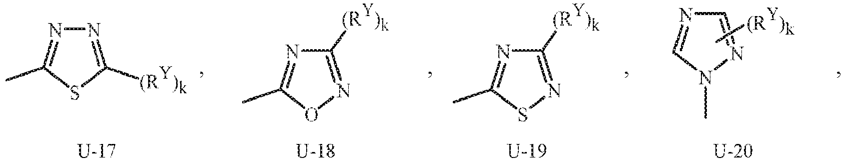

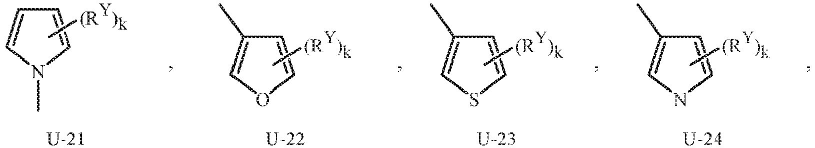

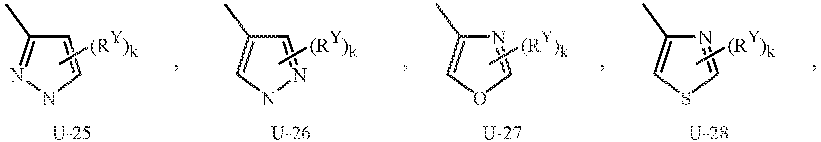

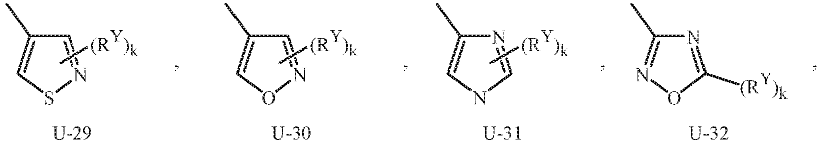

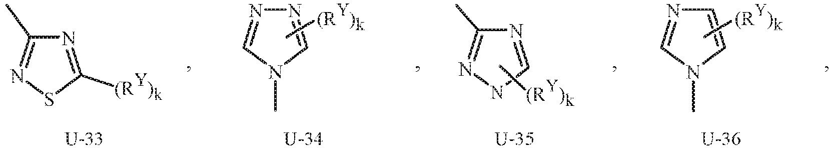

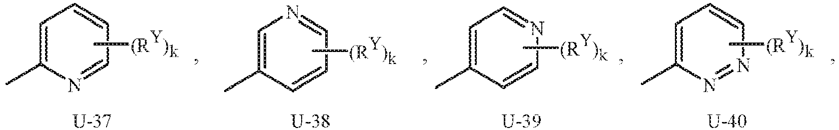

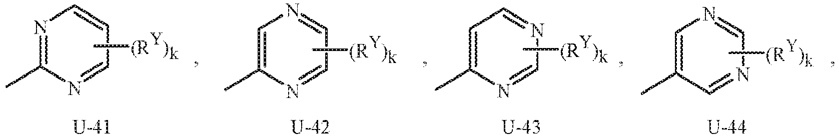

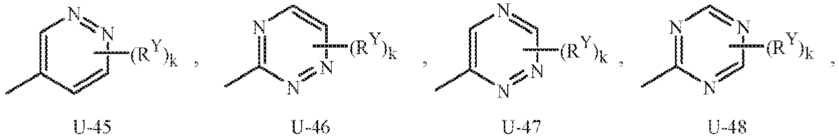

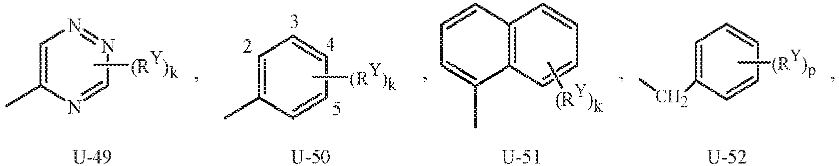

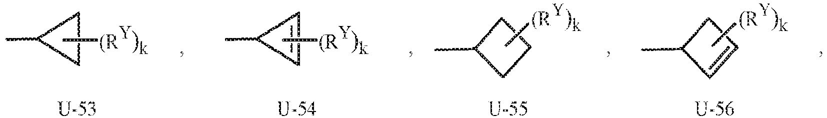

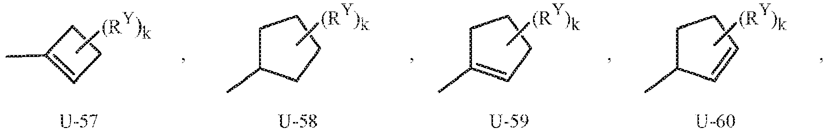

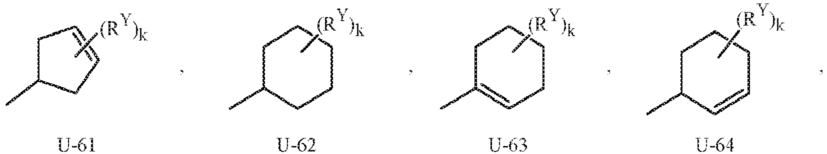

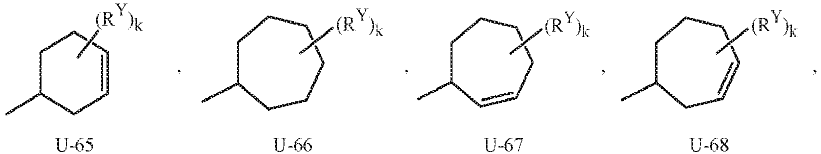

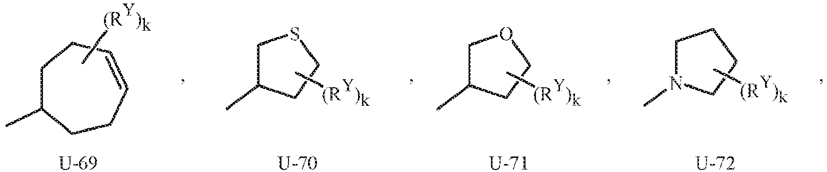

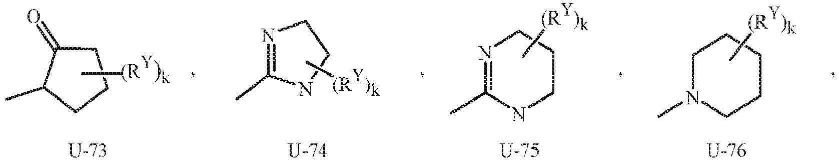

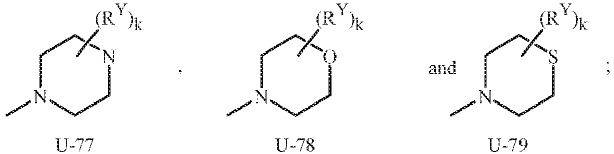

wherein when R^{Y} is attached to a carbon ring member, said R^{Y} is selected from R^{3a}, and when R^{Y} is attached to a nitrogen ring member (e.g., in U-4, U-11 through U-15, U-24 through U-26, U-31, U-35, U-74, U-75 and U-77), said R^{Y} is selected from R^{3b}; and k is 0, 1 or 2
Z² is a direct bond, NR⁵ or NR⁵CH₂; or CH₂, optionally substituted with up to 2 substituents independently selected from R⁶;
J is selected from J-1 through J-92 as shown in Exhibit B

### Exhibit B

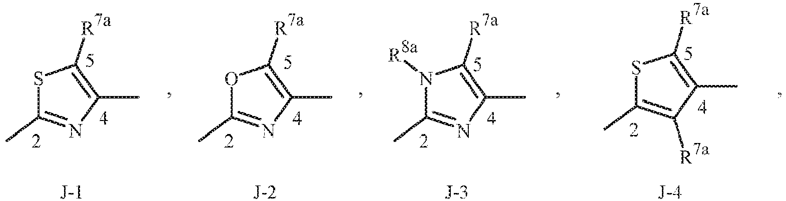

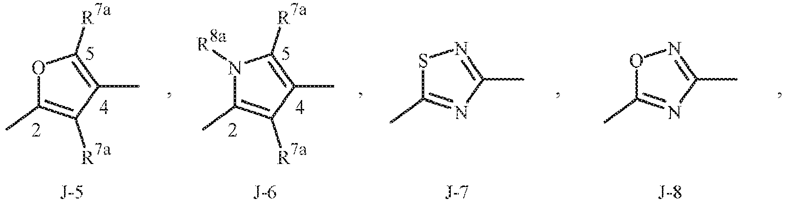

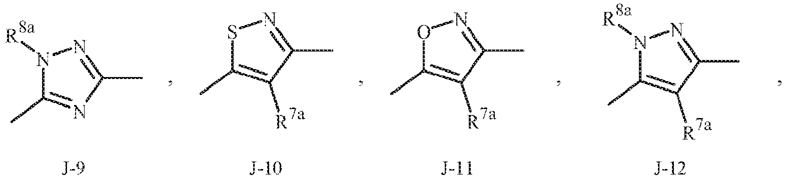

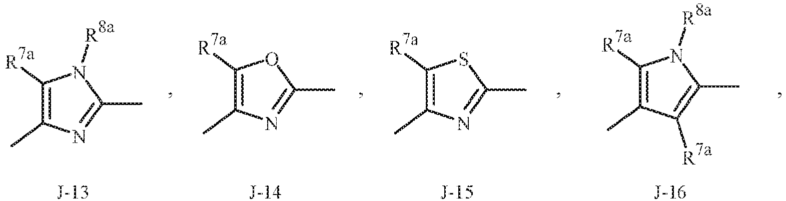

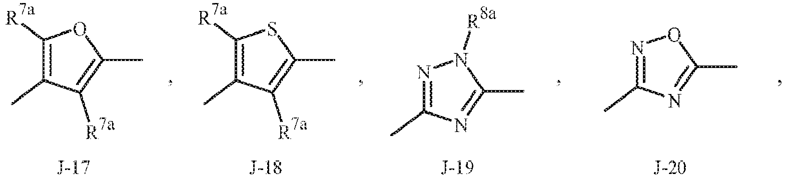

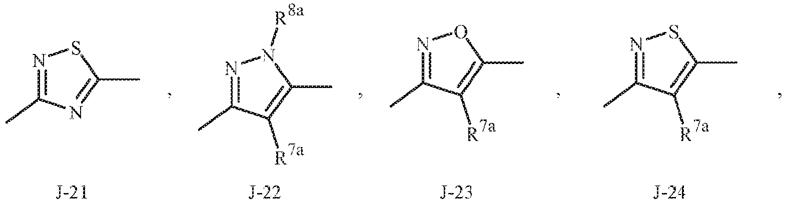

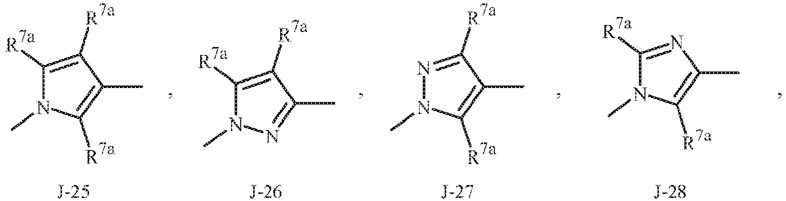

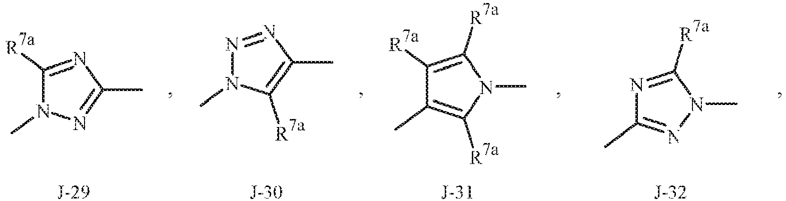

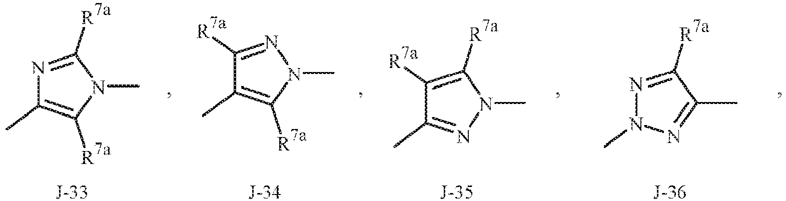

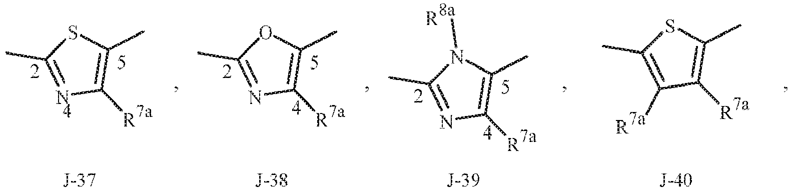

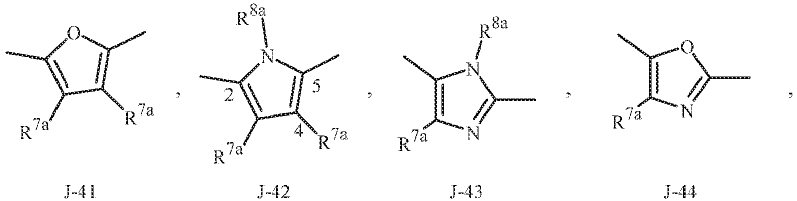

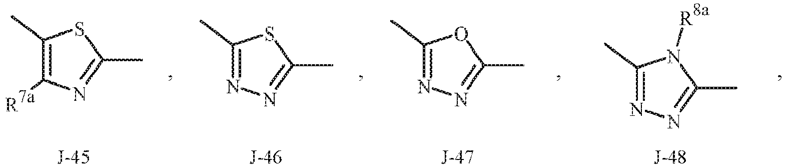

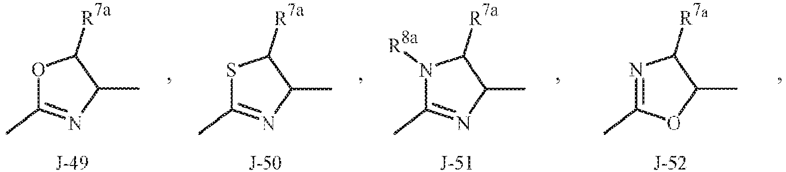

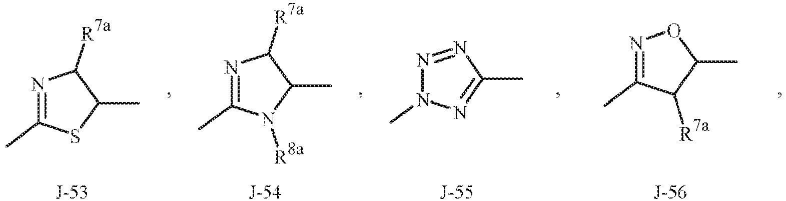

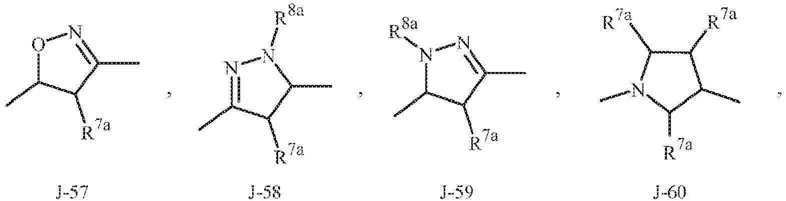

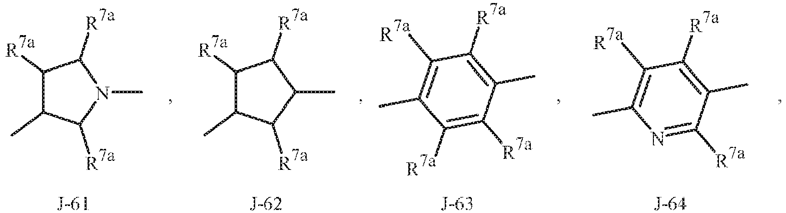

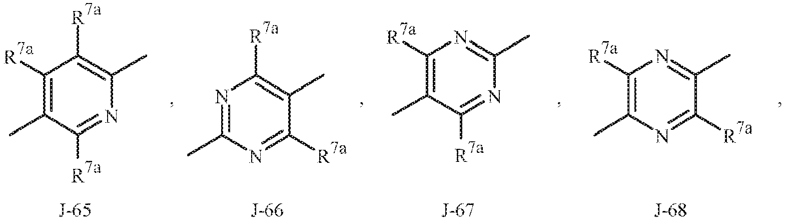

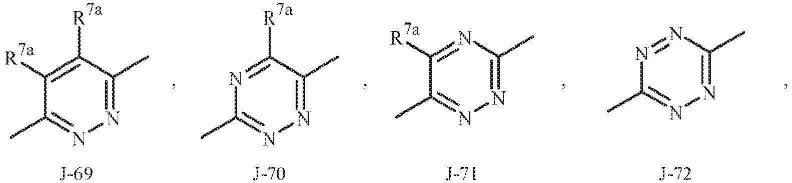

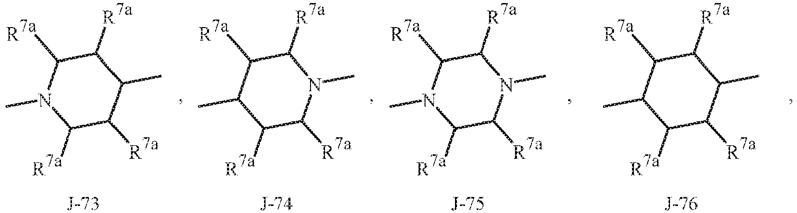

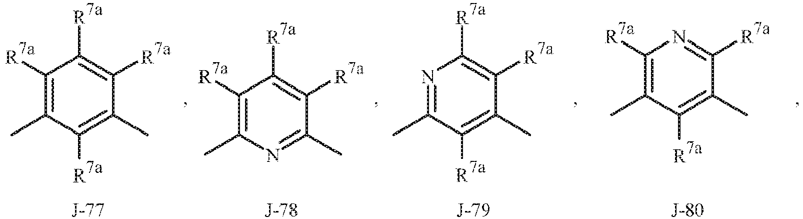

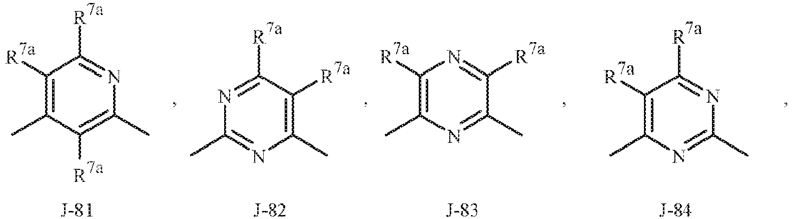

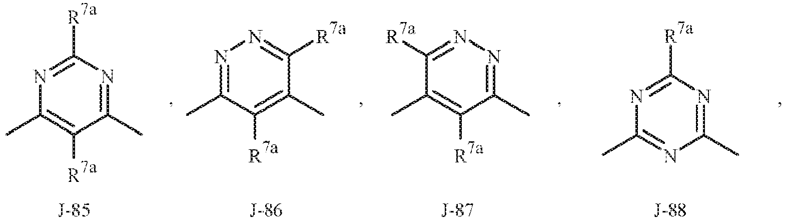

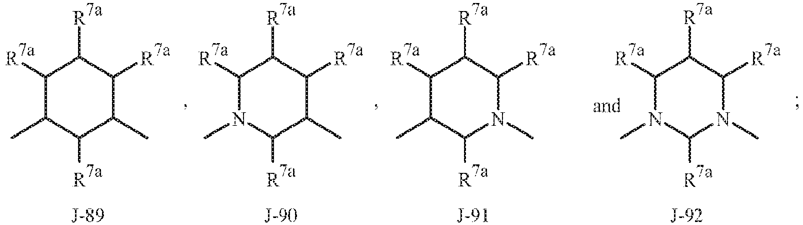

wherein the bond projecting to the left is bonded to Z², and the bond projecting to the right is bonded to the oxadiazole ring in Formula 1; each R^{7a} is independently H or R⁷; and R^{8b} is H; provided that at most, only two of R^{7a} are other than H;
each R³ is independently halogen, cyano, C₁-C₃ alkyl, C₁-C₃ haloalkyl, C₃-C₆ cycloalkyl, C₃-C₆ halocycloalkyl, C₁-C₃ alkoxy or C₁-C₃ haloalkoxy; or pyridinyl, pyrazolyl, imidazolyl, triazolyl, pyrrolidinyl, isoxazolinyl, tetrahydrofuranyl, piperidinyl, morpholinyl or piperazinyl, each optionally substituted with up to 2 substituents independently selected from R^{3c} on carbon atom ring members and R^{3d} on nitrogen atom ring members;
each R^{3a} and R^{3c} is independently halogen, cyano, nitro, C₁-C₄ alkyl, C₁-C₄ haloalkyl, C₂-C₄ alkenyl, C₂-C₄ alkynyl, C₃-C₆ cycloalkyl, C₁-C₄ alkoxy, C₁-C₄ haloalkoxy, C₂-C₄ alkenyloxy, C₂-C₄ alkynyloxy, C₁-C₄ alkylthio, C₁-C₄ haloalkylthio, C₁-C₄ alkylsulfonyl, C₁-C₄ haloalkylsulfonyl, C₁-C₄ alkylsulfonyloxy, C₁-C₄ alkylamino, C₂-C₈ dialkylamino, C₃-C₆ cycloalkylamino, C₂-C₄ alkylcarbonyl, C₂-C₅ alkoxycarbonyl, C₂-C₅ alkylaminocarbonyl or C₃-C₇ dialkylaminocarbonyl;
each R^{3b} and R^{3d} is independently methyl, methylcarbonyl or methoxycarbonyl;
R^{4a} and R^{4b} are each independently H, C₁-C₃ alkyl, C₁-C₃ haloalkyl, C₂-C₃ alkenyl, C₂-C₃ alkynyl, C₂-C₃ alkoxyalkyl, C₁-C₃ alkylsulfonyl, C₁-C₃ haloalkylsulfonyl, C₂-C₃ alkylcarbonyl, C₂-C₃ haloalkylcarbonyl, C₂-C₃ alkoxycarbonyl, C₂-C₃ alkylaminocarbonyl or C₃-C₅ dialkylaminocarbonyl; or
one pair of R¹ and R^{4a} substituents or one pair of R¹ and R^{4b} substituents are taken together with the atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 3 substituents independently selected from R⁹; or
one pair of R^{3a} and R^{4a} substituents or one pair of R^{3a} and R^{4b} substituents are taken together with the atoms to which they are attached to form a 5-to 6-membered ring containing ring members selected from carbon atoms and optionally up to 3 heteroatoms independently selected from up to 1 O, up to 1 S and up to 2 N atoms, the ring optionally substituted with up to 3 substituents independently selected from R¹⁰; or
one pair of R^{3b} and R^{4a} substituents or one pair of R^{3b} and R^{4b} substituents are taken together with the nitrogen atoms to which they are attached to form a 5- to 6-membered ring containing ring members selected from carbon atoms and optionally up to 3 heteroatoms independently selected from up to 1 O, up to 1 S and up to 2 N atoms, the ring optionally substituted with up to 3 substituents independently selected from R¹¹;
R⁵ is H, hydroxy, cyano, C₁-C₄ alkyl, C₁-C₄ haloalkyl, C₂-C₄ alkenyl, C₂-C₄ alkynyl, C₁-C₄ alkoxy, C₁-C₄ haloalkoxy, C₂-C₄ alkoxyalkyl, C₁-C₄ alkylsulfonyl, C₁-C₄ haloalkylsulfonyl C₂-C₄ alkylcarbonyl, C₂-C₄ haloalkylcarbonyl, C₂-C₅ alkoxycarbonyl, C₂-C₅ alkylaminocarbonyl or C₃-C₅ dialkylaminocarbonyl;
each R⁶ is independently halogen, cyano, nitro, methyl, trifluoromethyl or methoxy;
each R⁷ is independently halogen, cyano, nitro, C₁-C₄ alkyl, C₁-C₄ haloalkyl, C₂-C₄ alkenyl, C₂-C₄ alkynyl, C₃-C₆ cycloalkyl, C₁-C₄ alkoxy, C₁-C₄ haloalkoxy, C₂-C₄ alkenyloxy, C₂-C₄ alkynyloxy, C₁-C₄ alkylthio, C₁-C₄ haloalkylthio, C₁-C₄ alkylsulfonyl, C₁-C₄ haloalkylsulfonyl, C₁-C₄ alkylsulfonyloxy, C₁-C₄ alkylamino, C₂-C₆ dialkylamino, C₃-C₆ cycloalkylamino, C₂-C₄ alkylcarbonyl, C₂-C₆ alkoxycarbonyl, C₂-C₆ alkylaminocarbonyl or C₃-C₆ dialkylaminocarbonyl; and
each R⁹, R¹⁰ and R¹¹ is independently halogen, hydroxy, cyano, nitro, C₁-C₃ alkyl, C₁-C₃ haloalkyl, C₁-C₃ alkoxy or C₁-C₃ haloalkoxy.

Embodiment C. A compound of Embodiment B wherein
R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 2 substituents independently selected from R³; or U-1 through U-53, U-55, U-58, U-62, U-66 or U-74 through U-79;
Z¹ is a direct bond, NR^{4a};
Z² is a direct bond, NR⁵, NR⁵CH₂ or CH₂;
J is J-1 through J-5, J-17, J-18, J-37 through J-41, J-60, J-63 through J-71, J-73, J-74, J-75 or J-77 through J-85;
each R³ is independently halogen, cyano, C₁-C₃ alkyl, C₁-C₃ haloalkyl, C₃-C₆ cycloalkyl, C₃-C₆ halocycloalkyl, C₁-C₃ alkoxy or C₁-C₃ haloalkoxy; or pyridinyl, imidazolyl, triazolyl, pyrrolidinyl, piperidinyl, morpholinyl or piperazinyl, each optionally substituted with up to 2 substituents independently selected from R^{3c} on carbon atom ring members and R^{3d} on nitrogen atom ring members;
each R^{3a} and R^{3c} is independently halogen, cyano, nitro, C₁-C₃ alkyl, C₁-C₃ haloalkyl, C₂-C₃ alkenyl, C₂-C₃ alkynyl, C₃-C₆ cycloalkyl, C₁-C₃ alkoxy, C₁-C₃ haloalkoxy, C₂-C₃ alkenyloxy, C₂-C₃ alkynyloxy, C₁-C₄ alkylsulfonyl, C₁-C₄ haloalkylsulfonyl, C₁-C₄ alkylsulfonyloxy, C₂-C₄ alkylcarbonyl, C₂-C₅ alkoxycarbonyl, C₂-C₅ alkylaminocarbonyl or C₃-C₇ dialkylaminocarbonyl;
R^{4a} is H, methyl, C₁-C₂ haloalkyl, methoxymethyl, methylsulfonyl, methylcarbonyl or methoxycarbonyl;
R⁵ is H, C₁-C₃ alkyl, C₁-C₃ haloalkyl, C₂-C₃ alkenyl, C₂-C₃ alkynyl, C₂-C₃ alkoxyalkyl, C₁-C₃ alkylsulfonyl, C₁-C₃ haloalkylsulfonyl C₂-C₃ alkylcarbonyl, C₂-C₃ haloalkylcarbonyl, C₂-C₃ alkoxycarbonyl, C₂-C₅ alkylaminocarbonyl or C₃-C₅ dialkylaminocarbonyl; and
each R⁷ is independently halogen, cyano, nitro, C₁-C₃ alkyl, C₁-C₃ haloalkyl, C₂-C₃ alkenyl, C₂-C₃ alkynyl, C₃-C₆ cycloalkyl, C₁-C₃ alkoxy, C₁-C₃ haloalkoxy, C₂-C₃ alkenyloxy, C₂-C₃ alkynyloxy, C₁-C₄ alkylsulfonyl, C₁-C₄ haloalkylsulfonyl, C₁-C₄ alkylsulfonyloxy, C₂-C₄ alkylcarbonyl, C₂-C₆ alkoxycarbonyl, C₂-C₆ alkylaminocarbonyl or C₃-C₆ dialkylaminocarbonyl.

Embodiment D. A compound of Embodiment C wherein
R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 2 substituents independently selected from R³; or U-1, U-2, U-3, U-5, U-20, U-21, U-28, U-36, U-37, U-38, U-39, U-41, U-43, U-50, U-53, U-55, U-58, U-62 or U-74 through U-78;
J is J-4, J-5, J-40, J-41, J-63 through J-69, J-73 or J-77 through J-85;
each R³ is independently halogen, cyano, C₁-C₃ alkyl, C₁-C₃ haloalkyl, C₃-C₆ cycloalkyl, C₃-C₆ halocycloalkyl, C₁-C₃ alkoxy or C₁-C₃ haloalkoxy;
each R^{3a} is independently halogen, C₁-C₃ alkyl, C₁-C₃ haloalkyl, C₁-C₃ alkoxy or C₁-C₃ haloalkoxy;
R^{4a} is H or methyl;
R⁵ is H, methyl, methoxymethyl, methylcarbonyl or methoxycarbonyl; and
each R⁷ is independently halogen, C₁-C₃ alkyl, C₁-C₃ haloalkyl, C₂-C₃ alkenyl, C₁-C₃ alkoxy, C₁-C₃ haloalkoxy, C₂-C₄ alkylcarbonyl or C₂-C₆ alkoxycarbonyl.

Embodiment E. A compound of Embodiment D wherein
R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 1 substituent selected from R³; or is U-37, U-38, U-39, U-41, U-50, U-53, U-55 or U-58; and
J is J-63 through J-69 or J-73.

Embodiment F. A compound of Formula **1** wherein
R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 1 substituent selected from R³; or 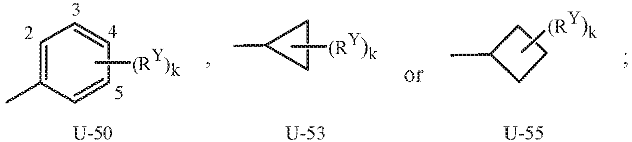
wherein R^{Y} is selected from R^{3a}; and k is 0, 1 or 2;
Z¹ is a direct bond or NR^{4a};
s is 0, 1 or 2 and f is 0;
Z² is a direct bond, NR⁵ or NR⁵CH₂; or CH₂, optionally substituted with up to 2 substituents independently selected from R⁶;
J is 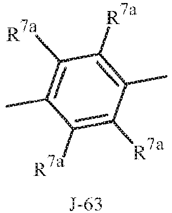
wherein the bond projecting to the left is bonded to Z², and the bond projecting to the right is bonded to the oxadiazole ring in Formula 1;
each R^{7a} is independently H or R⁷;
R³ is halogen, cyano, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₂-C₄ alkylcarbonyl, C₂-C₄ haloalkylcarbonyl or C₂-C₅ alkoxycarbonyl;
each R^{3a} is independently halogen, C₁-C₃ alkyl, C₁-C₃ haloalkyl, C₁-C₃ alkoxy or C₁-C₃ haloalkoxy;
R^{4a} is H, methyl, C₁-C₂ haloalkyl, methoxymethyl, methylsulfonyl, methylcarbonyl or methoxycarbonyl;
R⁵ is H, methyl, methoxymethyl, methylcarbonyl or methoxycarbonyl;
each R⁶ is independently halogen, cyano, nitro, methyl, trifluoromethyl or methoxy; and
each R⁷ is independently halogen, methyl, trifluoromethyl or methoxy.

Embodiment G. A compound of Embodiment F wherein
R¹ is C₁-C₄ alkyl, C₂-C₄ alkylcarbonyl or C₂-C₄ alkoxycarbonyl, each optionally substituted with up to 1 substituent selected from R³;
Z¹ is a direct bond;
Z² is CH₂;
each R^{7a} is H; and
each R³ is independently halogen, C₁-C₃ alkyl, C₁-C₃ haloalkyl, C₁-C₃ alkoxy or C₁-C₃ haloalkoxy.

Embodiment H. A compound of Embodiments F and G wherein
s is 2.

Specific embodiments include compounds of Formula 1 wherein R¹, Z¹, s, f, Z² and J are defined as follows, wherein J is connected to Z² and the oxadiazole ring in a 1,4-orientation. The compounds marked with a star (*) are compounds of the invention. The others are not.

| R¹ | Z¹ | s | f | Z² | J |
|---|---|---|---|---|---|
| PhCH2 | direct bond | 0 | 0 | CH₂ | phenyl |
| Et | direct bond | 0 | 0 | CH₂ | phenyl |
| *n*-Pr | direct bond | 0 | 0 | CH₂ | phenyl |
| *i*-Pr | direct bond | 0 | 0 | CH₂ | phenyl |
| *i*-Bu | direct bond | 0 | 0 | CH₂ | phenyl |
| *n*-Bu | direct bond | 0 | 0 | CH₂ | phenyl |
| CH≡CCH₂ | direct bond | 0 | 0 | CH₂ | phenyl |
| MeOC=OCH₂CH₂ | direct bond | 0 | 0 | CH₂ | phenyl |
| Me | direct bond | 2 | 0 | NH | phenyl ∗ |
| Ph | direct bond | 2 | 0 | NH | phenyl ∗ |
| 2-Cl-Ph | direct bond | 2 | 0 | NH | phenyl ∗ |
| Et | direct bond | 2 | 0 | CH₂ | phenyl ∗ |
| Me | direct bond | 2 | 0 | CH₂ | phenyl ∗ |
| 4-MeO-Ph | NH | 2 | 0 | direct bond | phenyl |
| *c*-Pr | direct bond | 2 | 0 | CH₂ | phenyl |
| Me | NH | 2 | 0 | CH₂ | phenyl |
| CH≡CCH₂ | NH | 2 | 0 | CH₂ | phenyl |
| *c*-Pr | direct bond | 2 | 0 | N(Me)CH₂ | phenyl |
| CH₂=CHCH₂ | NH | 2 | 0 | CH₂ | phenyl |
| *n*-Pr | NH | 2 | 0 | CH₂ | phenyl |
| Me | direct bond | 2 | 0 | N(Me)CH₂ | phenyl |
| Me | direct bond | 2 | 0 | N(C=OMe)CH₂ | phenyl |
| Et | direct bond | 2 | 0 | N(Me)CH₂ | phenyl |
| Et | direct bond | 2 | 0 | N(C=OMe)CH₂ | phenyl |
| Et | direct bond | 2 | 0 | NHCH₂ | phenyl |
| *c*-Pr | direct bond | 2 | 0 | NHCH₂ | phenyl |
| 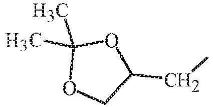 | NH | 2 | 0 | direct bond | phenyl |
| 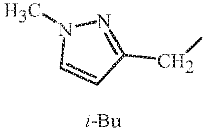 | NH | 2 | 0 | direct bond | phenyl |
| | NH | 2 | 0 | CH₂ | phenyl |
| Me | direct bond | 2 | 0 | N(SO₂Me) | phenyl |
| Et | N(Me) | 2 | 0 | CH₂ | phenyl |
| *c*-Pr | N(Me) | 2 | 0 | CH₂ | phenyl |
| Me | N(OMe) | 2 | 0 | direct bond | phenyl |
| c-Pr | NH | 2 | 0 | CH₂ | phenyl |
| MeOCH₂CH₂ | NH | 2 | 0 | CH₂ | phenyl |
| *i*-Pr | NH | 2 | 0 | CH₂ | phenyl |
| Et | NH | 2 | 0 | CH₂ | phenyl |
| Me | direct bond | 2 | 0 | (CH₂)₃ | phenyl |
| Et | direct bond | 2 | 0 | (CH₂)₃ | phenyl |
| *c*-Pr | direct bond | 2 | 0 | (CH₂)₃ | phenyl |
| Et | direct bond | 2 | 0 | CH(Ph) | phenyl |
| *n*-Pr | direct bond | 2 | 0 | NHCH₂ | phenyl |
| Me | direct bond | 2 | 0 | CH₂CH₂ | phenyl |
| *n*-Pr | direct bond | 2 | 0 | CH₂ | phenyl |
| *i*-Pr | direct bond | 2 | 0 | CH₂ | phenyl |
| *c*-Pr | direct bond | 2 | 0 | CH₂CH₂ | phenyl |
| *i-*Bu | direct bond | 2 | 0 | CH₂ | phenyl |
| *n*-Bu | direct bond | 2 | 0 | CH₂ | phenyl |
| CH≡CCH₂ | direct bond | 2 | 0 | CH₂ | phenyl |
| MeOC(=O)CH₂CH₂ | direct bond | 2 | 0 | CH₂ | phenyl |
| Cl₂CH | direct bond | 1 | 0 | direct bond | phenyl ∗ |
| PhCH₂ | direct bond | 1 | 0 | CH₂ | phenyl |
| Me | direct bond | 1 | 0 | CH₂ | phenyl |
| Et | direct bond | 1 | 0 | CH₂ | phenyl |
| *n*-Pr | direct bond | 1 | 0 | CH₂ | phenyl |
| *i*-Pr | direct bond | 1 | 0 | CH₂ | phenyl |
| *i*-Bu | direct bond | 1 | 0 | CH₂ | phenyl |
| *n*-Bu | direct bond | 1 | 0 | CH₂ | phenyl |
| MeOC(=O)CH₂CH₂ | direct bond | 1 | 0 | CH₂ | phenyl |
| EtOC(=O)CH₂CH₂ | direct bond | 1 | 0 | CH₂ | phenyl |

Of further note are specific embodiments including compounds of Formula **1** wherein R¹, Z¹, s, f, Z² and J are defined as follows, wherein J is connected to Z² and the oxadiazole ring in a 1,4-orientation. The first five compounds belong to the invention, the last two do not

| R¹ | Z¹ | s | f | Z² | J |
|---|---|---|---|---|---|
| Me | direct bond | 2 | 0 | NH | phenyl |
| Ph | direct bond | 2 | 0 | NH | phenyl |
| 2-Cl-Ph | direct bond | 2 | 0 | NH | phenyl |
| Et | direct bond | 2 | 0 | CH₂ | phenyl |
| Me | direct bond | 2 | 0 | CH₂ | phenyl |
| 4-MeO-Ph | NH | 2 | 0 | direct bond | phenyl |
| *c*-Pr | direct bond | 2 | 0 | CH₂ | phenyl |

This invention provides a fungicidal composition comprising a compound of Formula **1** according to claim 1 (including all stereoisomers and at least one other fungicide. Of note as embodiments of such compositions are compositions comprising a compound corresponding to any of the compound embodiments described above.

This invention provides a fungicidal composition comprising a compound of Formula **1** according to claim 1 (including all stereoisomers) (i.e. in a fungicidally effective amount), and at least one additional component selected from the group consisting of surfactants, solid diluents and liquid diluents. Of note as embodiments of such compositions are compositions comprising a compound corresponding to any of the compound embodiments described above.

This invention provides a method for controlling plant diseases caused by fungal plant pathogens comprising applying to the plant or portion thereof, or to the plant seed, a fungicidally effective amount of a compound of Formula **1** according to claim 1 (including all stereoisomers). Of note as embodiments of such methods are methods comprising applying a fungicidally effective amount of a compound corresponding to any of the compound embodiments describe above. Of particular note are embodiments where the compounds are applied as compositions of this invention.

Of note as an embodiment are compounds of Formula **1** that are compounds of Formula **1P** (including all geometric and stereoisomers), *N-*oxides, and salts thereof, and also agricultural compositions containing them and their use as fungicides: 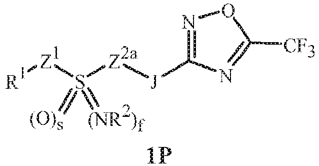 wherein
R¹ is C₁-C₆ alkyl, C₂-C₆ alkenyl, C₂-C₆ alkynyl, C₂-C₅ alkylcarbonyl or C₂-C₅ alkoxycarbonyl, each optionally substituted with up to 3 substituents independently selected from R³; or phenyl, benzyl, phenethyl or naphthalenyl, each optionally substituted with up to 5 substituents independently selected from R^{3a}; or a 3- to 7-membered carbocyclic ring, wherein up to 3 carbon atom ring members are independently selected from C(=O) and C(=S), each ring optionally substituted with up to 5 substituents independently selected from R^{3a}; or
R¹ is a 5- to 6-membered heterocyclic ring, each ring containing ring members selected from carbon atoms and 1 to 4 heteroatoms independently selected from up to 2 O, up to 2 S and up to 4 N atoms, wherein up to 3 carbon atom ring members are independently selected from C(=O) and C(=S), and the sulfur atom ring members are independently selected from S(=O)ᵤ(=NR¹²)ᵥ, each ring optionally substituted with up to 5 substituents independently selected from R^{3a} on carbon atom ring members and R^{3b} on nitrogen atom ring members;
Z¹ is a direct bond, O, NR^{4a} or NR^{4a}NR^{4b};
s and f are each 0, 1 or 2, provided that the sum of s and f is 0, 1 or 2;
each R² is independently H, cyano, C₁-C₃ alkyl or C₁-C₃ haloalkyl;
Z^{2a} is CH₂CH₂CH₂ optionally substituted with up to 4 substituents independently selected from R⁶ or up to 1 substituent selected from R¹³;
J is phenyl optionally substituted with up to 2 substituents independently selected from R⁷; or a 3- to 7-membered carbocyclic ring, wherein up to 3 carbon atom ring members are independently selected from C(=O) and C(=S), each ring optionally substituted with up to 2 substituents independently selected from R⁷; or
J is a 5- to 6-membered heterocyclic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and S(=O)₂, each ring optionally substituted with up to 2 substituents independently selected from R⁷ on carbon atom ring members and R⁸ on nitrogen atom ring members;
each R³ is independently halogen, hydroxy, cyano, -S-C≡N, -SH, amino, nitro, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₁-C₄ alkylthio, C₁-C₄ alkylsulfinyl, C₁-C₄ alkylsulfonyl, C₁-C₄ haloalkylsulfonyl, C₁-C₄ alkylamino, C₂-C₄ dialkylamino, C₂-C₄ alkylcarbonyl, C₂-C₄ haloalkylcarbonyl, C₂-C₅ alkoxycarbonyl, C₂-C₅ haloalkoxycarbonyl, C₂-C₅ alkylaminocarbonyl or C₃-C₅ dialkylaminocarbonyl; or pyridinyl, pyrimidinyl, pyrazolyl, imidazolyl, triazolyl, thiazolyl, oxazolyl, isoxazolyl, oxetanyl, 1,3-dioxolanyl, tetrahydropyranyl, thienyl, furanyl, pyrrolidinyl, isoxazolinyl, tetrahydrofuranyl, piperidinyl, morpholinyl or piperazinyl, each optionally substituted with up to 3 substituents independently selected from R^{3c} on carbon atom ring members and R^{3d} on nitrogen atom ring members;
each R^{3a} and R^{3c} is independently halogen, hydroxy, cyano, amino, nitro, C₁-C₄ alkyl, C₁-C₄ haloalkyl, C₂-C₄ alkenyl, C₂-C₄ alkynyl, C₁-C₄ hydroxyalkyl, C₃-C₆ cycloalkyl, C₄-C₇ cycloalkylalkyl, C₁-C₄ alkoxy, C₁-C₄ haloalkoxy, C₂-C₄ alkenyloxy, C₂-C₄ alkynyloxy, C₂-C₄ alkoxyalkyl, C₂-C₆ alkylcarbonyloxy, C₁-C₄ alkylthio, C₁-C₄ haloalkylthio, C₂-C₆ alkylcarbonylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfonyloxy, C₁-C₄ alkylamino, C₂-C₈ dialkylamino, C₃-C₆ cycloalkylamino, C₂-C₄ alkylcarbonyl, C₂-C₆ alkoxycarbonyl, C₂-C₆ alkylaminocarbonyl, C₃-C₈ dialkylaminocarbonyl or C₃-C₆ trialkylsilyl;
each R^{3b} and R^{3d} is independently C₁-C₃ alkyl, C₁-C₃ alkoxy, C₂-C₃ alkylcarbonyl or C₂-C₃ alkoxycarbonyl;
R^{4a} and R^{4b} are each independently H, hydroxy, cyano, C₁-C₄ alkyl, C₁-C₄ haloalkyl, C₂-C₄ alkenyl, C₂-C₄ haloalkenyl, C₂-C₄ alkynyl, C₂-C₄ haloalkynyl, C₁-C₄ alkoxy, C₁-C₄ haloalkoxy, C₂-C₄ alkoxyalkyl, C₂-C₄ alkylthioalkyl, C₁-C₄ alkylsulfonyl, C₁-C₄ haloalkylsulfonyl, C₂-C₄ alkylsulfinylalkyl, C₂-C₄ alkylsulfonylalkyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl, C₃-C₅ dialkylaminocarbonyl, C₃-C₇ alkylaminocarbonylalky or C₄-C₇ dialkylaminocarbonylalkyl; or
one pair of R¹ and R^{4a} substituents or one pair of R¹ and R^{4b} substituents are taken together with the atoms to which they are attached to form a 5- to 7-membered ring containing ring members selected from carbon atoms and optionally up to 3 heteroatoms independently selected from up to 1 O, up to 1 S and up to 2 N atoms, wherein up to 3 ring members are independently selected from C(=O), C(=S), S(=O) and S(=O)₂, the ring optionally substituted with up to 4 substituents independently selected from R⁹; or
one pair of R^{3a} and R^{4a} substituents or one pair of R^{3a} and R^{4b} substituents are taken together with the atoms to which they are attached to form a 5- to 7-membered ring containing ring members selected from carbon atoms and optionally up to 3 heteroatoms independently selected from up to 1 O, up to 1 S and up to 2 N atoms, wherein up to 3 ring members are independently selected from C(=O), C(=S), S(=O) and S(=O)₂, the ring optionally substituted with up to 4 substituents independently selected from R¹⁰; or
one pair of R^{3b} and R^{4a} substituents or one pair of R^{3b} and R^{4b} substituents are taken together with the nitrogen atoms to which they are attached to form a 5- to 7-membered ring containing ring members selected from carbon atoms and optionally up to 3 heteroatoms independently selected from up to 1 O, up to 1 S and up to 2 N atoms, wherein up to 3 ring members are independently selected from C(=O), C(=S), S(=O) and S(=O)₂, the ring optionally substituted with up to 4 substituents independently selected from R¹¹;
each R⁶ is independently halogen, hydroxy, cyano, nitro, C₁-C₂ alkyl, C₁-C₂ haloalkyl, C₁-C₂ alkoxy or C₁-C₂ haloalkoxy;
each R⁷ is independently halogen, hydroxy, cyano, amino, nitro, C₁-C₄ alkyl, C₁-C₄ haloalkyl, C₂-C₄ alkenyl, C₂-C₄ alkynyl, C₁-C₄ hydroxyalkyl, C₃-C₆ cycloalkyl, C₄-C₇ cycloalkylalkyl, C₁-C₄ alkoxy, C₁-C₄ haloalkoxy, C₂-C₄ alkenyloxy, C₂-C₄ alkynyloxy, C₂-C₄ alkoxyalkyl, C₂-C₄ alkylcarbonyloxy, C₁-C₄ alkylthio, C₁-C₄ haloalkylthio, C₂-C₄ alkylcarbonylthio, C₁-C₄ alkylsulfinyl, C₁-C₄ haloalkylsulfinyl, C₁-C₄ alkylsulfonyl, C₁-C₄ haloalkylsulfonyl, C₁-C₄ alkylsulfinyloxy, C₁-C₄ alkylsulfonyloxy, C₁-C₄ alkylamino, C₂-C₆ dialkylamino, C₃-C₆ cycloalkylamino, C₂-C₄ alkylcarbonyl, C₂-C₆ alkoxycarbonyl, C₂-C₆ alkylaminocarbonyl, C₃-C₆ dialkylaminocarbonyl or C₃-C₆ trialkylsilyl;
each R⁸ is independently C₁-C₃ alkyl;
each R⁹, R¹⁰ and R¹¹ is independently halogen, hydroxy, cyano, nitro, C₁-C₄ alkyl, C₁-C₄ haloalkyl, C₂-C₄ alkenyl, C₂-C₄ haloalkenyl, C₂-C₄ alkynyl, C₂-C₄ haloalkynyl, C₁-C₄ alkoxy, C₁-C₄ haloalkoxy, C₂-C₄ alkoxyalkyl, C₂-C₄ alkylthioalkyl, C₁-C₄ alkylsulfonyl, C₁-C₄ haloalkylsulfonyl, C₂-C₄ alkylsulfinylalkyl, C₂-C₄ alkylsulfonylalkyl, C₂-C₄ alkylcarbonyl, C₂-C₄ haloalkylcarbonyl, C₂-C₅ alkoxycarbonyl, C₃-C₅ alkoxycarbonylalkyl, C₂-C₅ alkylaminocarbonyl or C₃-C₅ dialkylaminocarbonyl;
each R¹² is independently H, cyano, C₁-C₃ alkyl or C₁-C₃ haloalkyl;
each u and v are independently 0, 1 or 2 in each instance of S(=O)ᵤ(=NR¹²)ᵥ, provided that the sum of u and v is 0, 1 or 2;
R¹³ is pheny optionally substituted with up to 3 substituents independently selected from R¹⁴; and
each R¹⁴ is independently halogen, hydroxy, cyano, nitro, C₁-C₂ alkyl, C₁-C₂ haloalkyl, C₁-C₂ alkoxy or C₁-C₂ haloalkoxy.

Accordingly, of note is a compound selected from Formula **1P** (including all geometric and stereoisomers), *N-*oxides, and salts thereof, as defined above. Also of note are counterpart embodiments to Embodiments 1 through 107 and Embodiments A through H wherein in said counterpart embodiments "Formula **1"** is replaced by "Formula **1P"** and the scope of said counterpart embodiments does not exceed the scope defined above for Formula **1P.** Examples of combinations of Embodiments 1 through 107 as applied to Formula **1P** are Embodiments AP, BP and CP below.

Embodiment AP. A compound of Formula **1P** wherein
R¹ is C₁-C₄ alkyl, C₂-C₄ alkenyl, C₂-C₄ alkynyl, C₂-C₄ alkylcarbonyl or C₂-C₄ alkoxycarbonyl, each optionally substituted with up to 1 substituent selected from R³; or 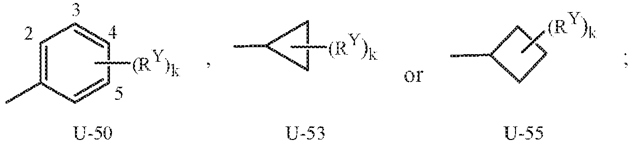
wherein R^{Y} is selected from R^{3a}; and k is 0, 1 or 2;
Z¹ is a direct bond or NR^{4a};
s is 0, 1 or 2 and f is 0; or s is 1 and f is 1; or s is 1 and f is 1;
Z^{2a} is CH₂CH₂CH₂ optionally substituted with up to 4 substituents independently selected from R⁶ or up to 1 substituent selected from R¹³;
J is 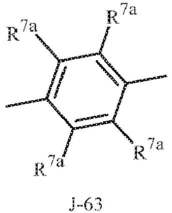
wherein the bond projecting to the left is bonded to Z^{2a}, and the bond projecting to the right is bonded to the oxadiazole ring in Formula 1;
each R^{7a} is independently H or R⁷;
R³ is halogen, cyano, C₁-C₄ alkyl, C₁-C₄ haloalkyl, C₃-C₆ cycloalkyl, C₃-C₆ halocycloalkyl, C₁-C₄ alkoxy, C₁-C₄ haloalkoxy, C₂-C₄ alkylcarbonyl, C₂-C₄ haloalkylcarbonyl or C₂-C₅ alkoxycarbonyl;
each R^{3a} is independently halogen, C₁-C₃ alkyl, C₁-C₃ haloalkyl, C₁-C₃ alkoxy or C₁-C₃ haloalkoxy;
R^{4a} is H, methyl, C₁-C₂ haloalkyl, methoxymethyl, methylsulfonyl, methylcarbonyl or methoxycarbonyl;
each R⁶ is independently halogen, cyano, nitro, methyl, trifluoromethyl or methoxy; and
each R⁷ is independently halogen, methyl, trifluoromethyl or methoxy.

Embodiment BP. A compound of Embodiment AP wherein
R¹ is C₁-C₄ alkyl, C₂-C₄ alkylcarbonyl or C₂-C₄ alkoxycarbonyl, each optionally substituted with up to 1 substituent selected from R³;
Z¹ is a direct bond;
Z^{2a} is CH₂CH₂CH₂ optionally substituted with up to 2 substituents independently selected from R⁶ or up to 1 substituent selected from R¹³;
each R^{7a} is H; and
R³ is halogen, C₁-C₃ alkyl, C₁-C₃ haloalkyl, C₁-C₃ alkoxy or C₁-C₃ haloalkoxy.

Embodiment CP. A compound of Embodiments AP and BP wherein
s is 2; and
Z^{2a} is CH₂CH₂CH₂.

Specific embodiments include compounds of Formula **1P** wherein R¹, Z¹, s, f, Z² and J are defined as follows, wherein J is connected to Z² and the oxadiazole ring in a 14-orientation:

| R¹ | Z¹ | s | f | Z² | J |
|---|---|---|---|---|---|
| Me | direct bond | 2 | 0 | (CH₂)₃ | phenyl |
| Et | direct bond | 2 | 0 | (CH₂)₃ | phenyl |
| *c*-Pr | direct bond | 2 | 0 | (CH₂)₃ | phenyl |

Also of note is a fungicidal composition comprising a fungicidally effective amount of a compound of Formula **1P** (including all geometric and stereoisomers, *N*-oxides, and salts thereof) or any one of counterpart embodiments that are embodiment counterparts to Embodiments 1 through 107 and Embodiments A through H (e.g., Embodiment AP, BP and CP), and at least one additional component selected from the group consisting of surfactants, solid diluents and liquid diluents. Also of note is a method for controlling plant diseases caused by fungal plant pathogens comprising applying to the plant or portion thereof, or to the plant seed, a fungicidally effective amount of a compound of Formula **1P** (including all geometric and stereoisomers, *N-*oxides, and salts thereof) or any one of said counterpart embodiments. Of particular note are embodiments where the compounds of Formula **1P** are applied as compositions of this invention.

Also of note as an embodiment of this invention are compounds of Formula 1 wherein Z¹ is O, NR^{4a} or NR^{4a}NR^{4b} and Z² is O, S, NR⁵ or NR⁵CH₂; or CH₂, CH₂CH₂ or CH₂CH₂CH₂, each optionally substituted with up to 4 substituents independently selected from R⁶ or up to 1 substituent selected from R¹³. Of further note as an embodiment are compounds of Formula **1** wherein Z¹ is NR^{4a} and Z² is NR⁵ or NR⁵CH₂; or CH₂, CH₂CH₂ or CH₂CH₂CH₂.

One or more of the following methods and variations as described in Schemes 1-7 can be used to prepare the compounds of Formula **1.** The definitions of R¹, Z¹, R², Z², J, s and f in the compounds of Formulae **1-7** below are as defined above in the Summary of the Invention unless otherwise noted. Compounds of Formulae **1a** through **1d** are various subsets of Formula **1,** and all substituents for Formulae **1a** through **1d** are as defined above for Formula **1** unless otherwise noted.

As shown in Scheme 1, compounds of Formula **1** can be prepared by reacting amide oximes of Formula **2** with trifluoroacetic anhydride (TFAA) or an equivalent. The reaction of Scheme 1 can be carried out without solvent other than the compounds of Formula **2** and TFAA. More typically the reaction is conducted in a liquid phase with a solvent such as tetrahydrofuran or toluene at a temperature between about 0 to 100 °C, and optionally in the presence of a base such as pyridine or trimethylamine. Preparation of oxadiazole rings by this method and others are known in the art; see, for example, Comprehensive Heterocyclic Chemistry, Vol. 6, Part 4B, pages 365-391, Kevin T. Potts editor, Pergamon Press, New York, 1984**.** The method of Scheme 1 is also illustrated in present Example 1 Step C, Example 2, Step C and Example 3, Step B.

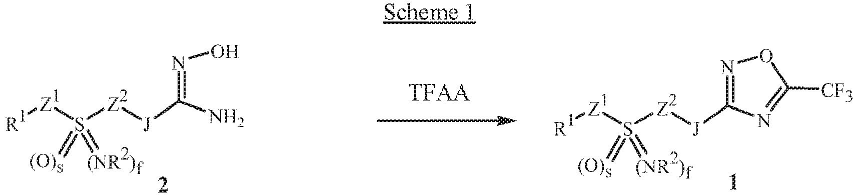

As shown in Scheme 2, oximes of Formula **2** can be prepared from corresponding nitriles of Formula **3** and hydroxylamine or a hydroxylamine salt (e.g., hydroxylamine hydrochloride) in a solvent such as ethanol or methanol at temperatures ranging from about 0 to 80 °C. A base is needed to liberate the hydroxylamine from its salt. Suitable bases include, but are not limited to, sodium hydroxide, sodium bicarbonate or sodium carbonate. For reaction conditions, see present Example 1, Step B, Example 2, Step B and Example 3, Step A. Intermediates of Formula **3** are readily prepared by methods known in the art, including, for example, coupling of sulfinic acid sodium salts and organic halides to provide sulfones as described in Organic Letters 2002, 4(25), 4423-4425. Copper-catalyzed cross-coupling methods are described in Journal of Organic Chemistry 2005, 70(7), 2696-2700 and Journal of Organic Chemistry 2013, 78(23), 12194-12201. Sulfonamides of Formula 3 (i.e. Formula **3** wherein Z¹ is NR^{4a} or NR^{4a}NR^{4b}) can be prepared by reacting amines with sulfonyl chlorides as described in Journal of Organic Chemistry 2009, 74(24), 9287-9291. Present Example 1, Step A illustrates the preparation of a sulfone of Formula **3;** and Example 2, Step A illustrates the preparation of a sulfonamide of Formula **3.**

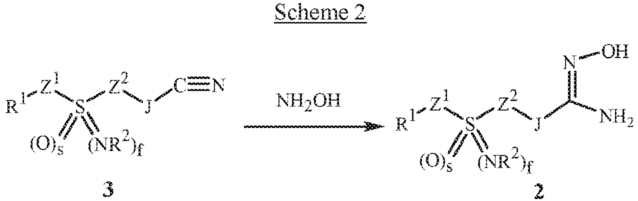

Compounds of Formula **1a** (i.e. Formula **1** wherein s is 1 or 2 and f is 0) can be prepared by oxidation of corresponding compounds of Formula **1b** (i.e. Formula 1 wherein s is 0 and f is 0), as shown in Scheme 3. In a typical procedure, an oxidizing agent in an amount ranging from about 1 to 4 equivalents, depending on the oxidation state of the desired product, is added to a compound of Formula **1b** in a solvent. Useful oxidizing agents include Oxone^{®} (potassium peroxymonosulfate) and *m*-chloroperbenzoic acid. The solvent is selected with regard to the oxidizing agent employed. Ethanol or aqueous acetone is preferably used with Oxone^{®}, and dichloromethane is generally preferable with *m*-chloroperbenzoic acid. Useful reaction temperatures typically range from about 0 to 80 °C. Reaction conditions for oxidizing sulfides to sulfoxides and sulfones are described in The Synthesis of Sulphones, Sulphoxides and Cyclic Sulphides, Eds. S. Patai and Z. Rappoport, John Wiley & Sons, New York, 1994**.** The method of Scheme 3 is also illustrated in present Examples 4 and 5.

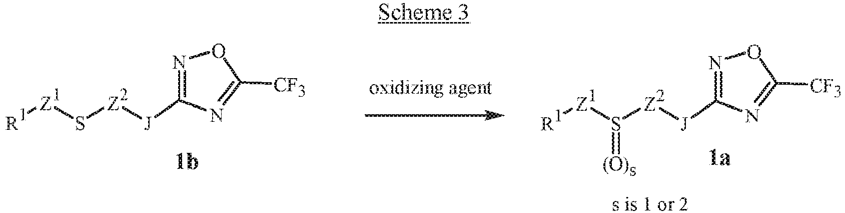

As shown in Scheme 4, sulfilimines of Formula **1c** (i.e. Formula **1** wherein s is 0 and f is 1) can be prepared by reacting compounds of Formula **1b** (i.e. Formula **1** wherein s is 0 and f is 0) and an amine of formula R²NH₂. The reaction is carried out in the presence of a suitable oxidizing agent such as *N*-bromosuccinimide and a base such as potassium *tert-*butoxide in a solvent such as methanol, at a temperature ranging from about 0 to 60 °C. A variety of general procedures have been reported in the literature for the preparation of sulfilimines; see, for example, The Chemistry of Sulfilimines, T. L. Gilchrist and C. J. Moody, Chemical Reviews 1977, 77(3), pages 409-435 and Organic Chemistry of Sulfur, Ed. S. Oae, Plenum Press, New York, 1977, pages 383-471. The method of Scheme 4 is also illustrated in present Example 6.

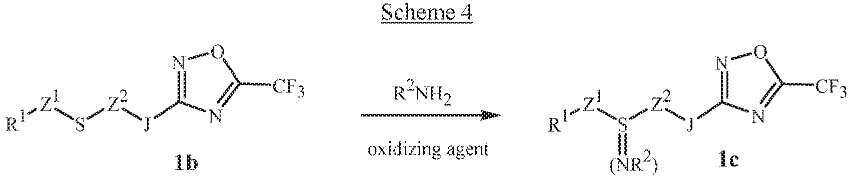

In a manner analogous to the method of Scheme 3, compounds of Formula **1d** (i.e. Formula **1** wherein s is 1 and f is 1) can be prepared by reaction of compounds of Formula **1c** (i.e. Formula **1** wherein s is 0 and f is 1) and a suitable oxidizing agent in the presence of a solvent, as shown in Scheme 5. Sulfoximines are disclosed in a variety of published references including, The Sulfoximines, by P. D. Kennewell and J. B. Taylor, in Chemical Society Reviews 1975, Vol. 4, Issue 2, pages 189-209. The method of Scheme 5 is also illustrated in present Example 7.

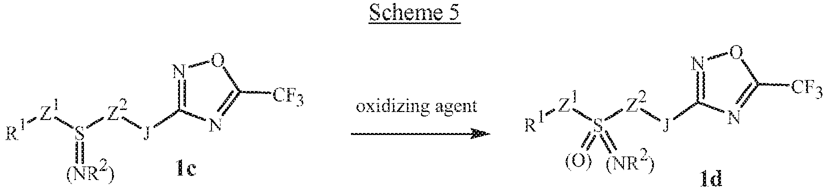

As shown in Scheme 6, compounds of Formula **1** can also be prepared from compounds of Formulae **4** and **5** wherein A¹ and A² are suitable functional groups, which when subjected to the appropriate reaction conditions form the desired compound of Formula 1. The A¹ and A² functional groups are selected from, but not limited to, hydrogen, halogen, alkyl, haloalkyl, carboxyl, amino, aminoalkyl, nitro, hydroxy, hydroxyalkyl, thiol, thioalkyl, alkylthio, chlorosulfonyl, chlorosulfonylalkyl, and the like. Suitable reaction conditions include, for example, oxidations, reductions, alkylations, aminations and sulfonylation. For example, reaction of a compound of Formula **4** wherein A¹ is Cl with a compound of Formula **5** wherein A² is OH will give a compound of Formula **1** wherein Z² is O (as illustrated in present Example 8). Simlarly, reaction of a compound of Formula **4** wherein A¹ is Cl with a compound of Formula 5 wherein A² is NH₂ will give a compound of Formula **1** wherein Z² is NH (as illustrated in present Example 9). One skilled in the art can easily determine the appropriate functional group needed for A¹ and A² for the construction of the desired compound of Formula 1. 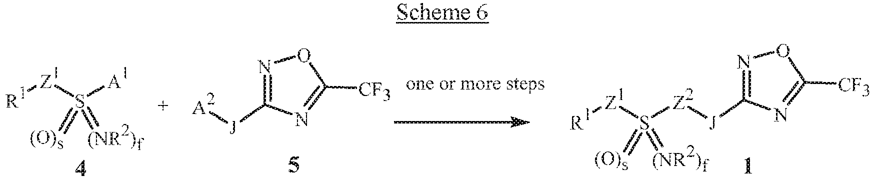
wherein A¹ and A² are functional groups
cable of forming a compound of Formula 1

As shown in Scheme 7, compounds of Formula **5** can be prepared by reaction of nitriles of Formula **6** with hydroxylamine to give the amide oximes of Formula **7** using conditions analogous to those described in Scheme 2. Treatment of compounds of Formula **7** with trifluoroacetic anhrdride, analogous to the transformation depicted in Scheme 1, provides compounds of Formula **5.** The nitriles of Formula **6** are known or can easily be prepared by one skilled in the art.

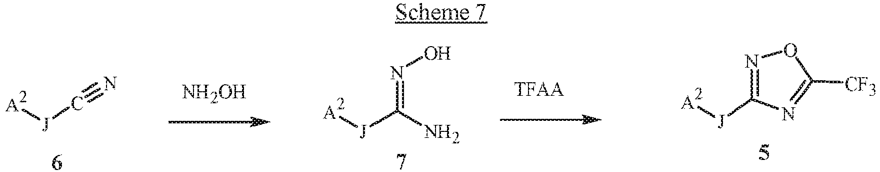

It is recognized that some reagents and reaction conditions described above for preparing compounds of Formula **1** may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (for references see, for example, T. W. Greene and P. G. M. Wuts, Protective Groups in Organic Synthesis, 2nd ed.; Wiley: New York, 1991). One skilled in the art will recognize that, in some cases, after the introduction of a given reagent as it is depicted in any individual scheme above, it may be necessary to perform additional routine synthetic steps not described in detail to complete the synthesis of compounds of Formula **1.** One skilled in the art will also recognize that it may be necessary to perform a combination of the steps illustrated in the above schemes in an order other than implied by the particular sequence presented to prepare the compounds of Formula **1.**

One skilled in the art will also recognize that compounds of Formula 1 and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. Steps in the following Examples illustrate a procedure for each step in an overall synthetic transformation, and the starting material for each step may not have necessarily been prepared by a particular preparative run whose procedure is described in other Examples or Steps. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; ¹⁹F NMR spectra are reported in ppm using trichlorofluoromethane as the reference; "s" means singlet, "d" means doublet, "m" means multiplet, "br s" means broad singlet and "br m" means broad multiplet.

Examples 1, 4, 5, 8 and 9 correspond to compounds of the invention. Examples 2, 3, 6 and 7 do not.

### EXAMPLE 1

### Preparation of 3-[4-[(methylsulfonyl)methyl]phenyl]-5-(trifluoromethyl)-1,2,4-oxadiazole (Compound 124)

### Step A: Preparation of 4-[(methylsulfonyl)methyl]benzonitrile

A mixture of sodium methanesulfinate (2.55 g, 25 mmol) and 4-(bromomethyl)benzonitrile (3.92 g, 20 mmol) in *N*,*N*-dimethylformamide (20 mL) was stirred at room temperature for 16 h, and then added to rapidly stirred ice water. The resulting solid precipitate was collected by filtration, washed with water and dried to provide the title compound as a white solid (3.41 g).
¹H NMR (CDCl₃): δ 2.84 (s, 3H), 4.31 (s, 2H), 7.55 (m, 2H), 7.72 (m, 2H).

### Step B: Preparation of N-hydroxy-4-[(methylsulfonyl)methyl]-benzenecarboximidamide

A mixture of 4-[(methylsulfonyl)methyl]benzonitrile (i.e. the product of Step A) (3.30 g, 16.9 mmol) and hydroxylamine (50% aqueous solution, 3.0 mL, 48 mmol) in ethanol (50 mL) was heated at 50 °C for 4 h, and then stirred at room temperature overnight. The resulting solid precipitate was collected by filtration, washed with ethanol and dried to provide the title compound as a white sold (3.50 g).
¹H NMR (DMSO-*d*₆): δ 2.90 (s, 3H), 4.50 (s, 2H), 5.82 (br s, 2H), 7.40 (m, 2H), 7.69 (m, 2H), 9.68 (s, 1H).

### Step C: Preparation of 3-[4-[(methylsulfonyl)methyl]phenyl]-5-(trifluoromethyl)-1,2,4-oxadiazole

To mixture of *N*-hydroxy-4-[(methylsulfonyl)methyl]benzenecarboximidamide (i.e. the product of Step B) (3.45 g, 15.3 mmol) and pyridine (1.5 mL, 18.5 mmol) in tetrahydrofuran (50 mL) at 0 °C was added a solution of trifluoroacetic anhydride (2.5 mL, 18 mmol) in tetrahydrofuran (20 mL) dropwise over 30 minutes. The reaction mixture was heated at 70 °C for 3 h, cooled to ambient temperature, and concentrated under reduced pressure. The resulting material was diluted with ethyl acetate, washed with water, aqueous hydrochloric acid solution (1 N), saturated aqueous sodium bicarbonate solution, saturated aqueous sodium chloride solution, dried over magnesium sulfate, filtered and concentrated under reduced pressure to a white solid (4.28 g). The solid was crystallized from ethanol to provide the title compound, a compound of the present invention, as a white solid melting at 138-141 °C.
¹H NMR (CDCl₃) δ 2.83 (s, 3H), 4.34 (s, 2H), 7.60 (m, 2H), 8.19 (m, 2H).
¹⁹F NMR (CDCl₃) δ -65.33.

### EXAMPLE 2

### Preparation of N-methyl-4-[5-(trifluoromethyl)-1,2,4-oxadiazol-3-yl]benzenesulfonamide (Compound 40)

### Step A: Preparation of 4-cyano-N-methylbenzenesulfonamide

To a mixture methylamine (2 M in tetrahydrofuran, 70 mL, 140 mmol) at 0 °C was added 4-cyanobenzenesulfonyl chloride (10.08 g, 50 mmol) portionwise. The reaction mixture was allowed to warm to ambient temperature, stirred for 2 h, and then filtered. The filtrate was concentrated under reduced pressure and the resulting material diluted with ethyl acetate. The ethyl acetate mixture was washed with aqueous hydrochloric acid solution (1 N) and saturated aqueous sodium chloride solution, dried over magnesium sulfate, filtered and concentrated under reduced pressure to provide a yellow solid (8.93 g). The solid was slurried in diethyl ether and filtered to provide the title compound as a white solid (8.4 g).
¹H NMR (CDCl₃): δ 2.72 (d, 3H), 4.70-4.80 (br m, 1H), 7.84 (m, 2H), 7.99 (m, 2H).

### Step B: Preparation of N-hydroxy-4-[(methylamino)sulfonyl]-benzenecarboximidamide

A mixture of 4-cyano-N-methylbenzenesulfonamide (i.e. the product of Step A) (8.4 g, 42.8 mmol) and hydroxylamine (50% aqueous solution, 5.0 mL, 81 mmol) in ethanol (50 mL) was stirred at room temperature for 16 h. The resulting solid was filtered, washed with ethanol and dried to provide the title compound as a white solid (8.50 g).
¹H NMR (DMSO-*d*₆): δ 2.42 (d, 3H), 5.95 (br s, 2H), 7.48 (br s, 1H), 7.76 (m, 2H), 7.87 (m, 2H), 9.91 (s, 1H).

### Step C: Preparation of N-methyl-4-[5-(trifluoromethyl)-1,2,4-oxadiazol-3-yl]benzenesulfonamide

To mixture of *N*-hydroxy-4-[(methylamino)sulfonyl]benzenecarboximidamide (i.e. the product of Step B) (8.5 g, 37 mmol) and pyridine (3.6 mL, 44 mmol) in tetrahydrofuran (50 mL) at 0 °C was added trifluoroacetic anhydride (6.1 mL, 44 mmol) in tetrahydrofuran (20 mL) dropwise over 30 minutes. The reaction mixture was heated at 70 °C for 3 h, cooled to ambient temperature, and concentrated under reduced pressure. The resulting material was diluted with ethyl acetate, washed with water, aqueous hydrochloric acid solution (1 N), saturated aqueous sodium bicarbonate solution, saturated aqueous sodium chloride solution, dried over magnesium sulfate, filtered and concentrated under reduced pressure to a white solid (10.35 g). The solid was crystallized from ethanol to provide the title compound, a compound of the present invention, as a white solid melting at 139-141 °C. ¹H NMR (CDCl₃) δ 2.74 (d, 3H), 4.65-4.72 (br s, 1H), 8.04 (m, 2H), 8.30 (m, 2H).
¹⁹F NMR (CDCl₃) δ -65.30.

### EXAMPLE 3

### Preparation of 3-[4-(methylthio)phenyl]-5-(trifluoromethyl)-1,2,4-oxadiazole (Compound 116)

### Step A: Preparation of N-hydroxy-4-(methylthio)benzenecarboximidamide

A mixture of 4-(methylthio)benzonitrile (6.0 g, 40 mmol) and hydroxylamine (50% aqueous solution, 5.0 mL, 81 mmol) in ethanol (100 mL) was stirred at room temperature for 16 h. The reaction mixture was concentrated under reduced pressure and the resulting material was diluted with acetonitrile (50 mL) and concentrated under reduced pressure (2x) to provide the title compound as a white solid (7.38 g).
¹H NMR (DMSO-*d*₆): δ 2.48 (s, 3H), 5.77 (br s, 2H), 7.24 (m, 2H), 7.61 (m, 2H), 9.58 (s, 1H).

### Step B: Preparation of 3-[4-(methylthio)phenyl]-5-(trifluoromethyl)-1,2,4-oxadiazole

To a mixture of *N*-hydroxy-4-(methylthio)benzenecarboximidamide (i.e. the product of Step A) (7.3 g, 40 mmol) and pyridine (3.9 mL, 48 mmol) in tetrahydrofuran (50 mL) at 0 °C was added trifluoroacetic anhydride (6.7 mL, 48 mmol) in tetrahydrofuran (20 mL) dropwise over 15 minutes. The reaction mixture was heated at 70 °C for 3 h, cooled to ambient temperature, and concentrated under reduced pressure. The resulting material was diluted with diethyl ether, washed with water, aqueous hydrochloric acid solution (1 N), saturated aqueous sodium bicarbonate solution, saturated aqueous sodium chloride solution, dried over magnesium sulfate, filtered and concentrated under reduced pressure to provide the title compound, a compound of the present invention, as a white sold (9.71 g).
¹H NMR (CDCl₃) δ 2.54 (s, 3H), 7.32 (m, 2H), 8.00 (m, 2H).
¹⁹F NMR (CDCl₃) δ -65.37.

### EXAMPLE 4

### Preparation of 3-[4-(methylsulfinyl)phenyl]-5-(trifluoromethyl)-1,2,4-oxadiazole (Compound 118)

To a mixture of 3-[4-(methylthio)phenyl]-5-(trifluoromethyl)-1,2,4-oxadiazole oxadiazole (i.e. the product of Example 3) (3.90 g, 15 mmol) in dichloromethane (50 mL) at 0°C was added *m*-chloroperbenzoic acid (77% assay, 3.37 g, 15 mmol). The reaction mixture was allowed to slowly warm to ambient temperature and stirred overnight. An aqueous solution of sodium metabisulfite (1 M, 10 mL) was added to the reaction mixture. The reaction mixture was vigorously stirred for 15 minutes, and then saturated aqueous sodium bicarbonate solution was added and stirring was continued for 30 minutes. The organic layer was separated, dried over magnesium sulfate, filtered and concentrated under reduced pressure to provide a white solid (4.35 g). The solid was crystalized from ethanol to provide the title compound, a compound of the present invention, as a white solid melting at at 110-112 °C.
¹H NMR (CDCl₃) δ 2.79 (s, 3H), 7.81 (m, 2H), 8.30 (m, 2H).
¹⁹F NMR (CDCl₃) δ -65.30.

### EXAMPLE 5

### Preparation of 3-[4-(methylsulfonyl)phenyl]-5-(trifluoromethyl)-1,2,4-oxadiazole (Compound 117)

To a mixture of 3-[4-(methylthio)phenyl]-5-(trifluoromethyl)-1,2,4-oxadiazole (i.e. the product of Example 3) (3.90 g, 15 mmol) in dichloromethane (100 mL) at 0 °C was added m-chloroperbenzoic acid (77% assay, 6.72 g, 30 mmol). The reaction mixture was allowed to slowly warm to ambient temperature and stirred overnight. An aqueous solution of sodium metabisulfite (1 M, 10 mL) was added to the reaction mixture. The reaction mixture was vigorously stirred for 15 minutes, and then saturated aqueous sodium bicarbonate solution was added and stirring was continued for 30 minutes. The organic layer was separated, dried over magnesium sulfate, filtered and concentrated under reduced pressure to provide a white solid (4.37 g). The solid was crystalized from i-propanol to provide the title compound, a compound of the present invention, as a white solid melting at at 152-154 °C.
¹H NMR (CDCl₃) δ 3.12 (s, 3H), 8.11 (m, 2H), 8.37 (m, 2H).
¹⁹F NMR (CDCl₃) δ -65.26.

### EXAMPLE 6

### Preparation of N-[methyl[4-[5-(trifluoromethyl)-1,2,4-oxadiazol-3-yl]phenyl]-λ⁴-sulfanylidene]cyanamide (Compound 80)

A mixture of 3-[4-(methylthio)phenyl]-5-(trifluoromethyl)-1,2,4-oxadiazole (i.e. the product of Example 3) (1.04 g, 4 mmol), cyanamide (218 mg, 5.2 mmol), potassium *tert-*butoxide (538 mg, 4.8 mmol) and *N*-bromosuccinamide (1.07 g, 6.0 mmol) in methanol (20 mL) was stirred at ambient temperature for 2 h, and then concentrated under reduce pressure. The resulting material was purified by silica gel medium pressure liquid chromatography (eluting with ethyl acetate) to provide the title compound, a compound of the present invention, as a colorless oil (820 mg).
¹H NMR (CDCl₃) δ 3.10 (s, 3H), 7.99 (m, 2H), 8.38 (m, 2H).
¹⁹F NMR (CDCl₃) δ -65.26.

### EXAMPLE 7

### Preparation of N-[methyloxido[4-[5-(trifluoromethyl)-1,2,4-oxadiazol-3-yl]phenyl]-λ⁴-sulfanylidene]cyanamide (Compound 87)

A mixture of *N*-[methyl[4-[5-(trifluoromethyl)-1,2,4-oxadiazol-3-yl]phenyl]-λ⁴-sulfanylidene]cyanamide (i.e. the product of Example 6) (600 mg, 2 mmol) and m-chloroperbenzoic acid (77% assay, 678 mg, 3 mmol) in dichloromethane (20 mL) was stirred at ambient temperature overnight. An aqueous solution of sodium metabisulfilte (10%, 10 mL) and aqueous sodium hydroxide solution (1 N) was added to the reaction mixture. The reaction mixture was stirred vigorously for 15 minutes, and then the organic layer was separated, dried over magnesium sulfate, filtered and concentrated under reduced pressure. The resulting material was purified by silica gel medium pressure liquid chromatography (eluting with a gradient of 0 to 100% ethyl acetate in hexanes) to provide the title compound, a compound of the present invention, as a white solid (50 mg).
¹H NMR (CDCl₃) δ 3.41 (s, 3H), 8.19 (m, 2H), 8.45 (m, 2H).
¹⁹F NMR (CDCl₃) δ -65.19.

### EXAMPLE 8

### Preparation of 4-[5-(trifluoromethyl)-1 ,2,4-oxadiazol-3-yl]phenylmethanesulfonate (Compound 108)

To a mixture of 4-[5-(trifluoromethyl)-1,2,4-oxadiazol-3-yl]phenol (290 mg, 1.26 mmol) and trimethylamine (0.25 mL, 1.8 mmol) in dichloromethane (20 mL) was added methanesulfonyl chloride (0.10 mL, 1.29 mmol). The reaction mixture stirred at ambient temperature for three days, and then diluted with dichloromethane, washed with aqueous citric acid solution, dried over magnesium sulfate, filtered and concentrated under reduced pressure. The resulting material was purified by silica gel medium pressure liquid chromatography (eluting with a gradient of 0 to 30% ethyl acetate in hexanes) to provide the title compound, a compound of the present invention, as a white solid (220 mg).
¹H NMR (CDCl₃): δ 3.22 (s, 3H), 7.48 (m, 2H), 8.20 (m, 2H).
¹⁹F NMR (CDCl₃): δ -65.33.

### EXAMPLE 9

### Preparation of N-[4-[5-(trifluoromethyl)-1,2,4-oxadiazol-3-yl]phenyl]methanesulfonamide (Compound 26)

To mixture of 4-[5-(trifluoromethyl)-1,2,4-oxadiazol-3-yl]aniline (229 mg, 1.3 mmol) in pyridine (5 mL) was added methanesulfonyl chloride (0.50 mL, 6.5 mmol). The reaction mixture was heated at reflux overnight, and then poured into cold aqueous hydrochloric acid (2 M) and extracted with ethyl acetate. The extract was washed with aqueous hydrochloric acid solution (1 M) and saturated aqueous sodium chloride solution, dried over magnesium sulfate, filtered and concentrated under reduced pressure. The resulting material was purified by silica gel medium pressure liquid chromatography (eluting with a gradient of 0 to 50% ethyl acetate in hexanes) to provide the title compound, a compound of the present invention, as an off-white solid (260 mg).
¹H NMR (CDCl₃): δ 3.13 (s, 3H), 6.99 (br s, 1H), 7.34 (m, 2H), 8.21 (m, 2H).
¹⁹F NMR (CDCl₃): δ -65.35.

By the procedures described herein, together with methods known in the art, the following compounds of Tables 1A to 392A can be prepared. The following abbreviations are used in the Tables: *t* means tertiary, s means secondary, *n* means normal, *i* means iso, *c* means cyclo, Me means methyl, Et means ethyl, Pr means propyl, *i*-Pr means isopropyl, *c*-Pr means cyclopropyl, Bu means butyl, *c*-Bu means cyclobutyl, CN means cyano and Ph means phenyl.

**Table 1A**

| 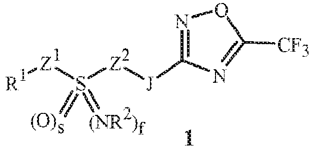 | | | | | | | | | |
|---|---|---|---|---|---|---|---|---|---|
| The definition of J in the following Table is shown in Exhibit B above. Substituents R^{7a} and R⁸ attached to the J are hydrogen unless otherwise noted. | | | | | | | | | |
| Z² is CH₂ and J is J-63. | | | | | Z² is CH₂ **and** J is J-63. | | | | |
| R¹ | Z¹ | s | f | R² | R¹ | Z¹ | s | f | R² |
| Me | bond | 0 | 0 | - | Cl₃C | bond | 0 | 1 | Me |
| Et | bond | 0 | 0 | - | N≡CCH₂ | bond | 0 | 1 | Me |
| *n*-Pr | bond | 0 | 0 | - | N≡CCH₂CH₂ | bond | 0 | 1 | Me |
| *i*-Pr | bond | 0 | 0 | - | MeOCH₂CH₂ | bond | 0 | 1 | Me |
| *c*-Pr | bond | 0 | 0 | - | MeOCOCH₂ | bond | 0 | 1 | Me |
| *n*-Bu | bond | 0 | 0 | - | MeNHCOCH₂ | bond | 0 | 1 | Me |
| *i*-Bu | bond | 0 | 0 | - | Me₂NCOCH₂ | bond | 0 | 1 | Me |
| *t*-Bu | bond | 0 | 0 | - | Ph | bond | 0 | 1 | Me |
| *c*-Bu | bond | 0 | 0 | - | 2-Cl-Ph | bond | 0 | 1 | Me |
| *c*-pentyl | bond | 0 | 0 | - | 4-Cl-Ph | bond | 0 | 1 | Me |
| *c*-hexyl | bond | 0 | 0 | - | Bn | bond | 0 | 1 | Me |
| ClCH₂ | bond | 0 | 0 | - | Ph-CH₂CH₂ | bond | 0 | 1 | Me |
| ClCH₂CH₂ | bond | 0 | 0 | - | 2-thienyl | bond | 0 | 1 | Me |
| Cl₂CH | bond | 0 | 0 | - | 3-thienyl | bond | 0 | 1 | Me |
| Cl₃C | bond | 0 | 0 | - | 2-furanyl | bond | 0 | 1 | Me |
| N≡CCH₂ | bond | 0 | 0 | - | 3-furanyl | bond | 0 | 1 | Me |
| N≡CCH₂CH₂ | bond | 0 | 0 | - | 2-pyridinyl | bond | 0 | 1 | Me |
| MeOCH₂CH₂ | bond | 0 | 0 | - | Me | bond | 0 | 1 | CN |
| MeOCOCH₂ | bond | 0 | 0 | - | Et | bond | 0 | 1 | CN |
| MeNHCOCH₂ | bond | 0 | 0 | - | *n*-Pr | bond | 0 | 1 | CN |
| Me₂NCOCH₂ | bond | 0 | 0 | - | *i*-Pr | bond | 0 | 1 | CN |
| Ph | bond | 0 | 0 | - | *c*-Pr | bond | 0 | 1 | CN |
| 2-Cl-Ph | bond | 0 | 0 | - | *n*-Bu | bond | 0 | 1 | CN |
| 4-Cl-Ph | bond | 0 | 0 | - | *i*-Bu | bond | 0 | 1 | CN |
| Bn | bond | 0 | 0 | - | *t*-Bu | bond | 0 | 1 | CN |
| Ph-CH₂CH₂ | bond | 0 | 0 | - | c-Bu | bond | 0 | 1 | CN |
| 2-thienyl | bond | 0 | 0 | - | *c*-pentyl | bond | 0 | 1 | CN |
| 3-thienyl | bond | 0 | 0 | - | *c*-hexyl | bond | 0 | 1 | CN |
| 2-furanyl | bond | 0 | 0 | - | ClCH₂ | bond | 0 | 1 | CN |
| 3-furanyl | bond | 0 | 0 | - | ClCH₂CH₂ | bond | 0 | 1 | CN |
| 2-pyridinyl | bond | 0 | 0 | - | Cl₂CH | bond | 0 | 1 | CN |
| Me | bond | 1 | 0 | - | Cl₃C | bond | 0 | 1 | CN |
| Et | bond | 1 | 0 | - | NCCH₂ | bond | 0 | 1 | CN |
| *n*-Pr | bond | 1 | 0 | - | NCCH₂CH₂ | bond | 0 | 1 | CN |
| *i*-Pr | bond | 1 | 0 | - | MeOCH₂CH₂ | bond | 0 | 1 | CN |
| *c*-Pr | bond | 1 | 0 | - | MeOCOCH₂ | bond | 0 | 1 | CN |
| *n*-Bu | bond | 1 | 0 | - | MeNHCOCH₂ | bond | 0 | 1 | CN |
| i-Bu | bond | 1 | 0 | - | Me₂NCOCH₂ | bond | 0 | 1 | CN |
| *t*-Bu | bond | 1 | 0 | - | Ph | bond | 0 | 1 | CN |
| c-Bu | bond | 1 | 0 | - | 2-Cl-Ph | bond | 0 | 1 | CN |
| *c*-pentyl | bond | 1 | 0 | - | 4-Cl-Ph | bond | 0 | 1 | CN |
| *c*-hexyl | bond | 1 | 0 | - | Bn | bond | 0 | 1 | CN |
| ClCH₂ | bond | 1 | 0 | - | Ph-CH₂CH₂ | bond | 0 | 1 | CN |
| ClCH₂CH₂ | bond | 1 | 0 | - | 2-thienyl | bond | 0 | 1 | CN |
| Cl₂CH | bond | 1 | 0 | - | 3-thienyl | bond | 0 | 1 | CN |
| Cl₃C | bond | 1 | 0 | - | 2-furanyl | bond | 0 | 1 | CN |
| N≡CCH₂ | bond | 1 | 0 | - | 3-furanyl | bond | 0 | 1 | CN |
| N≡CCH₂CH₂ | bond | 1 | 0 | - | 2-pyridinyl | bond | 0 | 1 | CN |
| MeOCH₂CH₂ | bond | 1 | 0 | - | Me | bond | 1 | 1 | H |
| MeOCOCH₂ | bond | 1 | 0 | - | Et | bond | 1 | 1 | H |
| MeNHCOCH₂ | bond | 1 | 0 | - | *n*-Pr | bond | 1 | 1 | H |
| Me₂NCOCH₂ | bond | 1 | 0 | - | *i*-Pr | bond | 1 | 1 | H |
| Ph | bond | 1 | 0 | - | *c*-Pr | bond | 1 | 1 | H |
| 2-Cl-Ph | bond | 1 | 0 | - | *n*-Bu | bond | 1 | 1 | H |
| 4-Cl-Ph | bond | 1 | 0 | - | *i*-Bu | bond | 1 | 1 | H |
| Bn | bond | 1 | 0 | - | *t*-Bu | bond | 1 | 1 | H |
| Ph-CH₂CH₂ | bond | 1 | 0 | - | *c*-Bu | bond | 1 | 1 | H |
| 2-thienyl | bond | 1 | 0 | - | *c*-pentyl | bond | 1 | 1 | H |
| 3-thienyl | bond | 1 | 0 | - | *c*-hexyl | bond | 1 | 1 | H |
| 2-furanyl | bond | 1 | 0 | - | ClCH₂ | bond | 1 | 1 | H |
| 3-furanyl | bond | 1 | 0 | - | ClCH₂CH₂ | bond | 1 | 1 | H |
| 2-pyridinyl | bond | 1 | 0 | - | Cl₂CH | bond | 1 | 1 | H |
| Me | bond | 2 | 0 | - | Cl₃C | bond | 1 | 1 | H |
| Et | bond | 2 | 0 | - | N≡CCH₂ | bond | 1 | 1 | H |
| *n*-Pr | bond | 2 | 0 | - | N≡CCH₂CH₂ | bond | 1 | 1 | H |
| *i*-Pr | bond | 2 | 0 | - | MeOCH₂CH₂ | bond | 1 | 1 | H |
| *c*-Pr | bond | 2 | 0 | - | MeOCOCH₂ | bond | 1 | 1 | H |
| *n*-Bu | bond | 2 | 0 | - | MeNHCOCH₂ | bond | 1 | 1 | H |
| *i*-Bu | bond | 2 | 0 | - | Me₂NCOCH₂ | bond | 1 | 1 | H |
| *t*-Bu | bond | 2 | 0 | - | Ph | bond | 1 | 1 | H |
| *c*-Bu | bond | 2 | 0 | - | 2-Cl-Ph | bond | 1 | 1 | H |
| *c*-pentyl | bond | 2 | 0 | - | 4-Cl-Ph | bond | 1 | 1 | H |
| *c*-hexyl | bond | 2 | 0 | - | Bn | bond | 1 | 1 | H |
| ClCH₂ | bond | 2 | 0 | - | Ph-CH₂CH₂ | bond | 1 | 1 | H |
| ClCH₂CH₂ | bond | 2 | 0 | - | 2-thienyl | bond | 1 | 1 | H |
| Cl₂CH | bond | 2 | 0 | - | 3-thienyl | bond | 1 | 1 | H |
| Cl₃C | bond | 2 | 0 | - | 2-furanyl | bond | 1 | 1 | H |
| N≡CCH₂ | bond | 2 | 0 | - | 3-furanyl | bond | 1 | 1 | H |
| N≡CCH₂CH₂ | bond | 2 | 0 | - | 2-pyridinyl | bond | 1 | 1 | H |
| MeOCH₂CH₂ | bond | 2 | 0 | - | Me | bond | 1 | 1 | Me |
| MeOCOCH₂ | bond | 2 | 0 | - | Et | bond | 1 | 1 | Me |
| MeNHCOCH₂ | bond | 2 | 0 | - | *n*-Pr | bond | 1 | 1 | Me |
| Me₂NCOCH₂ | bond | 2 | 0 | - | *i*-Pr | bond | 1 | 1 | Me |
| Ph | bond | 2 | 0 | - | *c*-Pr | bond | 1 | 1 | Me |
| 2-Cl-Ph | bond | 2 | 0 | - | *n*-Bu | bond | 1 | 1 | Me |
| 4-Cl-Ph | bond | 2 | 0 | - | i-Bu | bond | 1 | 1 | Me |
| Bn | bond | 2 | 0 | - | *t*-Bu | bond | 1 | 1 | Me |
| Ph-CH₂CH₂ | bond | 2 | 0 | - | *c*-Bu | bond | 1 | 1 | Me |

| Z² is CH₂ and J is J-63. | | | | | Z² is CH₂ **and** J is J-63. | | | | |
|---|---|---|---|---|---|---|---|---|---|
| R¹ | Z¹ | s | f | R² | R¹ | Z¹ | s | f | R² |
| 2-thienyl | bond | 2 | 0 | - | *c*-pentyl | bond | 1 | 1 | Me |
| 3-thienyl | bond | 2 | 0 | - | *c*-hexyl | bond | 1 | 1 | Me |
| 2-furanyl | bond | 2 | 0 | - | ClCH₂ | bond | 1 | 1 | Me |
| 3-furanyl | bond | 2 | 0 | - | ClCH₂CH₂ | bond | 1 | 1 | Me |
| 2-pyridinyl | bond | 2 | 0 | - | Cl₂CH | bond | 1 | 1 | Me |
| Me | bond | 0 | 1 | H | Cl₃C | bond | 1 | 1 | Me |
| Et | bond | 0 | 1 | H | N≡CCH₂ | bond | 1 | 1 | Me |
| *n*-Pr | bond | 0 | 1 | H | N≡CCH₂CH₂ | bond | 1 | 1 | Me |
| *i*-Pr | bond | 0 | 1 | H | MeOCH₂CH₂ | bond | 1 | 1 | Me |
| *c*-Pr | bond | 0 | 1 | H | MeOCOCH₂ | bond | 1 | 1 | Me |
| *n*-Bu | bond | 0 | 1 | H | MeNHCOCH₂ | bond | 1 | 1 | Me |
| *i*-Bu | bond | 0 | 1 | H | Me₂NCOCH₂ | bond | 1 | 1 | Me |
| *t*-Bu | bond | 0 | 1 | H | Ph | bond | 1 | 1 | Me |
| *c*-Bu | bond | 0 | 1 | H | 2-Cl-phenyl | bond | 1 | 1 | Me |
| *c*-pentyl | bond | 0 | 1 | H | 4-Cl-Ph | bond | 1 | 1 | Me |
| *c*-hexyl | bond | 0 | 1 | H | Bn | bond | 1 | 1 | Me |
| ClCH₂ | bond | 0 | 1 | H | Ph-CH₂CH₂ | bond | 1 | 1 | Me |
| ClCH₂CH₂ | bond | 0 | 1 | H | 2-thienyl | bond | 1 | 1 | Me |
| Cl₂CH | bond | 0 | 1 | H | 3-thienyl | bond | 1 | 1 | Me |
| Cl₃C | bond | 0 | 1 | H | 2-furanyl | bond | 1 | 1 | Me |
| NCCH₂ | bond | 0 | 1 | H | 3-furanyl | bond | 1 | 1 | Me |
| NCCH₂CH₂ | bond | 0 | 1 | H | 2-pyridinyl | bond | 1 | 1 | Me |
| MeOCH₂CH₂ | bond | 0 | 1 | H | Me | bond | 1 | 1 | CN |
| MeOCOCH₂ | bond | 0 | 1 | H | Et | bond | 1 | 1 | CN |
| MeNHCOCH₂ | bond | 0 | 1 | H | *n*-Pr | bond | 1 | 1 | CN |
| Me₂NCOCH₂ | bond | 0 | 1 | H | *i*-Pr | bond | 1 | 1 | CN |
| Ph | bond | 0 | 1 | H | *c*-Pr | bond | 1 | 1 | CN |
| 2-Cl-Ph | bond | 0 | 1 | H | *n*-Bu | bond | 1 | 1 | CN |
| 4-Cl-Ph | bond | 0 | 1 | H | *i*-Bu | bond | 1 | 1 | CN |
| Bn | bond | 0 | 1 | H | *t*-Bu | bond | 1 | 1 | CN |
| Ph-CH₂CH₂ | bond | 0 | 1 | H | *c*-Bu | bond | 1 | 1 | CN |
| 2-thienyl | bond | 0 | 1 | H | *c*-pentyl | bond | 1 | 1 | CN |
| 3-thienyl | bond | 0 | 1 | H | *c*-hexyl | bond | 1 | 1 | CN |
| 2-furanyl | bond | 0 | 1 | H | ClCH₂ | bond | 1 | 1 | CN |
| 3-furanyl | bond | 0 | 1 | H | ClCH₂CH₂ | bond | 1 | 1 | CN |
| 2-pyridinyl | bond | 0 | 1 | H | Cl₂CH | bond | 1 | 1 | CN |
| Me | bond | 0 | 1 | Me | Cl₃C | bond | 1 | 1 | CN |
| Et | bond | 0 | 1 | Me | NCCH₂ | bond | 1 | 1 | CN |
| *n*-Pr | bond | 0 | 1 | Me | NCCH₂CH₂ | bond | 1 | 1 | CN |
| *i*-Pr | bond | 0 | 1 | Me | MeOCH₂CH₂ | bond | 1 | 1 | CN |
| *c*-Pr | bond | 0 | 1 | Me | MeOCOCH₂ | bond | 1 | 1 | CN |
| *n*-Bu | bond | 0 | 1 | Me | MeNHCOCH₂ | bond | 1 | 1 | CN |
| *i*-Bu | bond | 0 | 1 | Me | Me₂NCOCH₂ | bond | 1 | 1 | CN |
| *t*-Bu | bond | 0 | 1 | Me | Ph | bond | 1 | 1 | CN |
| *c*-Bu | bond | 0 | 1 | Me | 2-Cl-Ph | bond | 1 | 1 | CN |
| *c*-pentyl | bond | 0 | 1 | Me | 4-Cl-Ph | bond | 1 | 1 | CN |
| *c*-hexyl | bond | 0 | 1 | Me | Bn | bond | 1 | 1 | CN |
| ClCH₂ | bond | 0 | 1 | Me | Ph-CH₂CH₂ | bond | 1 | 1 | CN |
| ClCH₂CH₂ | bond | 0 | 1 | Me | 2-thienyl | bond | 1 | 1 | CN |
| Cl₂CH | bond | 0 | 1 | Me | 3-thienyl | bond | 1 | 1 | CN |

The present disclosure also includes Tables 2A through 392A, each of which is constructed the same as Table 1A above, except that the row heading in Table 1A (i.e. "Z² is CH₂ and J is J-63") is replaced with the respective row heading shown below. For Example, in Table 2A the row heading is "Z² is CH² and J is J-1", and R¹, Z¹ s, f and R² are as defined in Table 1A above.

| Table | Row Heading | Table | Row Heading |
|---|---|---|---|
| 2A | Z² is CH₂ and J is J-1. | 198A | Z² is CH₂CH₂CH₂ and J is J-11. |
| 3A | Z² is CH₂ and J is J-2. | 199A | Z² is CH₂CH₂CH₂, J is J-12 and R^{8a} is Me. |
| 4A | Z² is CH₂, J is J-3 and R^{8a} is Me. | 200A | Z² is CH₂CH₂CH₂, J is J-13 and R^{8a} is Me. |
| 5A | Z² is CH₂ and J is J-4. | 201A | Z² is CH₂CH₂CH₂ and J is J-14. |
| 6A | Z² is CH₂ and J is J-5. | 202A | Z² is CH₂CH₂CH₂ and J is J-15. |
| 7A | Z² is CH₂, J is J-6 and R^{8a} is Me. | 203A | Z² is CH₂CH₂CH₂, J is J-16 and R^{8a} is Me. |
| 8A | Z² is CH₂ and J is J-7. | 204A | Z² is CH₂CH₂CH₂ and J is J-17. |
| 9A | Z² is CH₂ and J is J-8. | 205A | Z² is CH₂CH₂CH₂ and J is J-18. |
| 10A | Z² is CH₂, J is J-9 and R^{8a} is Me. | 206A | Z² is CH₂CH₂CH₂, Jis J-19 and R^{8a} is Me. |
| 11A | Z² is CH₂ and J is J-10. | 207A | Z² is CH₂CH₂CH₂ and J is J-20. |
| 12A | Z² is CH₂ and J is J-11. | 208A | Z² is CH₂CH₂CH₂ and J is J-21. |
| 13A | Z² is CH₂, Jis J-12 and R^{8a} is Me. | 209A | Z² is CH₂CH₂CH₂, J is J-22 and R^{8a} is Me. |
| 14A | Z² is CH₂, J is J-13 and R^{8a} is Me. | 210A | Z² is CH₂CH₂CH₂ and J is J-23. |
| 15A | Z² is CH₂ and J is J-14. | 211A | Z² is CH₂CH₂CH₂ and J is J-24. |
| 16A | Z² is CH₂ and J is J-15. | 212A | Z² is CH₂CH₂CH₂ and J is J-25. |
| 17A | Z² is CH₂, J is J-16 and R^{8a} is Me. | 213A | Z² is CH₂CH₂CH₂ and J is J-26. |
| 18A | Z² is CH₂ and J is J-17. | 214A | Z² is CH₂CH₂CH₂ and J is J-27. |
| 19A | Z² is CH₂ and J is J-18. | 215A | Z² is CH₂CH₂CH₂ and J is J-28. |
| 20A | Z² is CH₂, Jis J-19 and R^{8a} is Me. | 216A | Z² is CH₂CH₂CH₂ and J is J-29. |
| 21A | Z² is CH₂ and J is J-20. | 217A | Z² is CH₂CH₂CH₂ and J is J-30. |
| 22A | Z² is CH₂ and J is J-21. | 218A | Z² is CH₂CH₂CH₂ and J is J-31. |
| 23A | Z² is CH₂, Jis J-22 and R^{8a} is Me. | 219A | Z² is CH₂CH₂CH₂ and J is J-32. |
| 24A | Z² is CH₂ and J is J-23. | 220A | Z² is CH₂CH₂CH₂ and J is J-33. |
| 25A | Z² is CH₂ and J is J-24. | 221A | Z² is CH₂CH₂CH₂ and J is J-34. |
| 26A | Z² is CH₂ and J is J-25. | 222A | Z² is CH₂CH₂CH₂ and J is J-35. |
| 27A | Z² is CH₂ and J is J-26. | 223A | Z² is CH₂CH₂CH₂ and J is J-36. |
| 28A | Z² is CH₂ and J is J-27. | 224A | Z² is CH₂CH₂CH₂ and J is J-37. |
| 29A | Z² is CH₂ and J is J-28. | 225A | Z² is CH₂CH₂CH₂ and J is J-38. |
| 30A | Z² is CH₂ and J is J-29. | 226A | Z² is CH₂CH₂CH₂, J is J-39 and R^{8a} is Me. |
| 31A | Z² is CH₂ and J is J-30. | 227A | Z² is CH₂CH₂CH₂ and J is J-40. |
| 32A | Z² is CH₂ and J is J-31. | 228A | Z² is CH₂CH₂CH₂ and J is J-41. |
| 33A | Z² is CH₂ and J is J-32. | 229A | Z² is CH₂CH₂CH₂, J is J-42 and R^{8a} is Me. |
| 34A | Z² is CH₂ and J is J-33. | 230A | Z² is CH₂CH₂CH₂, J is J-43 and R^{8a} is Me. |
| 35A | Z² is CH₂ and J is J-34. | 231A | Z² is CH₂CH₂CH₂ and J is J-44. |
| 36A | Z² is CH₂ and J is J-35. | 232A | Z² is CH₂CH₂CH₂ and J is J-45. |
| 37A | Z² is CH₂ and J is J-36. | 233A | Z² is CH₂CH₂CH₂ and J is J-46. |
| 38A | Z² is CH₂ and J is J-37. | 234A | Z² is CH₂CH₂CH₂ and J is J-47. |
| 39A | Z² is CH₂ and J is J-38. | 235A | Z² is CH₂CH₂CH₂, J is J-48 and R^{8a} is Me. |
| 40A | Z² is CH₂, Jis J-39 and R^{8a} is Me. | 236A | Z² is CH₂CH₂CH₂ and J is J-49. |
| 41A | Z² is CH₂ and J is J-40. | 237A | Z² is CH₂CH₂CH₂ and J is J-50. |
| 42A | Z² is CH₂ and J is J-41. | 238A | Z² is CH₂CH₂CH₂, J is J-51 and R^{8a} is Me. |
| 43A | Z² is CH₂, J is J-42 and R^{8a} is Me. | 239A | Z² is CH₂CH₂CH₂ and J is J-52. |
| 44A | Z² is CH₂, J is J-43 and R^{8a} is Me. | 240A | Z² is CH₂CH₂CH₂ and J is J-53. |
| 45A | Z² is CH₂ and J is J-44. | 241A | Z² is CH₂CH₂CH₂, J is J-54 and R^{8a} is Me. |
| 46A | Z² is CH₂ and J is J-45. | 242A | Z² is CH₂CH₂CH₂ and J is J-55. |
| 47A | Z² is CH₂ and J is J-46. | 243A | Z² is CH₂CH₂CH₂ and J is J-56. |
| 48A | Z² is CH₂ and J is J-47. | 244A | Z² is CH₂CH₂CH₂ and J is J-57. |
| 49A | Z² is CH₂, J is J-48 and R^{8a} is Me. | 245A | Z² is CH₂CH₂CH₂, J is J-58 and R^{8a} is Me. |
| 50A | Z² is CH₂ and J is J-49. | 246A | Z² is CH₂CH₂CH₂, J is J-59 and R^{8a} is Me. |
| 51A | Z² is CH₂ and J is J-50. | 247A | Z² is CH₂CH₂CH₂ and J is J-60. |
| 52A | Z² is CH₂, J is J-51 and R^{8a} is Me. | 248A | Z² is CH₂CH₂CH₂ and J is J-61. |
| 53A | Z² is CH₂ and J is J-52. | 249A | Z² is CH₂CH₂CH₂ and J is J-62. |
| 54A | Z² is CH₂ and J is J-53. | 250A | Z² is CH₂CH₂CH₂, J is J-63 and R^{7a} is 2-F |
| 55A | Z² is CH₂, J is J-54 and R^{8a} is Me. | 251A | Z² is CH₂CH₂CH₂ and J is J-64. |
| 56A | Z² is CH₂ and J is J-55. | 252A | Z² is CH₂CH₂CH₂ and J is J-65. |
| 57A | Z² is CH₂ and J is J-56. | 253A | Z² is CH₂CH₂CH₂ and J is J-66. |
| 58A | Z² is CH₂J-57. | 254A | Z² is CH₂CH₂CH₂ and J is J-67. |
| 59A | Z² is CH₂, J is J-58 and R^{8a} is Me. | 255A | Z² is CH₂CH₂CH₂ and J is J-68. |
| 60A | Z² is CH₂, Jis J-59 and R^{8a} is Me. | 256A | Z² is CH₂CH₂CH₂ and J is J-69. |
| 61A | Z² is CH₂ and J is J-60. | 257A | Z² is CH₂CH₂CH₂ and J is J-70. |
| 62A | Z² is CH₂ and J is J-61. | 258A | Z² is CH₂CH₂CH₂ and J is J-71. |
| 63A | Z² is CH₂ and J is J-62. | 259A | Z² is CH₂CH₂CH₂ and J is J-72. |
| 64A | Z² is CH₂, J is J-63 and R^{7a} is 2-F. | 260A | Z² is CH₂CH₂CH₂ and J is J-73. |
| 65A | Z² is CH₂ and J is J-64. | 261A | Z² is CH₂CH₂CH₂ and J is J-74. |
| 66A | Z² is CH₂ and J is J-65. | 262A | Z² is CH₂CH₂CH₂ and J is J-75. |
| 67A | Z² is CH₂ and J is J-66. | 263A | Z² is CH₂CH₂CH₂ and J is J-76. |
| 68A | Z² is CH₂ and J is J-67. | 264A | Z² is CH₂CH₂CH₂ and J is J-77. |
| 69A | Z² is CH₂ and J is J-68. | 265A | Z² is CH₂CH₂CH₂ and J is J-78. |
| 70A | Z² is CH₂ and J is J-69. | 266A | Z² is CH₂CH₂CH₂ and J is J-79. |
| 71A | Z² is CH₂ and J is J-70. | 267A | Z² is CH₂CH₂CH₂ and J is J-80. |
| 72A | Z² is CH₂ and J is J-71. | 268A | Z² is CH₂CH₂CH₂ and J is J-81. |
| 73A | Z² is CH₂ and J is J-72. | 269A | Z² is CH₂CH₂CH₂ and J is J-82. |
| 74A | Z² is CH₂ and J is J-73. | 270A | Z² is CH₂CH₂CH₂ and J is J-83. |
| 75A | Z² is CH₂ and J is J-74. | 271A | Z² is CH₂CH₂CH₂ and J is J-84. |
| 76A | Z² is CH₂ and J is J-75. | 272A | Z² is CH₂CH₂CH₂ and J is J-85. |
| 77A | Z² is CH₂ and J is J-76. | 273A | Z² is CH₂CH₂CH₂ and J is J-86. |
| 78A | Z² is CH₂ and J is J-77. | 274A | Z² is CH₂CH₂CH₂ and J is J-87. |
| 79A | Z² is CH₂ and J is J-78. | 275A | Z² is CH₂CH₂CH₂ and J is J-88. |
| 80A | Z² is CH₂ and J is J-79. | 276A | Z² is CH₂CH₂CH₂ and J is J-89. |
| 81A | Z² is CH₂ and J is J-80. | 277A | Z² is CH₂CH₂CH₂ and J is J-90. |
| 82A | Z² is CH₂ and J is J-81. | 278A | Z² is CH₂CH₂CH₂ and J is J-91. |
| 83A | Z² is CH₂ and J is J-82. | 279A | Z² is CH₂CH₂CH₂ and J is J-92. |
| 84A | Z² is CH₂ and J is J-83. | 280A | Z² is CH₂CH₂CH(Ph) and J is J-63. |
| 85A | Z² is CH₂ and J is J-84. | 281A | Z² is bond and J is J-1. |
| 86A | Z² is CH₂ and J is J-85. | 282A | Z² is bond and J is J-2. |
| 87A | Z² is CH₂ and J is J-86. | 283A | Z² is bond, J is J-3 and R^{8a} is Me. |
| 88A | Z² is CH₂ and J is J-87. | 284A | Z² is bond and J is J-4. |
| 89A | Z² is CH₂ and J is J-88. | 285A | Z² is bond and J is J-5. |
| 90A | Z² is CH₂ and J is J-89. | 286A | Z² is bond, J is J-6 and R^{8a} is Me. |
| 91A | Z² is CH₂ and J is J-90. | 287A | Z² is bond and J is J-7. |
| 92A | Z² is CH₂ and J is J-91. | 288A | Z² is bond and J is J-8. |
| 93A | Z² is CH₂ and J is J-92. | 289A | Z² is bond, J is J-9 and R^{8a} is Me. |
| 94A | Z² is CH(Ph) and J is J-63. | 290A | Z² is bond and J is J-10. |
| 95A | Z² is CH₂CH₂ and J is J-1. | 291A | Z² is bond and J is J-1 1. |
| 96A | Z² is CH₂CH₂ and J is J-2. | 292A | Z² is bond, J is J-12 and R^{8a} is Me. |
| 97A | Z² is CH₂CH₂, J is J-3 and R^{8a} is Me. | 293A | Z² is bond, Jis J-13 and R^{8a} is Me. |
| 98A | Z² is CH₂CH₂ and J is J-4. | 294A | Z² is bond and J is J-14. |
| 99A | Z² is CH₂CH₂ and J is J-5. | 295A | Z² is bond and J is J-15. |
| 100A | Z² is CH₂CH₂, J is J-6 and R^{8a} is Me. | 296A | Z² is bond, J is J-16 and R^{8a} is Me. |
| 101A | Z² is CH₂CH₂ and J is J-7. | 297A | Z² is bond and J is J-17. |
| 102A | Z² is CH₂CH₂ and J is J-8. | 298A | Z² is bond and J is J-18. |
| 103A | Z² is CH₂CH₂, J is J-9 and R^{8a} is Me. | 299A | Z² is bond, J is J-19 and R^{8a} is Me. |
| 104A | Z² is CH₂CH₂ and J is J-10. | 300A | Z² is bond and J is J-20. |
| 105A | Z² is CH₂CH₂ and J is J-11. | 301A | Z² is bond and J is J-21. |
| 106A | Z² is CH₂CH₂, Jis J-12 and R^{8a} is Me. | 302A | Z² is bond, J is J-22 and R^{8a} is Me. |
| 107A | Z² is CH₂CH₂, Jis J-13 and R^{8a} is Me. | 303A | Z² is bond and J is J-23. |
| 108A | Z² is CH₂CH₂ and J is J-14. | 304A | Z² is bond and J is J-24. |
| 109A | Z² is CH₂CH₂ and J is J-15. | 305A | Z² is bond and J is J-31. |
| 110A | Z² is CH₂CH₂, J is J-16 and R^{8a} is Me. | 306A | Z² is bond and J is J-32. |
| 111A | Z² is CH₂CH₂ and J is J-17. | 307A | Z² is bond and J is J-33. |
| 112A | Z² is CH₂CH₂ and J is J-18. | 308A | Z² is bond and J is J-34. |
| 113A | Z² is CH₂CH₂, J is J-19 and R^{8a} is Me. | 309A | Z² is bond and J is J-35. |
| 114A | Z² is CH₂CH₂ and J is J-20. | 310A | Z² is bond and J is J-37. |
| 115A | Z² is CH₂CH₂ and J is J-21. | 311A | Z² is bond and J is J-38. |
| 116A | Z² is CH₂CH₂, J is J-22 and R^{8a} is Me. | 312A | Z² is bond, J is J-39 and R^{8a} is Me. |
| 117A | Z² is CH₂CH₂ and J is J-23. | 313A | Z² is bond and J is J-40. |
| 118A | Z² is CH₂CH₂ and J is J-24. | 314A | Z² is bond and J is J-41. |
| 119A | Z² is CH₂CH₂ and J is J-25. | 315A | Z² is bond, J is J-42 and R^{8a} is Me. |
| 120A | Z² is CH₂CH₂ and J is J-26. | 316A | Z² is bond, J is J-43 and R^{8a} is Me. |
| 121A | Z² is CH₂CH₂ and J is J-27. | 317A | Z² is bond and J is J-44. |
| 122A | Z² is CH₂CH₂ and J is J-28. | 318A | Z² is bond and J is J-45. |
| 123A | Z² is CH₂CH₂ and J is J-29. | 319A | Z² is bond and J is J-46. |
| 124A | Z² is CH₂CH₂ and J is J-30. | 320A | Z² is bond and J is J-47. |
| 125A | Z² is CH₂CH₂ and J is J-31. | 321A | Z² is bond, J is J-48 and R^{8a} is Me. |
| 126A | Z² is CH₂CH₂ and J is J-32. | 322A | Z² is bond and J is J-49. |
| 127A | Z² is CH₂CH₂ and J is J-33. | 323A | Z² is bond and J is J-50. |
| 128A | Z² is CH₂CH₂ and J is J-34. | 324A | Z² is bond, J is J-51 and R^{8a} is Me. |
| 129A | Z² is CH₂CH₂ and J is J-35. | 325A | Z² is bond and J is J-52. |
| 130A | Z² is CH₂CH₂ and J is J-36. | 326A | Z² is bond and J is J-53. |
| 131A | Z² is CH₂CH₂ and J is J-37. | 327A | Z² is bond, J is J-54 and R^{8a} is Me. |
| 132A | Z² is CH₂CH₂ and J is J-38. | 328A | Z² is bond and J is J-55. |
| 133A | Z² is CH₂CH₂, Jis J-39 and R^{8a} is Me. | 329A | Z² is bond and J is J-56. |
| 134A | Z² is CH₂CH₂ and J is J-40. | 330A | Z² is bond and J is J-57. |
| 135A | Z² is CH₂CH₂ and J is J-41. | 331A | Z² is bond, J is J-58 and R^{8a} is Me. |
| 136A | Z² is CH₂CH₂, J is J-42 and R^{8a} is Me. | 332A | Z² is bond, J is J-59 and R^{8a} is Me. |
| 137A | Z² is CH₂CH₂, J is J-43 and R^{8a} is Me. | 333A | Z² is bond and J is J-60. |
| 138A | Z² is CH₂CH₂ and J is J-44. | 334A | Z² is bond and J is J-61. |
| 139A | Z² is CH₂CH₂ and J is J-45. | 335A | Z² is bond and J is J-62. |
| 140A | Z² is CH₂CH₂ and J is J-46. | 336A | Z² is bond, Jis J-63 and R^{7a} is 2-F. |
| 141A | Z² is CH₂CH₂ and J is J-47. | 337A | Z² is bond and J is J-64. |
| 142A | Z² is CH₂CH₂, Jis J-48 and R^{8a} is Me. | 338A | Z² is bond and J is J-65. |
| 143A | Z² is CH₂CH₂ and J is J-49. | 339A | Z² is bond and J is J-66. |
| 144A | Z² is CH₂CH₂ and J is J-50. | 340A | Z² is bond and J is J-67. |
| 145A | Z² is CH₂CH₂, Jis J-51 and R^{8a} is Me. | 341A | Z² is bond and J is J-68. |
| 146A | Z² is CH₂CH₂ and J is J-52. | 342A | Z² is bond and J is J-69. |
| 147A | Z² is CH₂CH₂ and J is J-53. | 343A | Z² is bond and J is J-70. |
| 148A | Z² is CH₂CH₂, Jis J-54 and R^{8a} is Me. | 344A | Z² is bond and J is J-71. |
| 149A | Z² is CH₂CH₂ and J is J-55. | 345A | Z² is bond and J is J-72. |
| 150A | Z² is CH₂CH₂ and J is J-56. | 346A | Z² is bond and J is J-74. |
| 151A | Z² is CH₂CH₂ and J is J-57. | 347A | Z² is bond and J is J-76. |
| 152A | Z² is CH₂CH₂, J is J-58 and R^{8a} is Me. | 348A | Z² is bond and J is J-77. |
| 153A | Z² is CH₂CH₂, Jis J-59 and R^{8a} is Me. | 349A | Z² is bond and J is J-78. |
| 154A | Z² is CH₂CH₂ and J is J-60. | 350A | Z² is bond and J is J-79. |
| 155A | Z² is CH₂CH₂ and J is J-61. | 351A | Z² is bond and J is J-80. |
| 156A | Z² is CH₂CH₂ and J is J-62. | 352A | Z² is bond and J is J-81. |
| 157A | Z² is CH₂CH₂, Jis J-63 and R^{7a} is 2-F. | 353A | Z² is bond and J is J-82. |
| 158A | Z² is CH₂CH₂ and J is J-64. | 354A | Z² is bond and J is J-83. |
| 159A | Z² is CH₂CH₂ and J is J-65. | 355A | Z² is bond and J is J-84. |
| 160A | Z² is CH₂CH₂ and J is J-66. | 356A | Z² is bond and J is J-85. |
| 161A | Z² is CH₂CH₂ and J is J-67. | 357A | Z² is bond and J is J-86. |
| 162A | Z² is CH₂CH₂ and J is J-68. | 358A | Z² is bond and J is J-87. |
| 163A | Z² is CH₂CH₂ and J is J-69. | 359A | Z² is bond and J is J-88. |
| 164A | Z² is CH₂CH₂ and J is J-70. | 360A | Z² is bond and J is J-89. |
| 165A | Z² is CH₂CH₂ and J is J-71. | 361A | Z² is bond and J is J-91. |
| 166A | Z² is CH₂CH₂ and J is J-72. | 362A | Z² is bond and J-63 and R^{7a} is 2,4-diCl. |
| 167A | Z² is CH₂CH₂ and J is J-73. | 363A | Z² is bond, J is J-63 and R^{7a} is 2-Cl. |
| 168A | Z² is CH₂CH₂ and J is J-74. | 364A | Z² is bond, J is J-63 and R^{7a} is 2-Me. |
| 169A | Z² is CH₂CH₂ and J is J-75. | 365A | Z² is bond, J is J-63 and R^{7a} is 2-MeO. |
| 170A | Z² is CH₂CH₂ and J is J-76. | 366A | Z² is bond, J is J-63 and R^{7a} is 3-F. |
| 171A | Z² is CH₂CH₂ and J is J-77. | 367A | Z² is bond, J is J-63 and R^{7a} is 3-Cl. |
| 172A | Z² is CH₂CH₂ and J is J-78. | 368A | Z² is bond, J is J-63 and R^{7a} is 3-Me. |
| 173A | Z² is CH₂CH₂ and J is J-79. | 369A | Z² is bond, J is J-63 and R^{7a} is 3-MeO. |
| 174A | Z² is CH₂CH₂ and J is J-80. | 370A | Z² is bond, J is J-63 and R^{7a} is 2,6-diF. |
| 175A | Z² is CH₂CH₂ and J is J-81. | 371A | Z² is bond, J is J-63 and R^{7a} is 2,3-diF. |
| 176A | Z² is CH₂CH₂ and J is J-82. | 372A | Z² is bond, J is J-63 and R^{7a} is 3,5-diF. |
| 177A | Z² is CH₂CH₂ and J is J-83. | 373A | Z² is bond, J is J-63 and R^{7a} is 2,6-di-Cl. |
| 178A | Z² is CH₂CH₂ and J is J-84. | 374A | Z² is bond, J is J-63 and R^{7a} is 2,3-di-Cl. |
| 179A | Z² is CH₂CH₂ and J is J-85. | 375A | Z² is bond, J is J-63 and R^{7a} is 3,5-di-Cl. |
| 180A | Z² is CH₂CH₂ and J is J-86. | 376A | Z² is CH₂, J is J-63 and R^{7a} is 2-Cl. |
| 181A | Z² is CH₂CH₂ and J is J-87. | 377A | Z² is CH₂, J is J-63 and R^{7a} is 2-Me. |
| 182A | Z² is CH₂CH₂ and J is J-88. | 378A | Z² is CH₂, J is J-63 and R^{7a} is 2-MeO. |
| 183A | Z² is CH₂CH₂ and J is J-89. | 379A | Z² is CH₂, J is J-63 and R^{7a} is 3-F. |
| 184A | Z² is CH₂CH₂ and J is J-90. | 380A | Z² is CH₂, J is J-63 and R^{7a} is 3-Cl. |
| 185A | Z² is CH₂CH₂ and J is J-91. | 381A | Z² is CH₂, J is J-63 and R^{7a} is 3-Me. |
| 186A | Z² is CH₂CH₂ and J is J-92. | 382A | Z² is CH₂, J is J-63 and R^{7a} is 3-MeO. |
| 187A | Z² is CH₂CH(Ph) and J is J-63. | 383A | Z² is CH₂, J is J-63 and R7a is 2,6-diF. |
| 188A | Z² is CH₂CH₂CH₂ and J is J-1. | 384A | Z² is CH₂, J is J-63 and R^{7a} is 2,3-diF. |
| 189A | Z² is CH₂CH₂CH₂ and J is J-2. | 385A | Z² is CH₂, J is J-63 and R^{7a} is 3,5-diF. |
| 190A | Z² is CH₂CH₂CH₂, J is J-3 and R^{8a} is Me. | 386A | Z² is CH₂, J is J-63 and R^{7a} is 2,6-di-Cl. |
| 191A | Z² is CH₂CH₂CH₂ and J is J-4. | 387A | Z² is CH₂, J is J-63 and R^{7a} is 2,3-di-Cl. |
| 192A | Z² is CH₂CH₂CH₂ and J is J-5. | 388A | Z² is CH₂, J is J-63 and R^{7a} is 3,5-di-Cl. |
| 193A | Z² is CH₂CH₂CH₂, J is J-6 and R^{8a} is Me. | 389A | Z² is CH₂CH(OH) and J is J-63. |
| 194A | Z² is CH₂CH₂CH₂ and J is J-7. | 390A | Z² is CH₂CH(OMe) and J is J-63. |
| 195A | Z² is CH₂CH₂CH₂ and J is J-8. | 391A | Z² is CH₂CH(OAc) and J is J-63. |
| 196A | Z² is CH₂CH₂CH₂, J is J-9 and R^{8a} is Me. | 392A | Z² is CH₂CH(CN) and J is J-63. |
| 197A | Z² is CH₂CH₂CH₂ and J is J-10. | | |

### Formulation/Utility

A compound of Formula 1 of this invention will generally be used as a fungicidal active ingredient in a composition, i.e. formulation, with at least one additional component selected from the group consisting of surfactants, solid diluents and liquid diluents, which serve as a carrier. The formulation or composition ingredients are selected to be consistent with the physical properties of the active ingredient, mode of application and environmental factors such as soil type, moisture and temperature.

Useful formulations include both liquid and solid compositions. Liquid compositions include solutions (including emulsifiable concentrates), suspensions, emulsions (including microemulsions and/or suspoemulsions) and the like, which optionally can be thickened into gels. The general types of aqueous liquid compositions are soluble concentrate, suspension concentrate, capsule suspension, concentrated emulsion, microemulsion and suspo-emulsion. The general types of nonaqueous liquid compositions are emulsifiable concentrate, microemulsifiable concentrate, dispersible concentrate and oil dispersion.

The general types of solid compositions are dusts, powders, granules, pellets, pills, pastilles, tablets, filled films (including seed coatings) and the like, which can be water-dispersible ("wettable") or water-soluble. Films and coatings formed from film-forming solutions or flowable suspensions are particularly useful for seed treatment. Active ingredient can be (micro)encapsulated and further formed into a suspension or solid formulation; alternatively the entire formulation of active ingredient can be encapsulated (or "overcoated"). Encapsulation can control or delay release of the active ingredient. An emulsifiable granule combines the advantages of both an emulsifiable concentrate formulation and a dry granular formulation. High-strength compositions are primarily used as intermediates for further formulation.

Sprayable formulations are typically extended in a suitable medium before spraying. Such liquid and solid formulations are formulated to be readily diluted in the spray medium, usually water. Spray volumes can range from about one to several thousand liters per hectare, but more typically are in the range from about ten to several hundred liters per hectare. Sprayable formulations can be tank mixed with water or another suitable medium for foliar treatment by aerial or ground application, or for application to the growing medium of the plant. Liquid and dry formulations can be metered directly into drip irrigation systems or metered into the furrow during planting. Liquid and solid formulations can be applied onto seeds of crops and other desirable vegetation as seed treatments before planting to protect developing roots and other subterranean plant parts and/or foliage through systemic uptake.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble Granules, Tablets and Powders. | 0.001-90 | 0-99.999 | 0-15 |
| Oil Dispersions, Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-95 | 5-99.999 | 0-15 |
| High Strength Compositions | 90-99 | 0-10 | 0-2 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al., Handbook of Insecticide Dust Diluents and Carriers, 2nd Ed., Dorland Books, Caldwell, New Jersey.

Liquid diluents include, for example, water, *N*,*N*-dimethylalkanamides (e.g., *N*,*N-*dimethylformamide), limonene, dimethyl sulfoxide, *N*-alkylpyrrolidones (e.g., *N-*methylpyrrolidinone), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters and γ-butyrolactone, and alcohols, which can be linear, branched, saturated or unsaturated, such as methanol, ethanol, *n*-propanol, isopropyl alcohol, *n*-butanol, isobutyl alcohol, *n*-hexanol, 2-ethylhexanol, *n*-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol and benzyl alcohol. Liquid diluents also include glycerol esters of saturated and unsaturated fatty acids (typically C₆-C₂₂), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950.

The solid and liquid compositions of the present invention often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents.

Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolin-based derivatives, polyethoxylate esters such as polyethoxylated sorbitan fatty acid esters, polyethoxylated sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides and alkyl polysaccharides.

Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as *N*,*N*-alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts.

Useful cationic surfactants include, but are not limited to: amides and ethoxylated amides; amines such as *N*-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as quaternary salts, ethoxylated quaternary salts and diquaternary salts; and amine oxides such as alkyldimethylamine oxides and bis-(2-hydroxyethyl)-alkylamine oxides.

Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987.

Compositions of this invention may also contain formulation auxiliaries and additives, known to those skilled in the art as formulation aids. Such formulation auxiliaries and additives may control: pH (buffers), foaming during processing (antifoams such polyorganosiloxanes (e.g., Rhodorsil^{®} 416)), sedimentation of active ingredients (suspending agents), viscosity (thixotropic thickeners), in-container microbial growth (antimicrobials), product freezing (antifreezes), color (dyes/pigment dispersions (e.g., Pro-Ized^{®} Colorant Red)), wash-off (film formers or stickers), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed in McCutcheon's Volume 2: Functional Materials, annual International and North American editions published by McCutcheon's Division, The Manufacturing Confectioner Publishing Co.; and PCT Publication WO 03/024222.

Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2,000 µm can be wet milled using media mills to obtain particles with average diameters below 3 µm. Aqueous slurries can be made into finished suspension concentrates (see, for example, U.S. 3,060,084) or further processed by spray drying to form water-dispersible granules. Dry formulations usually require dry milling processes, which produce average particle diameters in the 2 to 10 µm range. Dusts and powders can be prepared by blending and, usually, grinding as in a hammer mill or fluid-energy mill. Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all percentages are by weight and all formulations are prepared in conventional ways. Compound numbers refer to compounds in Index

Tables A-E. Without further elaboration, it is believed that one skilled in the art using the preceding description can utilize the present invention to its fullest extent. The following Examples are, therefore, to be construed as merely illustrative, and not limiting of the disclosure in any way whatsoever. Percentages are by weight except where otherwise indicated.

### Example A

### High Strength Concentrate

| | |
|---|---|
| Compound 245 | 98.5% |
| silica aerogel | 0.5% |
| synthetic amorphous fine silica | 1.0% |

### Example B

### Wettable Powder

| | |
|---|---|
| Compound 240 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

### Example C

### Granule

| | |
|---|---|
| Compound 239 | 10.0% |
| attapulgite granules (low volatile matter, 0.71/0.30 mm; U.S.S. No. 25-50 sieves) | 90.0% |

### Example D

### Aqueous Suspension

| | |
|---|---|
| Compound 238 | 25.0% |
| hydrated attapulgite | 3.0% |
| crude calcium ligninsulfonate | 10.0% |
| sodium dihydrogen phosphate | 0.5% |
| water | 61.5% |

### Example E

### Extruded Pellet

| | |
|---|---|
| Compound 236 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

### Example F

### Microemulsion

| | |
|---|---|
| Compound 234 | 5.0% |
| polyvinylpyrrolidone-vinyl acetate copolymer | 30.0% |
| C₈-C₁₀ alkylpolyglycoside | 30.0% |
| glyceryl monooleate | 15.0% |
| Water | 20.0% |

### Example G

### Emulsifiable Concentrate

| | |
|---|---|
| Compound 220 | 10.0% |
| polyoxyethylene sorbitol hexoleate | 20.0% |
| C₆-C₁₀ fatty acid methyl ester | 70.0% |

Water-soluble and water-dispersible formulations are typically diluted with water to form aqueous compositions before application. Aqueous compositions for direct applications to the plant or portion thereof (e.g., spray tank compositions) typically at least about 1 ppm or more (e.g., from 1 ppm to 100 ppm) of the compound(s) of this invention.

The compounds of this invention are useful as plant disease control agents. The present invention therefore further comprises a method for controlling plant diseases caused by fungal plant pathogens comprising applying to the plant or portion thereof to be protected, or to the plant seed to be protected, an effective amount of a compound of the invention or a fungicidal composition containing said compound. This aspect of the present invention can also be described as a method for protecting a plant or plant seed from diseases caused by fungal pathogens comprising applying a fungicidally effective amount of a compound of the invention (e.g., as a composition described herein) to the plant (or portion thereof) or plant seed (directly or through the environment (e.g., growing medium) of the plant or plant seed). The compounds and/or compositions of this invention provide control of diseases caused by a broad spectrum of fungal plant pathogens in the Basidiomycete, Ascomycete, Oomycete and Deuteromycete classes. They are effective in controlling a broad spectrum of plant diseases, particularly foliar pathogens of ornamental, turf, vegetable, field, cereal, and fruit crops. These pathogens include: Oomycetes, including *Phytophthora* diseases such as *Phytophthora infestans*, *Phytophthora megasperma*, *Phytophthora parasitica*, *Phytophthora cinnamomi* and *Phytophthora capsid, Pythium* diseases such as *Pythium aphanidermatum,* and diseases in the Peronosporaceae family such as *Plasmopara viticola, Peronospora* spp. (including *Peronospora tabacina* and *Peronospora parasitica*)*, Pseudoperonospora* spp. (including *Pseudoperonospora cubensis*) and *Bremia lactucae*; Ascomycetes, including *Alternaria* diseases such as *Alternaria solani* and *Alternaria brassicae*, *Guignardia* diseases such as *Guignardia bidwell, Venturia* diseases such as *Yenturia inaequalis, Septoria* diseases such as *Septoria nodorum* and *Septoria tritici,* powdery mildew diseases such as *Erysiphe* spp. (including *Erysiphe graminis* and *Erysiphe polygoni*), *Uncinula neaatur, Sphaerotheca fuligena* and *Podosphaera leucotricha, Pseudocercosporella herpotrichoides, Botrytis* diseases such as *Botrytis cinerea, Monilinia fructicola*, *Sclerotinia* diseases such as *Sclerotinia sclerotiorum, Magnaporthe grisea, Phomopsis viticola, Helminthosporium* diseases such as *Helminthosporium tritici repentis, Pyrenophora teres,* anthracnose diseases such as *Glomerella* or *Colletotrichum* spp. (such as *Colletotrichum graminicola* and *Colletotrichum orbiculare*)*,* and *Gaeumannomyces graminis*; Basidiomycetes, including rust diseases caused by *Puccinia* spp. (such as *Puccinia recondita*, *Puccinia striiformis, Puccinia hordei, Puccinia graminis* and *Puccinia arachidis*)*, Hemileia vastatrix* and *Phakopsora pαchyrhizi*; other pathogens including *Rutstroemia floccosum* (also known as *Sclerontina homoeocarpa); Rhizoctonia* spp. (such as *Rhizoctonia solani)*; *Fusarium* diseases such as *Fusarium roseum, Fusarium graminearum* and *Fusarium oxysporum; Verticillium dahliae; Sclerotium rolfsii*; *Rynchosporium secalis; Cercosporidium personatum*, *Cercospora arachidicola* and *Cercospora beticola*; and other genera and species closely related to these pathogens. In addition to their fungicidal activity, the compositions or combinations also have activity against bacteria such as *Erwinia amylovora*, *Xanthomonas campestris, Pseudomonas syringae,* and other related species. Furthermore, the compounds of this invention are useful in treating postharvest diseases of fruits and vegetables caused by fungi and bacteria. These infections can occur before, during and after harvest. For example, infections can occur before harvest and then remain dormant until some point during ripening (e.g., host begins tissue changes in such a way that infection can progress); also infections can arise from surface wounds created by mechanical or insect injury. In this respect, the compounds of this invention can reduce losses (i.e. losses resulting from quantity and quality) due to postharvest diseases which may occur at any time from harvest to consumption. Treatment of postharvest diseases with compounds of the invention can increase the period of time during which perishable edible plant parts (e.g, fruits, seeds, foliage, stems, bulbs, tubers) can be stored refrigerated or un-refrigerated after harvest, and remain edible and free from noticeable or harmful degradation or contamination by fungi or other microorganisms. Treatment of edible plant parts before or after harvest with compounds of the invention can also decrease the formation of toxic metabolites of fungi or other microorganisms, for example, mycotoxins such as aflatoxins.

Plant disease control is ordinarily accomplished by applying an effective amount of a compound of this invention either pre- or post-infection, to the portion of the plant to be protected such as the roots, stems, foliage, fruits, seeds, tubers or bulbs, or to the media (soil or sand) in which the plants to be protected are growing. The compounds can also be applied to seeds to protect the seeds and seedlings developing from the seeds. The compounds can also be applied through irrigation water to treat plants. Control of postharvest pathogens which infect the produce before harvest is typically accomplished by field application of a compound of this invention, and in cases where infection occurs after harvest the compounds can be applied to the harvested crop as dips, sprays, fumigants, treated wraps and box liners.

Rates of application for these compounds (i.e. a fungicidally effective amount) can be influenced by factors such as the plant diseases to be controlled, the plant species to be protected, ambient moisture and temperature and should be determined under actual use conditions. One skilled in the art can easily determine through simple experimentation the fungicidally effective amount necessary for the desired level of plant disease control. Foliage can normally be protected when treated at a rate of from less than about 1 g/ha to about 5,000 g/ha of active ingredient. Seed and seedlings can normally be protected when seed is treated at a rate of from about 0.1 to about 10 g per kilogram of seed.

Compounds of this invention can also be mixed with one or more other biologically active compounds or agents including fungicides, insecticides, nematocides, bactericides, acaricides, herbicides, herbicide safeners, growth regulators such as insect molting inhibitors and rooting stimulants, chemosterilants, semiochemicals, repellents, attractants, pheromones, feeding stimulants, plant nutrients, other biologically active compounds or entomopathogenic bacteria, virus or fungi to form a multi-component pesticide giving an even broader spectrum of agricultural protection. Thus the present invention also pertains to a composition comprising a compound of Formula **1** (in a fungicidally effective amount) and at least one additional biologically active compound or agent (in a biologically effective amount) and can further comprise at least one of a surfactant, a solid diluent or a liquid diluent. The other biologically active compounds or agents can be formulated in compositions comprising at least one of a surfactant, solid or liquid diluent. For mixtures of the present invention, one or more other biologically active compounds or agents can be formulated together with a compound of Formula **1,** to form a premix, or one or more other biologically active compounds or agents can be formulated separately from the compound of Formula **1,** and the formulations combined together before application (e.g., in a spray tank) or, alternatively, applied in succession.

Of note is a composition which in addition to the compound of Formula **1** include at least one fungicidal compound selected from the group consisting of the classes (1) methyl benzimidazole carbamate (MBC) fungicides; (2) dicarboximide fungicides; (3) demethylation inhibitor (DMI) fungicides; (4) phenylamide fungicides; (5) amine/morpholine fungicides; (6) phospholipid biosynthesis inhibitor fungicides; (7) carboxamide fungicides; (8) hydroxy(2-amino-)pyrimidine fungicides; (9) anilinopyrimidine fungicides; (10) *N*-phenyl carbamate fungicides; (11) quinone outside inhibitor (QoI) fungicides; (12) phenylpyrrole fungicides; (13) quinoline fungicides; (14) lipid peroxidation inhibitor fungicides; (15) melanin biosynthesis inhibitors-reductase (MBI-R) fungicides; (16) melanin biosynthesis inhibitors-dehydratase (MBI-D) fungicides; (17) hydroxyanilide fungicides; (18) squalene-epoxidase inhibitor fungicides; (19) polyoxin fungicides; (20) phenylurea fungicides; (21) quinone inside inhibitor (QiI) fungicides; (22) benzamide fungicides; (23) enopyranuronic acid antibiotic fungicides; (24) hexopyranosyl antibiotic fungicides; (25) glucopyranosyl antibiotic: protein synthesis fungicides; (26) glucopyranosyl antibiotic: trehalase and inositol biosynthesis fungicides; (27) cyanoacetamideoxime fungicides; (28) carbamate fungicides; (29) oxidative phosphorylation uncoupling fungicides; (30) organo tin fungicides; (31) carboxylic acid fungicides; (32) heteroaromatic fungicides; (33) phosphonate fungicides; (34) phthalamic acid fungicides; (35) benzotriazine fungicides; (36) benzene-sulfonamide fungicides; (37) pyridazinone fungicides; (38) thiophene-carboxamide fungicides; (39) pyrimidinamide fungicides; (40) carboxylic acid amide (CAA) fungicides; (41) tetracycline antibiotic fungicides; (42) thiocarbamate fungicides; (43) benzamide fungicides; (44) host plant defense induction fungicides; (45) multi-site contact activity fungicides; (46) fungicides other than classes (1) through (45); and salts of compounds of classes (1) through (46).

Further descriptions of these classes of fungicidal compounds are provided below.
(1) "Methyl benzimidazole carbamate (MBC) fungicides" (Fungicide Resistance Action Committee (FRAC) code 1) inhibit mitosis by binding to β-tubulin during microtubule assembly. Inhibition of microtubule assembly can disrupt cell division, transport within the cell and cell structure. Methyl benzimidazole carbamate fungicides include benzimidazole and thiophanate fungicides. The benzimidazoles include benomyl, carbendazim, fuberidazole and thiabendazole. The thiophanates include thiophanate and thiophanate-methyl.
(2) "Dicarboximide fungicides" (Fungicide Resistance Action Committee (FRAC) code 2) are proposed to inhibit a lipid peroxidation in fungi through interference with NADH cytochrome c reductase. Examples include chlozolinate, iprodione, procymidone and vinclozolin.
(3) "Demethylation inhibitor (DMI) fungicides" (Fungicide Resistance Action Committee (FRAC) code 3) inhibit C14-demethylase, which plays a role in sterol production. Sterols, such as ergosterol, are needed for membrane structure and function, making them essential for the development of functional cell walls. Therefore, exposure to these fungicides results in abnormal growth and eventually death of sensitive fungi. DMI fungicides are divided between several chemical classes: azoles (including triazoles and imidazoles), pyrimidines, piperazines and pyridines. The triazoles include azaconazole, bitertanol, bromuconazole, cyproconazole, difenoconazole, diniconazole (including diniconazole-M), epoxiconazole, fenbuconazole, fluquinconazole, flusilazole, flutriafol, hexaconazole, imibenconazole, ipconazole, metconazole, myclobutanil, penconazole, propiconazole, prothioconazole, simeconazole, tebuconazole, tetraconazole, triadimefon, triadimenol, triticonazole and uniconazole. The imidazoles include clotrimazole, imazalil, oxpoconazole, prochloraz, pefuraroate and triflumizole. The pyrimidines include fenarimol and nuarimol. The piperazines include triforine. The pyridines include pyrifenox. Biochemical investigations have shown that all of the above mentioned fungicides are DMI fungicides as described by K. H. Kuck et al. in Modern Selective Fungicides - Properties, Applications and Mechanisms of Action, H. Lyr (Ed.), Gustav Fischer Verlag: New York, 1995, 205-258.
(4) "Phenylamide fungicides" (Fungicide Resistance Action Committee (FRAC) code 4) are specific inhibitors of RNA polymerase in Oomycete fungi. Sensitive fungi exposed to these fungicides show a reduced capacity to incorporate uridine into rRNA. Growth and development in sensitive fungi is prevented by exposure to this class of fungicide. Phenylamide fungicides include acylalanine, oxazolidinone and butyrolactone fungicides. The acylalanines include benalaxyl, benalaxyl-M, furalaxyl, metalaxyl and metalaxyl-M/mefenoxam. The oxazolidinones include oxadixyl. The butyrolactones include ofurace.
(5) "Amine/morpholine fungicides" (Fungicide Resistance Action Committee (FRAC) code 5) inhibit two target sites within the sterol biosynthetic pathway, Δ⁸ → Δ⁷ isomerase and Δ¹⁴ reductase. Sterols, such as ergosterol, are needed for membrane structure and function, making them essential for the development of functional cell walls. Therefore, exposure to these fungicides results in abnormal growth and eventually death of sensitive fungi. Amine/morpholine fungicides (also known as non-DMI sterol biosynthesis inhibitors) include morpholine, piperidine and spiroketal-amine fungicides. The morpholines include aldimorph, dodemorph, fenpropimorph, tridemorph and trimorphamide. The piperidines include fenpropidin and piperalin. The spiroketal-amines include spiroxamine.
(6) "Phospholipid biosynthesis inhibitor fungicides" (Fungicide Resistance Action Committee (FRAC) code 6) inhibit growth of fungi by affecting phospholipid biosynthesis. Phospholipid biosynthesis fungicides include phophorothiolate and dithiolane fungicides. The phosphorothiolates include edifenphos, iprobenfos and pyrazophos. The dithiolanes include isoprothiolane.
(7) "Carboxamide fungicides" (Fungicide Resistance Action Committee (FRAC) code 7) inhibit Complex II (succinate dehydrogenase) fungal respiration by disrupting a key enzyme in the Krebs Cycle (TCA cycle) named succinate dehydrogenase. Inhibiting respiration prevents the fungus from making ATP, and thus inhibits growth and reproduction. Carboxamide fungicides include benzamides, furan carboxamides, oxathiin carboxamides, thiazole carboxamides, pyrazole carboxamides and pyridine carboxamides. The benzamides include benodanil, flutolanil and mepronil. The furan carboxamides include fenfuram. The oxathiin carboxamides include carboxin and oxycarboxin. The thiazole carboxamides include thifluzamide. The pyrazole carboxamides include furametpyr, penthiopyrad, bixafen, isopyrazam, *N*-[2-(1*S*,2*R*)-[1,1'-bicyclopropyl]-2-ylphenyl]-3-(difluoromethyl)-1-methyl-1*H-*pyrazole-4-carboxamide and penflufen (*N*-[2-(1,3-dimethylbutyl)phenyl]-5-fluoro-1,3-dimethy]-1*H*-pyrazole-4-carboxamide). The pyridine carboxamides include boscalid.
(8) "Hydroxy(2-amino-)pyrimidine fungicides" (Fungicide Resistance Action Committee (FRAC) code 8) inhibit nucleic acid synthesis by interfering with adenosine deaminase. Examples include bupirimate, dimethirimol and ethirimol.
(9) "Anilinopyrimidine fungicides" (Fungicide Resistance Action Committee (FRAC) code 9) are proposed to inhibit biosynthesis of the amino acid methionine and to disrupt the secretion of hydrolytic enzymes that lyse plant cells during infection. Examples include cyprodinil, mepanipyrim and pyrimethanil.
(10) "*N*-Phenyl carbamate fungicides" (Fungicide Resistance Action Committee (FRAC) code 10) inhibit mitosis by binding to β-tubulin and disrupting microtubule assembly. Inhibition of microtubule assembly can disrupt cell division, transport within the cell and cell structure. Examples include diethofencarb.
(11) "Quinone outside inhibitor (QoI) fungicides" (Fungicide Resistance Action Committee (FRAC) code 11) inhibit Complex III mitochondrial respiration in fungi by affecting ubiquinol oxidase. Oxidation of ubiquinol is blocked at the "quinone outside" (Qₒ) site of the cytochrome *bc*₁ complex, which is located in the inner mitochondrial membrane of fungi. Inhibiting mitochondrial respiration prevents normal fungal growth and development. Quinone outside inhibitor fungicides (also known as strobilurin fungicides) include methoxyacrylate, methoxycarbamate, oximinoacetate, oximinoacetamide, oxazolidinedione, dihydrodioxazine, imidazolinone and benzylcarbamate fungicides. The methoxyacrylates include azoxystrobin, enestroburin (SYP-Z071), picoxystrobin and pyraoxystrobin (SYP-3343). The methoxycarbamates include pyraclostrobin and pyrametostrobin (SYP-4155). The oximinoacetates include kresoxim-methyl and trifloxystrobin. The oximinoacetamides include dimoxystrobin, metominostrobin, orysastrobin, α-[methoxyimino]-*N*-methyl-2-[[[-1-[3-(trifluoromethyl)phenyl]ethoxy]imino]-methyl]benzeneacetamide and 2-[[[3-(2,6-dichlorophenyl)-1-methyl-2-propen-1-ylidene]-amino]oxy]methyl]-α-(methoxyimino)-*N*-methylbenzeneacetamide. The oxazolidinediones include famoxadone. The dihydrodioxazines include fluoxastrobin. The imidazolinones include fenamidone. The benzylcarbamates include pyribencarb. Class (11) also includes 2-[(2,5-dimethylphenoxy)methyl]-α-methoxy-*N*-benzeneacetamide.
(12) "Phenylpyrrole fungicides" (Fungicide Resistance Action Committee (FRAC) code 12) inhibit a MAP protein kinase associated with osmotic signal transduction in fungi. Fenpiclonil and fludioxonil are examples of this fungicide class.
(13) "Azanaphthalene fungicides" (Fungicide Resistance Action Committee (FRAC) code 13) are proposed to inhibit signal transduction by affecting G-proteins in early cell signaling. They have been shown to interfere with germination and/or appressorium formation in fungi that cause powder mildew diseases. Azanaphthalene fungicides include aryloxyquinolines and quinazolinone. The aryloxyquinolines include quinoxyfen and tebufloquin. The quinazolinones include proquinazid.
(14) "Lipid peroxidation inhibitor fungicides" (Fungicide Resistance Action Committee (FRAC) code 14) are proposed to inhibit lipid peroxidation which affects membrane synthesis in fungi. Members of this class, such as etridiazole, may also affect other biological processes such as respiration and melanin biosynthesis. Lipid peroxidation fungicides include aromatic carbon and 1,2,4-thiadiazole fungicides. The aromatic carbon fungicides include biphenyl, chloroneb, dicloran, quintozene, tecnazene and tolclofos-methyl. The 1,2,4-thiadiazole fungicides include etridiazole.
(15) "Melanin biosynthesis inhibitors-reductase (MBI-R) fungicides" (Fungicide Resistance Action Committee (FRAC) code 16.1) inhibit the naphthal reduction step in melanin biosynthesis. Melanin is required for host plant infection by some fungi. Melanin biosynthesis inhibitors-reductase fungicides include isobenzofuranone, pyrroloquinolinone and triazolobenzothiazole fungicides. The isobenzofuranones include flhalide. The pyrroloquinolinones include pyroquilon. The triazolobenzothiazoles include tricyclazole.
(16) "Melanin biosynthesis inhibitors-dehydratase (MBI-D) fungicides" (Fungicide Resistance Action Committee (FRAC) code 16.2) inhibit scytalone dehydratase in melanin biosynthesis. Melanin in required for host plant infection by some fungi. Melanin biosynthesis inhibitors-dehydratase fungicides include cyclopropanecarboxamide, carboxamide and propionamide fungicides. The cyclopropanecarboxamides include carpropamid. The carboxamides include diclocymet. The propionamides include fenoxanil.
(17) "Hydroxyanilide fungicides (Fungicide Resistance Action Committee (FRAC) code 17) inhibit C4-demethylase which plays a role in sterol production. Examples include fenhexamid.
(18) "Squalene-epoxidase inhibitor fungicides" (Fungicide Resistance Action Committee (FRAC) code 18) inhibit squalene-epoxidase in ergosterol biosynthesis pathway. Sterols such as ergosterol are needed for membrane structure and function, making them essential for the development of functional cell walls. Therefore exposure to these fungicides results in abnormal growth and eventually death of sensitive fungi. Squalene-epoxidase inhibitor fungicides include thiocarbamate and allylamine fungicides. The thiocarbamates include pyributicarb. The allylamines include naftifine and terbinafine.
(19) "Polyoxin fungicides" (Fungicide Resistance Action Committee (FRAC) code 19) inhibit chitin synthase. Examples include polyoxin.
(20) "Phenylurea fungicides" (Fungicide Resistance Action Committee (FRAC) code 20) are proposed to affect cell division. Examples include pencycuron.
(21) "Quinone inside inhibitor (Qil) fungicides" (Fungicide Resistance Action Committee (FRAC) code 21) inhibit Complex III mitochondrial respiration in fungi by affecting ubiquinol reductase. Reduction of ubiquinol is blocked at the "quinone inside" (Qᵢ) site of the cytochrome *bc*₁ complex, which is located in the inner mitochondrial membrane of fungi. Inhibiting mitochondrial respiration prevents normal fungal growth and development. Quinone inside inhibitor fungicides include cyanoimidazole and sulfamoyltriazole fungicides. The cyanoimidazoles include cyazofamid. The sulfamoyltriazoles include amisulbrom
(22) "Benzamide fungicides" (Fungicide Resistance Action Committee (FRAC) code 22) inhibit mitosis by binding to β-tubulin and disrupting microtubule assembly. Inhibition of microtubule assembly can disrupt cell division, transport within the cell and cell structure. Examples include zoxamide.
(23) "Enopyranuronic acid antibiotic fungicides" (Fungicide Resistance Action Committee (FRAC) code 23) inhibit growth of fungi by affecting protein biosynthesis. Examples include blasticidin-S.
(24) "Hexopyranosyl antibiotic fungicides" (Fungicide Resistance Action Committee (FRAC) code 24) inhibit growth of fungi by affecting protein biosynthesis. Examples include kasugamycin.
(25) "Glucopyranosyl antibiotic: protein synthesis fungicides" (Fungicide Resistance Action Committee (FRAC) code 25) inhibit growth of fungi by affecting protein biosynthesis. Examples include streptomycin.
(26) "Glucopyranosyl antibiotic: trehalase and inositol biosynthesis fungicides" (Fungicide Resistance Action Committee (FRAC) code 26) inhibit trehalase in inositol biosynthesis pathway. Examples include validamycin.
(27) "Cyanoacetamideoxime fungicides (Fungicide Resistance Action Committee (FRAC) code 27) include cymoxanil.
(28) "Carbamate fungicides" (Fungicide Resistance Action Committee (FRAC) code 28) are considered multi-site inhibitors of fungal growth. They are proposed to interfere with the synthesis of fatty acids in cell membranes, which then disrupts cell membrane permeability. Propamacarb, propamacarb-hydrochloride, iodocarb, and prothiocarb are examples of this fungicide class.
(29) "Oxidative phosphorylation uncoupling fungicides" (Fungicide Resistance Action Committee (FRAC) code 29) inhibit fungal respiration by uncoupling oxidative phosphorylation. Inhibiting respiration prevents normal fungal growth and development. This class includes 2,6-dinitroanilines such as fluazinam, pyrimidonehydrazones such as ferimzone and dinitrophenyl crotonates such as dinocap, meptyldinocap and binapacryl.
(30) "Organo tin fungicides" (Fungicide Resistance Action Committee (FRAC) code 30) inhibit adenosine triphosphate (ATP) synthase in oxidative phosphorylation pathway. Examples include fentin acetate, fentin chloride and fentin hydroxide.
(31) "Carboxylic acid fungicides" (Fungicide Resistance Action Committee (FRAC) code 31) inhibit growth of fungi by affecting deoxyribonucleic acid (DNA) topoisomerase type II (gyrase). Examples include oxolinic acid.
(32) "Heteroaromatic fungicides" (Fungicide Resistance Action Committee (FRAC) code 32) are proposed to affect DNA/ribonucleic acid (RNA) synthesis. Heteroaromatic fungicides include isoxazole and isothiazolone fungicides. The isoxazoles include hymexazole and the isothiazolones include octhilinone.
(33) "Phosphonate fungicides" (Fungicide Resistance Action Committee (FRAC) code 33) include phosphorous acid and its various salts, including fosetyl-aluminum.
(34) "Phthalamic acid fungicides" (Fungicide Resistance Action Committee (FRAC) code 34) include teclofthalam.
(35) "Benzotriazine fungicides" (Fungicide Resistance Action Committee (FRAC) code 35) include triazoxide.
(36) "Benzene-sulfonamide fungicides" (Fungicide Resistance Action Committee (FRAC) code 36) include flusulfamide.
(37) "Pyridazinone fungicides" (Fungicide Resistance Action Committee (FRAC) code 37) include diclomezine.
(38) "Thiophene-carboxamide fungicides" (Fungicide Resistance Action Committee (FRAC) code 38) are proposed to affect ATP production. Examples include silthiofam.
(39) "Pyrimidinamide fungicides" (Fungicide Resistance Action Committee (FRAC) code 39) inhibit growth of fungi by affecting phospholipid biosynthesis and include diflumetorim.
(40) "Carboxylic acid amide (CAA) fungicides" (Fungicide Resistance Action Committee (FRAC) code 40) are proposed to inhibit phospholipid biosynthesis and cell wall deposition. Inhibition of these processes prevents growth and leads to death of the target fungus. Carboxylic acid amide fungicides include cinnamic acid amide, valinamide carbamate and mandelic acid amide fungicides. The cinnamic acid amides include dimethomorph and flumorph. The valinamide carbamates include benthiavalicarb, benthiavalicarb-isopropyl, iprovalicarb, valifenalate and valiphenal. The mandelic acid amides include mandipropamid, N [2-[4-[[3-(4-chlorophenyl)-2-propyn-1-yl]oxy]-3-methoxyphenyl]ethyl]-3-methyl-2-[(methylsulfonyl)amino]butanamide and N [2-[4-[[3-(4-chlorophenyl)-2-propyn-1 -yl] oxy] -3-methoxyphenyl] ethyl] -3-methyl-2-[(ethylsulfonyl)amino]butanamide.
(41) "Tetracycline antibiotic fungicides" (Fungicide Resistance Action Committee (FRAC) code 41) inhibit growth of fungi by affecting complex 1 nicotinamide adenine dinucleotide (NADH) oxidoreductase. Examples include oxytetracycline.
(42) "Thiocarbamate fungicides" (Fungicide Resistance Action Committee (FRAC) code 42) include methasulfocarb.
(43) "Benzamide fungicides" (Fungicide Resistance Action Committee (FRAC) code 43) inhibit growth of fungi by delocalization of spectrin-like proteins. Examples include acylpicolide fungicides such as fluopicolide.
(44) "Host plant defense induction fungicides" (Fungicide Resistance Action Committee (FRAC) code P) induce host plant defense mechanisms. Host plant defense induction fungicides include benzothiadiazoles, benzisothiazoles and thiadiazolecarboxamides. The benzothiadiazoles include acibenzolar-S-methyl. The benzisothiazoles include probenazole. The thiadiazolecarboxamides include tiadinil and isotianil.
(45) "Multi-site contact fungicides" inhibit fungal growth through multiple sites of action and have contact/preventive activity. This class of fungicides includes: (45.1) "copper fungicides" (Fungicide Resistance Action Committee (FRAC) code M1)", (45.2) "sulfur fungicides" (Fungicide Resistance Action Committee (FRAC) code M2), (45.3) "dithiocarbamate fungicides" (Fungicide Resistance Action Committee (FRAC) code M3), (45.4) "phthalimide fungicides" (Fungicide Resistance Action Committee (FRAC) code M4), (45.5) "chloronitrile fungicides" (Fungicide Resistance Action Committee (FRAC) code M5), (45.6) "sulfamide fungicides" (Fungicide Resistance Action Committee (FRAC) code M6), (45.7) "guanidine fungicides" (Fungicide Resistance Action Committee (FRAC) code M7), (45.8) "triazine fungicides" (Fungicide Resistance Action Committee (FRAC) code M8) and (45.9) "quinone fungicides" (Fungicide Resistance Action Committee (FRAC) code M9). "Copper fungicides" are inorganic compounds containing copper, typically in the copper(II) oxidation state; examples include copper oxychloride, copper sulfate and copper hydroxide, including compositions such as Bordeaux mixture (tribasic copper sulfate). "Sulfur fungicides" are inorganic chemicals containing rings or chains of sulfur atoms; examples include elemental sulfur. "Dithiocarbamate fungicides" contain a dithiocarbamate molecular moiety; examples include mancozeb, metiram, propineb, ferbam, maneb, thiram, zineb and ziram. "Phthalimide fungicides" contain a phthalimide molecular moiety; examples include folpet, captan and captafol. "Chloronitrile fungicides" contain an aromatic ring substituted with chloro and cyano; examples include chlorothalonil. "Sulfamide fungicides" include dichlofluanid and tolyfluanid. "Guanidine fungicides" include dodine, guazatine, iminoctadine albesilate and iminoctadine triacetate. "Triazine fungicides" include anilazine. "Quinone fungicides" include dithianon.
(46) "Fungicides other than fungicides of classes (1) through (45)" include certain fungicides whose mode of action may be unknown. These include: (46.1) "thiazole carboxamide fungicides" (Fungicide Resistance Action Committee (FRAC) code U5), (46.2) "phenylacetamide fungicides" (Fungicide Resistance Action Committee (FRAC) code U6), (46.3) "arylphenylketone fungicides" (Fungicide Resistance Action Committee (FRAC) code U8) and (46.4) "triazolopyrimidine fungicides". The thiazole carboxamides include ethaboxam. The phenylacetamides include cyflufenamid and *N*-[[(cyclopropylmethoxy)-amino][6-(difluoromethoxy)-2,3-difluorophenyl]-methylene]benzeneacetamide. The arylphenylketones include benzophenones such as metrafenone and benzoylpyridines such as pyriofenone. The triazolopyrimidines include ametoctradin. Class (46) (i.e. "Fungicides other than classes (1) through (45)") also includes bethoxazin, fluxapyroxad, neo-asozin (ferric methanearsonate), pyrrolnitrin, quinomethionate, tebufloquin, isofetamid, *N*-[2-[4-[[3-(4-chlorophenyl)-2-propyn-1-yl]oxy]-3-methoxyphenyl]ethyl]-3-methyl-2-[(methylsulfonyl)amino]butanamide, *N-*[2-[4-[[3-(4-chlorophenyl)-2-propyn-1-yl]oxy]-3-methoxyphenyl]ethyl]-3-methyl-2-[(ethylsulfonyl)amino]butanamide, 2-[[2-fluoro-5-(trifluoromethyl)phenyl]thio]-2-[3-(2-methoxyphenyl)-2-thiazolidinylidene]acetonitrile, 3-[5-(4-chlorophenyl)-2,3-dimethyl-3-isoxazolidinyl]pyridine, 4-fluorophenyl *N*-[1-[[[1-(4-cyanophenyl)ethyl]sulfonyl]methyl]propyl]carbamate, 5-chloro-6-(2,4,6-trifluorophenyl)-7-(4-methylpiperidin-1-yl)[1,2,4]triazolo[1,5-a]pyrimidine, *N*-(4-chloro-2-nitrophenyl)-N-ethyl-4-methylbenzenesulfonamide, *N*-[[(cyclopropylmethoxy)amino][6-(difluoromethoxy)-2,3-difluorophenyl]methylene]benzeneacetamide, *N'*-[4-[4-chloro-3-(trifluoromethyl)-phenoxy]-2,5-dimethylphenyl]-*N*-ethyl-*N*-methylmethanimidamide, 1-[(2-propenylthio)-carbonyl]-2-(1-methylethyl)-4-(2-methylphenyl)-5-amino-1*H*-pyrazol-3-one, *N'*-[4-[[3-[(4-chlorophenyl)methyl]-1,2,4-thiadiazol-5-yl]oxy]-2,5-dimethylphenyl]-*N*-ethyl-*N*-methyl-methanimidamide, 1,1-dimethylethyl *N*-[6-[[[[(1-methyl-1*H-*tetrazol-5-yl)phenylmethylene]-amino]oxy]methyl]-2-pyridinyl]carbamate, 3-butyn-1-yl *N*-[6-[[[[(1-methyl-1*H*-tetrazol-5-yl)phenylmethylene]amino]oxy]methyl]-2-pyridinyl]carbamate, 2,6-dimethyl-1*H*,5*H-*[1,4]dithiino[2,3-*c*:5,6-*c*']dipyrrole-1,3,5,7(2*H*,6*H*)-tetrone, 5-fluoro-2-[(4-methylphenyl)-methoxy]-4-pyrimidinamine, 5-fluoro-2-[(4-fluorophenyl)methoxy]-4-pyrimidinamine, α-[3-(4-chloro-2-fluorophenyl)-5-(2,4-difluorophenyl)isoxazol-4-yl]pyrid-3-ylmethanol, (a*S*)-[3-(4-chloro-2-fluorophenyl)-5-(2,4-difluorophenyl)isoxazol-4-yl]pyrid-3-ylmethanol and (α*R*)-[3-(4-chloro-2-fluorophenyl)-5-(2,4-difluorophenyl)isoxazol-4-yl]pyrid-3-ylmethanol.

Therefore of note is a mixture (i.e. composition) comprising a compound of Formula 1 and at least one fungicidal compound selected from the group consisting of the aforedescribed classes (1) through (46). Also of note is a composition comprising said mixture (in fungicidally effective amount) and further comprising at least one additional component selected from the group consisting of surfactants, solid diluents and liquid diluents. Of particular note is a mixture (i.e. composition) comprising a compound of Formula **1** and at least one fungicidal compound selected from the group of specific compounds listed above in connection with classes (1) through (46). Also of particular note is a composition comprising said mixture (in fungicidally effective amount) and further comprising at least one additional surfactant selected from the group consisting of surfactants, solid diluents and liquid diluents.

Examples of other biologically active compounds or agents with which compounds of this invention can be formulated are: insecticides such as abamectin, acephate, acetamiprid, acrinathrin, amidoflumet (S-1955), avermectin, azadirachtin, azinphos-methyl, bifenthrin, bifenazate, buprofezin, carbofuran, cartap, chlorantraniliprole, chlorfenapyr, chlorfluazuron, chlorpyrifos, chlorpyrifos-methyl, chromafenoride, clothianidin, cyantraniliprole (3-bromo-1-(3-chloro-2-pyridinyl)-*N*-[4-cyano-2-methyl-6-[(methylamino)carbonyl]phenyl]-1*H-*pyrazole-5-carboxamide), cyflumetofen, cyfluthrin, beta-cyfluthrin, cyhalothrin, lambda-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim (UR-50701), flufenoxuron, fonophos, halofenozide, hexaflumuron, hydramethylnon, imidacloprid, indoxacarb, isofenphos, lufenuron, malathion, metaflumizone, metaldehyde, methamidophos, methidathion, methomyl, methoprene, methoxychlor, metofluthrin, milbemycin oxime, monocrotophos, methoxyfenozide, nicotine, nitenpyram, nithiazine, novaluron, noviflumuron (XDE-007), oxamyl, parathion, parathion-methyl, permethrin, phorate, phosalone, phosmet, phosphamidon, pirimicarb, profenofos, profluthrin, pymetrozine, pyrafluprole, pyrethrin, pyridalyl, pyrifluquinazon, pyriprole, pyriproxyfen, rotenone, ryanodine, spinetoram, spinosad, spirodiclofen, spiromesifen (BSN 2060), spirotetramat, sulprofos, tebufenozide, teflubenzuron, tefluthrin, terbufos, tetrachlorvinphos, thiacloprid, thiamethoxam, thiodicarb, thiosultap-sodium, tolfenpyrad, tralomethrin, triazamate, trichlorfon and triflumuron; and biological agents including entomopathogenic bacteria, such as *Bacillus thuringiensis* subsp. *aizawai, Bacillus thuringiensis* subsp. *kurstaki*, and the encapsulated delta endotoxins of *Bacillus thuringiensis* (e.g., Cellcap, MPV, MPVII); entomopathogenic fungi, such as green muscardine fungus; and entomopathogenic virus including baculovirus, nucleopolyhedro virus (NPV) such as HzNPV, AfNPV; and granulosis virus (GV) such as CpGV.

Compounds of this invention and compositions thereof can be applied to plants genetically transformed to express proteins toxic to invertebrate pests (such as *Bacillus thuringiensis* delta endotoxins). The effect of the exogenously applied fungicidal compounds of this invention may be synergistic with the expressed toxin proteins.

General references for agricultural protectants (i.e. insecticides, fungicides, nematocides, acaricides, herbicides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Famham, Surrey, U.K., 2001.

In certain instances, combinations of a compound of this invention with other biologically active (particularly fungicidal) compounds or agents (i.e. active ingredients) can result in a greater-than-additive (i.e. synergistic) effect. Reducing the quantity of active ingredients released in the environment while ensuring effective pest control is always desirable. When synergism of fungicidal active ingredients occurs at application rates giving agronomically satisfactory levels of fungal control, such combinations can be advantageous for reducing crop production cost and decreasing environmental load.

Of note is a combination of a compound of Formula **1** with at least one other fungicidal active ingredient. Of particular note is such a combination where the other fungicidal active ingredient has different site of action from the compound of Formula **1.** In certain instances, a combination with at least one other fungicidal active ingredient having a similar spectrum of control but a different site of action will be particularly advantageous for resistance management. Thus, a composition of the present invention can further comprise a biologically effective amount of at least one additional fungicidal active ingredient having a similar spectrum of control but a different site of action.

Of particular note are compositions which in addition to compound of Formula **1** include at least one compound selected from the group consisting of (1) alkylenebis(dithiocarbamate) fungicides; (2) cymoxanil; (3) phenylamide fungicides; (4) pyrimidinone fungicides; (5) chlorothalonil; (6) carboxamides acting at complex II of the fungal mitochondrial respiratory electron transfer site; (7) quinoxyfen; (8) metrafenone; (9) cyflufenamid; (10) cyprodinil; (11) copper compounds; (12) phthalimide fungicides; (13) fosetyl-aluminum; (14) benzimidazole fungicides; (15) cyazofamid; (16) fluazinam; (17) iprovalicarb; (18) propamocarb; (19) validomycin; (20) dichlorophenyl dicarboximide fungicides; (21) zoxamide; (22) fluopicolide; (23) mandipropamid; (24) carboxylic acid amides acting on phospholipid biosynthesis and cell wall deposition; (25) dimethomorph; (26) non-DMI sterol biosynthesis inhibitors; (27) inhibitors of demethylase in sterol biosynthesis; (28) *bc*₁ complex fungicides; and salts of compounds of (1) through (28).

Further descriptions of classes of fungicidal compounds are provided below.

Pyrimidinone fungicides (group (4)) include compounds of Formula **A1** 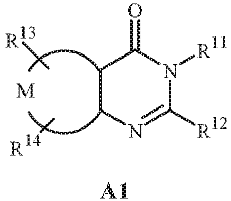 wherein M forms a fused phenyl, thiophene or pyridine ring; R¹¹ is C₁-C₆ alkyl; R¹² is C₁-C₆ alkyl or C₁-C₆ alkoxy; R¹³ is halogen; and R¹⁴ is hydrogen or halogen.

Pyrimidinone fungicides are described in PCT Patent Application Publication WO 94/26722 and U.S. Patents 6,066,638, 6,245,770, 6,262,058 and 6,277,858. Of note are pyrimidinone fungicides selected from the group: 6-bromo-3-propyl-2-propyloxy-4(3*H*)-quinazolinone, 6,8-diiodo-3-propyl-2-propyloxy-4(3*H*)-quinazolinone, 6-iodo-3-propyl-2-propyloxy-4(3*H*)-quinazolinone (proquinazid), 6-chloro-2-propoxy-3-propylthieno[2,3-*d*]pyrimidin-4(3*H*)-one, 6-bromo-2-propoxy-3-propylthieno[2,3-*d*]pyrimidin-4(3*H*)-one, 7-bromo-2-propoxy-3-propylthieno[3,2-*d*]pyrimidin-4(3*H*)-one, 6-bromo-2-propoxy-3-propylpyrido[2,3-*d*]pyrimidin-4(3*H*)-one, 6,7-dibromo-2-propoxy-3-propylthieno[3,2-*d*]pyrimidin-4(3*H*)-one, and 3-(cyclopropylmethyl)-6-iodo-2-(propylthio)pyrido[2,3-*d*]pyrimidin-4(3*H*)-one.

Sterol biosynthesis inhibitors (group (27)) control fungi by inhibiting enzymes in the sterol biosynthesis pathway. Demethylase-inhibiting fungicides have a common site of action within the fungal sterol biosynthesis pathway, involving inhibition of demethylation at position 14 of lanosterol or 24-methylene dihydrolanosterol, which are precursors to sterols in fungi. Compounds acting at this site are often referred to as demethylase inhibitors, DMI fungicides, or DMIs. The demethylase enzyme is sometimes referred to by other names in the biochemical literature, including cytochrome P-450 (14DM). The demethylase enzyme is described in, for example, J. Biol. Chem. 1992, 267, 13175-79 and references cited therein. DMI fungicides are divided between several chemical classes: azoles (including triazoles and imidazoles), pyrimidines, piperazines and pyridines. The triazoles include azaconazole, bromuconazole, cyproconazole, difenoconazole, diniconazole (including diniconazole-M), epoxiconazole, etaconazole, fenbuconazole, fluquinconazole, flusilazole, flutriafol, hexaconazole, imibenconazole, ipconazole, metconazole, myclobutanil, penconazole, propiconazole, prothioconazole, quinconazole, simeconazole, tebuconazole, tetraconazole, triadimefon, triadimenol, triticonazole and uniconazole. The imidazoles include clotrimazole, econazole, imazalil, isoconazole, miconazole, oxpoconazole, prochloraz and triflumizole. The pyrimidines include fenarimol, nuarimol and triarimol. The piperazines include triforine. The pyridines include buthiobate and pyrifenox. Biochemical investigations have shown that all of the above mentioned fungicides are DMI fungicides as described by K. H. Kuck et al. in Modern Selective Fungicides - Properties, Applications and Mechanisms of Action, H. Lyr (Ed.), Gustav Fischer Verlag: New York, 1995, 205-258.

*bc*₁ Complex Fungicides (group 28) have a fungicidal mode of action which inhibits the *bc*₁ complex in the mitochondrial respiration chain. The *bc*₁ complex is sometimes referred to by other names in the biochemical literature, including complex III of the electron transfer chain, and ubihydroquinone:cytochrome *c* oxidoreductase. This complex is uniquely identified by Enzyme Commission number EC1.10.2.2. The *bc*₁ complex is described in, for example, J. Biol. Chem. 1989, 264, 14543-48; Methods Enzymol. 1986, 126, 253-71; and references cited therein. Strobilurin fungicides such as azoxystrobin, dimoxystrobin, enestroburin (SYP-Z071), fluoxastrobin, kresoxim-methyl, metominostrobin, orysastrobin, picoxystrobin, pyraclostrobin, pyrametostrobin, pyraoxystrobin and trifloxystrobin are known to have this mode of action (H. Sauter et al., Angew. Chem. Int. Ed 1999, 38, 1328-1349). Other fungicidal compounds that inhibit the *bc*₁ complex in the mitochondrial respiration chain include famoxadone and fenamidone.

Alkylenebis(dithiocarbamate)s (group (1)) include compounds such as mancozeb, maneb, propineb and zineb. Phenylamides (group (3)) include compounds such as metalaxyl, benalaxyl, furalaxyl and oxadixyl. Carboxamides (group (6)) include compounds such as boscalid, carboxin, fenfuram, flutolanil, furametpyr, mepronil, oxycarboxin, thifluzamide, penthiopyrad and *N*-[2-(1,3-dimethylbutyl)phenyl]-5-fluoro-1,3-dimethyl-1*H-*pyrazole-4-carboxamide (PCT Patent Publication WO 2003/010149), and are known to inhibit mitochondrial function by disrupting complex II (succinate dehydrogenase) in the respiratory electron transport chain. Copper compounds (group (11)) include compounds such as copper oxychloride, copper sulfate and copper hydroxide, including compositions such as Bordeaux mixture (tribasic copper sulfate). Phthalimides (group (12)) include compounds such as folpet and captan. Benzimidazole fungicides (group (14)) include benomyl and carbendazim Dichlorophenyl dicarboximide fungicides (group (20)) include chlozolinate, dichlozoline, iprodione, isovaledione, myclozolin, procymidone and vinclozolin.

Non-DMI sterol biosynthesis inhibitors (group (26)) include morpholine and piperidine fungicides. The morpholines and piperidines are sterol biosynthesis inhibitors that have been shown to inhibit steps in the sterol biosynthesis pathway at a point later than the inhibitions achieved by the DMI sterol biosynthesis (group (27)). The morpholines include aldimorph, dodemorph, fenpropimorph, tridemorph and trimorphamide. The piperidines include fenpropidin.

Of further note are combinations of compounds of Formula 1 with azoxystrobin, kresoxim-methyl, trifloxystrobin, pyraclostrobin, picoxystrobin, dimoxystrobin, metominostrobin/fenominostrobin, carbendazim, chlorothalonil, quinoxyfen, metrafenone, cyflufenamid, fenpropidine, fenpropimorph, bromuconazole, cyproconazole, difenoconazole, epoxiconazole, fenbuconazole, flusilazole, hexaconazole, ipconazole, metconazole, penconazole, propiconazole, proquinazid, prothioconazole, tebuconazole, triticonazole, famoxadone, prochloraz, penthiopyrad and boscalid (nicobifen).

Preferred for better control of plant diseases caused by fungal plant pathogens (e.g., lower use rate or broader spectrum of plant pathogens controlled) or resistance management are mixtures of a compound of this invention with a fungicide selected from the group: azoxystrobin, kresoxim-methyl, trifloxystrobin, pyraclostrobin, picoxystrobin, dimoxystrobin, metominostrobin/fenominostrobin, quinoxyfen, metrafenone, cyflufenamid, fenpropidine, fenpropimorph, cyproconazole, epoxiconazole, flusilazole, metconazole, propiconazole, proquinazid, prothioconazole, tebuconazole, triticonazole, famoxadone and penthiopyrad.

The following TESTS demonstrate the control efficacy of compounds of this invention on specific pathogens. The pathogen control protection afforded by the compounds is not limited, however, to these species. See Index Tables A-E for compound descriptions. The following abbreviations are used in the Index Tables: Me means methyl, Et means ethyl, *n*-Pr means *n*-propyl, *i*-Pr means *iso*-propyl, *c*-Pr means cyclopropyl, *i*-Bu means *iso*-butyl, Ph means phenyl, MeO means methoxy, EtO means ethoxy and Bn means benzyl. The abbreviation "Cmpd." stands for "Compound", and the abbreviation "Ex." stands for "Example" and is followed by a number indicating in which example the compound is prepared. ¹⁹F NMR spectra data are reported in ppm relative to trichlorofluoromethane, in CDCl₃ solution unless indicated otherwise. ¹⁹F NMR experiments were run on a Bruker Avance III 500 MHz NMR spectrometer, fitted with a 5 mm (1H/BBF) probe. Mass spectra (MS) are reported as the molecular weight of the highest isotopic abundance positively charged parent ion (M+1) formed by addition of H+ (molecular weight of 1) to the molecule, or the highest isotopic abundance negatively charged ion (M-1) formed by loss of H+ (molecular weight of 1), observed by mass spectrometry using electrospray ionization (ESI).

The definition of J in Index Tables A-D is shown in Exhibit B above, and the substituent R^{7a} attached to the J is hydrogen.

Compounds 25, 26, 32, 43, 46, 56, 60, 63, 66, 70-73, 75, 76, 78, 90, 93, 102, 103, 108, 111, 114, 115, 117 and 124 of Index Table B and compounds 118 and 130 of Index Table C are the compounds of the invention. All the other compounds are not.

### INDEX TABLE A

| 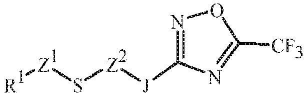 | | | | | |
|---|---|---|---|---|---|
| Cmpd. | R¹ | Z¹ | Z² | J | ¹⁹F NMR |
| 1 | Et | - | - | J-80 | -65.23 |
| 2 | Et | - | - | J-64 | -65.69 |
| 5 | CF₃CH₂ | - | - | J-80 | -65.22, -66.19 |
| 6 | Et | - | - | J-79 | -65.56 |
| 19 | Me | - | - | J-64 | -65.32 |
| 33 | MeOC(=O)CH₂ | - | - | J-64 | -65.25 |
| 38 | 4-Cl-Ph | - | - | J-64 | -65.28 |
| 49 | CF₃CH₂ | - | - | J-64 | -65.28, -66.48 |
| 52 | Ph | - | - | J-64 | -65.29 |
| 57 | CH₂=CHCH₂ | - | - | J-64 | -65.32 |
| 58 | *n*-Pr | - | - | J-64 | -65.25 |
| 67 | i-Pr | - | - | J-64 | -65.33 |
| 69 | i-Bu | - | - | J-64 | -65.33 |
| 81 | Bn | - | - | J-64 | -65.31 |
| 89 | Me | - | - | J-66 | -65.21 |
| 116 (Ex. 3) | Me | - | - | J-63 | -65.37 |
| 174 | Me | - | - | J-40 | -65.26 |
| 175 | EtOC=OCH₂ | - | CH₂ | J-63 | -65.36 |
| 178 | MeOC=OCH₂ | - | CH₂ | J-63 | -65.37 |
| 184 | PhCH₂ | - | CH₂ | J-63 | -65.36 |
| 225 | Me | - | CH₂ | J-63 | -65.37 |
| 227 | Et | - | CH₂ | J-63 | -65.37 |
| 229 | *n*-Pr | - | CH₂ | J-63 | -65.36 |
| 232 | *i*-Pr | - | CH₂ | J-63 | -65.42 |
| 235 | *i*-Bu | - | CH₂ | J-63 | -65.37 |
| 236 | *n*-Bu | - | CH₂ | J-63 | -65.43 |
| 243 | CH=CCH₂ | - | CH₂ | J-63 | -65.35 |
| 246 | MeOC=OCH₂CH₂ | - | CH₂ | J-63 | -65.36 |
| 249 | EtOC=OCH₂CH₂ | - | CH₂ | J-63 | -65.38 |

### INDEX TABLE B

| 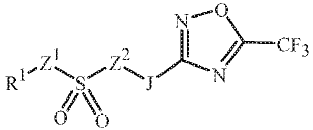 | | | | | | |
|---|---|---|---|---|---|---|
| Cmpd. | R¹ | Z¹ | Z² | J | MS | ¹⁹F NMR |
| 4 | Et | - | - | J-64 | | -65.16 |
| 7 | 2-CF₃-Ph | NH | - | J-63 | | -60.12, -65.33 |
| 8 | 3-Cl-Ph | NH | - | J-63 | | -65.30 |
| 9 | 3-CF₃-Ph | NH | - | J-63 | | -62.84, -65.30 |
| 10 | 2,4-di-Cl-Ph | NH | - | J-63 | | -65.29 |
| 11 | 4-Me-Ph | NH | - | J-63 | | -65.31 |
| 12 | 4-F-Ph | NH | - | J-63 | | -65.30, -114.78 |
| 13 | 4-Cl-Ph | NH | - | J-63 | | -65.30 |
| 14 | 2-Cl-Ph | NH | - | J-63 | | -65.30 |
| 15 | 4-CF₃-Ph | NH | - | J-63 | | -62.42, -65.32 |
| 16 | 4-Br-Ph | NH | - | J-63 | | -65.30 |
| 17 | 4-I-Ph | NH | - | J-63 | | -65.29 |
| 18 | 2,4-di-F-Ph | NH | - | J-63 | | -65.31, -110.42, -123.11 |
| 21 | Me | - | - | J-64 | | -65.15 |
| 22 | Ph | - | - | J-27 | | -65.34 |
| 23 | Me | - | - | J-79 | | -65.17 |
| 24 | Me | - | - | J-78 | | -65.14 |
| 25 | (MeO)₂CHCH₂ | NH | - | J-63 | | -65.26 |
| 26 (Ex. 9) | Me | - | NH | J-63 | | -65.35 |
| 27 | Et | - | NH | J-63 | | -65.35 |
| 28 | Ph | N(Me) | - | J-63 | | -65.29 |
| 29 | Ph | N(Et) | - | J-63 | | -65.29 |
| 30 | Ph | N(CH₂CH=CH₂) | - | J-63 | | -65.29 |
| 31 | Ph | N(C(=O)OMe) | - | J-63 | | -65.25 |
| 32 | Ph | - | N(Me) | J-63 | | -65.42 |
| 35 | MeOC(=O)CH₂ | - | - | J-64 | | -65.15 |
| 36 | 3-Cl-Ph | - | - | J-64 | | -65.17 |
| 37 | 4-Cl-Ph | - | - | J-64 | | -65.17 |
| 39 | 2-F-4-CF₃-Ph | NH | - | J-63 | | -62.56, -65.30, -127.83 |
| 40 (Ex. 2) | Me | NH | - | J-63 | | -65.30 |
| 41 | Me | N(Me) | - | J-63 | | -65.29 |
| 42 | 2,6-di-Cl-Ph | NH | - | J-63 | | -65.27 |
| 43 | Ph | - | NH | J-63 | | -65.34 |
| 44 | 2,4,6-tri-Cl-Ph | NH | - | J-63 | | -65.27 |
| 45 | Ph | - | NH | J-77 | | -65.36 |
| 46 | CH₃C(=O) | N(Me) | - | J-63 | | -65.24 |
| 47 | 2,6-di-F-Ph | NH | - | J-63 | | -65.30, -117.90 |
| 48 | 3,4-di-Cl-Ph | NH | - | J-63 | | -65.30 |
| 50 | CF₃CH₂ | - | - | J-64 | | -60.92, -65.14 |
| 54 | Ph | - | - | J-64 | | -65.18 |
| 55 | 2,5-di-Cl-Ph | NH | - | J-63 | | -65.30 |
| 56 | 3,5-di-Cl-Ph | NH | - | J-63 | | -65.31 |
| 59 | n-Pr | - | - | J-64 | | -65.22 |
| 60 | CH₂=CHCH₂ | - | - | J-64 | | -65.23 |
| 61 | Ph | - | - | J-73 | | -65.37 |
| 62 | Ph | NH | - | J-77 | | -65.38 |
| 63 | Ph | - | NH | J-64 | | -65.28 |
| 64 | Bn | - | - | J-73 | | -65.39 |
| 65 | Et | - | - | J-27 | | -65.33 |
| 66 | 4-EtOC(=O)-Ph | NH | - | J-63 | | -65.29 |
| 68 | *i*-Pr | - | - | J-64 | | -65.16 |
| 70 | i-Bu | - | - | J-64 | | -65.17 |
| 71 | 4-F-Ph | - | NH | J-64 | | -65.17, -103.46 |
| 72 | 4-Me-Ph | - | NH | J-64 | | -65.29 |
| 73 | 2-Cl-Ph | - | NH | J-64 | | -65.25 |
| 74 | 3-Cl-Ph | - | NH | J-64 | | -65.23 |
| 75 | 4-Cl-Ph | - | NH | J-64 | | -65.27 |
| 76 | 4-MeO-Ph | - | NH | J-64 | | -65.28 |
| 77 | 4-CF₃O-Ph | - | NH | J-64 | | -58.51, -66.50^{a} |
| 78 | Me | - | NHCH₂ | J-63 | | -65.35 |
| 79 | CF₃CH₂ | - | NH | J-63 | | -63.32, -65.33 |
| 82 | Bn | - | - | J-64 | | -65.10 |
| 83 | 3-CF₃-Ph | - | NH | J-64 | | -63.40, -66.48^{a} |
| 84 | 4-CF₃-Ph | - | NH | J-64 | | -63.67, -66.50^{a} |
| 85 | 2-CF₃-Ph | - | NH | J-64 | | -57.87, -65.30 |
| 86 | ClCH₂ | - | NH | J-63 | | -65.33 |
| 88 | Et | NH | - | J-63 | | -65.29 |
| 90 | Ph | - | NHCH₂ | J-63 | | -65.36 |
| 91 | Et | N(Me) | - | J-63 | | -65.30 |
| 92 | Et | N(Et) | - | J-63 | | -65.30 |
| 93 | 3-Cl-Ph | - | NH | J-63 | | -65.38 |
| 94 | Me | N(OMe) | - | J-63 | | -65.25 |
| 95 | Et | N(OMe) | - | J-63 | | -65.29 |
| 96 | ClCH₂ | - | - | J-63 | | -65.35 |
| 99 | n-Pr | NH | - | J-63 | | -65.30 |
| 100 | Ph | NH | - | J-63 | | -65.31 |
| 101 | Me | - | - | J-73 | | -65.34 |
| 102 | 2-Cl-Ph | - | NH | J-63 | | -65.27 |
| 103 | 2-CF₃-Ph | - | NH | J-63 | | -57.98, -65.41 |
| 104 | 4-Cl-Ph | - | NH | J-63 | | -65.40 |
| 105 | 3-CF₃-Ph | - | NH | J-63 | | -63.01, -65.43 |
| 106 | 4-CF₃-Ph | - | NH | J-63 | | -63.29, -65.40 |
| 107 | Me | - | O | J-64 | | -65.24 |
| 108 (Ex. 8) | Me | - | O | J-63 | | -65.33 |
| 109 | i-Pr | NH | - | J-63 | | -65.32 |
| 110 | Cl₃C | - | - | J-63 | | -65.30 |
| 111 | CH₃C(=O) | N(OMe) | - | J-63 | | -65.24 |
| 112 | Me | - | - | J-66 | | -65.03 |
| 113 | CF₃ | - | - | J-63 | | -65.18, -77.92 |
| 114 | CH₃C(=O) | N(Et) | - | J-63 | | -65.24 |
| 115 | Et | - | CH₂ | J-63 | | -65.32 |
| 117 (Ex. 5) | Me | - | - | J-63 | | -65.26 |
| 119 | Cl₂CH | - | - | J-63 | | -65.23 |
| 120 | 4-Me-Ph | - | NH | J-63 | | -65.41 |
| 121 | 4-F-Ph | - | NH | J-63 | | -65.42, -103.23 |
| 122 | 4-MeO-Ph | - | NH | J-63 | | -65.40 |
| 123 | 4-CF₃O-Ph | - | NH | J-63 | | -57.52, -65.40 |
| 124 (Ex. 1) | Me | - | CH₂ | J-63 | | -65.33 |
| 125 | Ph | - | CH₂ | J-63 | | -65.28 |
| 126 | 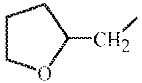 | NH | - | J-63 | | -65.28 |
| 127 | 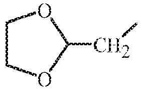 | NH | - | J-63 | | -65.28 |
| 128 | 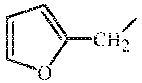 | N(Me) | - | J-63 | | -65.28 |
| 129 | MeOCH₂CH₂ | NH | - | J-63 | | -65.30 |
| 131 | 4-MeO-Ph | NH | - | J-63 | | -65.31 |
| 132 | MeOCH₂CH₂CH₂ | NH | - | J-63 | | -65.29 |
| 134 | *c*-Pr | - | CH₂ | J-63 | | -65.33 |
| 138 | 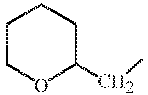 | NH | - | J-63 | | -65.28 |
| 139 | 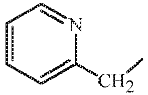 | NH | - | J-63 | | -65.28 |
| 140 | 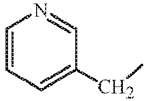 | NH | - | J-63 | | -65.26 |
| 142 | 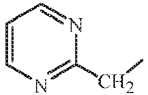 | NH | - | J-63 | | -65.26 |
| 144 | *c*-Pr | - | NH | J-63 | | -65.35 |
| 145 | Me | N(Me) | CH₂ | J-63 | | -65.34 |
| 146 | Me | NH | CH₂ | J-63 | | -65.33 |
| 149 | 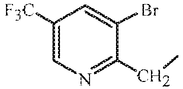 | NH | - | J-63 | | -62.26, -65.29 |
| 151 | 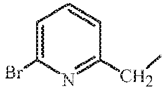 | NH | - | J-63 | | -65.24 |
| 154 | CH=CCH₂ | NH | CH₂ | J-63 | | -65.33 |
| 155 | *c*-Pr | - | NH | J-64 | | -65.28 |
| 156 | Me | - | NH | J-64 | | -65.29 |
| 158 | *c*-Pr | - | N(Me)CH₂ | J-63 | | -65.35 |
| 159 | CH₂=CHCH₂ | NH | CH₂ | J-63 | | -65.34 |
| 160 | n-Pr | NH | CH₂ | J-63 | | -65.33 |
| 164 | Me | - | N(Me)CH₂ | J-63 | | -65.38 |
| 165 | Me | - | N(C=OMe)CH₂ | J-63 | | -65.41 |
| 166 | Et | - | N(Me)CH₂ | J-63 | | -65.39 |
| 167 | Et | - | N(C=OMe)CH₂ | J-63 | | -65.35 |
| 168 | Me | - | NHCH₂ | J-40 | | -65.39 |
| 170 | Et | - | - | J-60 | | -65.32 |
| 171 | Ph | - | - | J-60 | | -65.30 |
| 172 | Et | - | NHCH₂ | J-63 | | -65.33 |
| 173 | *c*-Pr | - | NHCH₂ | J-63 | | -65.33 |
| 177 | EtOC=OCH₂ | - | CH₂ | J-63 | | -65.30 |
| 180 | MeOC=OCH₂ | - | CH₂ | J-63 | | -65.35 |
| 181 | 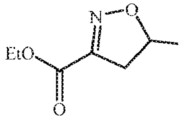 | - | - | J-63 | 418 (ES⁻) | |
| 182 | Me | - | CH₂CH₂ | J-27 | | -65.40 |
| 183 | Me | - | (CH₂)₃ | J-27 | | -65.41 |
| 186 | PhCH₂ | - | CH₂ | J-63 | | -65.30 |
| 187 | 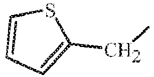 | NH | - | J-63 | | -65.26 |
| 188 | 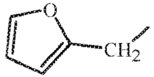 | NH | - | J-63 | | -65.26 |
| 189 | 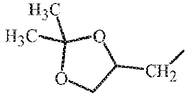 | NH | - | J-63 | | -65.27 |
| 190 | 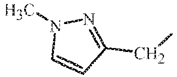 | NH | - | J-63 | | -65.27 |
| 191 | 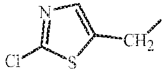 | NH | - | J-63 | | -65.23 |
| 192 | 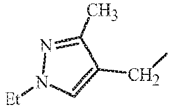 | NH | - | J-63 | | -65.27 |
| 196 | Ph | NH | - | J-64 | 369 (ES⁻) | |
| 197 | Me | - | N(Me) | J-63 | | -65.36 |
| 198 | *c*-Pr | - | N(Me) | J-63 | | -65.37 |
| 200 | (MeO)₂CHCH₂ | NH | CH₂ | J-63 | | -65.34 |
| 201 | i-Bu | NH | CH₂ | J-63 | | -65.33 |
| 203 | Me | - | N(SO₂Me) | J-40 | 390 (ES⁻) | |
| 204 | Et | N(Me) | CH₂ | J-63 | | -65.41 |
| 205 | *c*-Pr | N(Me) | CH₂ | J-63 | | -65.34 |
| 207 | Me | N(OMe) | - | J-63 | | -65.36 |
| 208 | *c*-Pr | NH | CH₂ | J-63 | | -65.33 |
| 209 | MeOCH₂CH₂ | NH | CH₂ | J-63 | | -65.34 |
| 210 | *i*-Pr | NH | CH₂ | J-63 | | -65.41 |
| 211 | Et | NH | CH₂ | J-63 | | -65.34 |
| 213 | Me | - | NH | J-40 | 312 (ES⁻) | |
| 214 | Me | - | CH₂ | J-40 | | -65.37 |
| 216 | Me | - | (CH₂)₃ | J-63 | | -65.40 |
| 217 | Et | - | (CH₂)₃ | J-63 | | -65.38 |
| 218 | *c*-Pr | - | (CH₂)₃ | J-63 | | -65.38 |
| 219 | Me | - | CH₂ | J-74 | | -66.35 |
| 220 | Et | - | CH(Ph) | J-63 | | -65.31 |
| 221 | *n*-Pr | - | NHCH₂ | J-63 | | -65.36 |
| 222 | Me | - | CH₂CH₂ | J-63 | | -65.36 |
| 223 | Me | - | CH₂CH₂ | J-73 | | -65.42 |
| 224 | Me | - | (CH₂)₃ | J-73 | | -65.46 |
| 231 | *n*-Pr | - | CH₂ | J-63 | | -65.31 |
| 234 | *i*-Pr | - | CH₂ | J-63 | | -65.37 |
| 237 | Et | - | CH₂CH₂ | J-63 | | -65.36 |
| 238 | *c*-Pr | - | CH₂CH₂ | J-63 | | -65.36 |
| 240 | *i*-Bu | - | CH₂ | J-63 | | -65.33 |
| 242 | *n*-Bu | - | CH₂ | J-63 | | -65.33 |
| 245 | CH=CCH₂ | - | CH₂ | J-63 | | -65.35 |
| 248 | MeOC(=O)CH₂CH₂ | - | CH₂ | J-63 | | -65.33 |
| 251 | EtOC(=O)CH₂CH₂ | - | CH₂ | J-63 | | -65.30 |
| | a. ¹⁹F NMR run in acetone-*d*₆ | | | | | |

### INDEX TABLE C

| 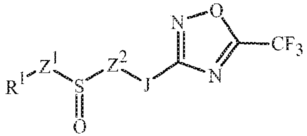 | | | | | |
|---|---|---|---|---|---|
| Cmpd. | R¹ | Z¹ | Z² | J | ¹⁹F NMR |
| 3 | Et | - | - | J-64 | -65.22 |
| 20 | Me | - | - | J-64 | -65.15 |
| 34 | MeOC(=O)CH₂ | - | - | J-64 | -65.21 |
| 51 | CF₃CH₂ | - | - | J-64 | -60.57, -65.18 |
| 53 | Ph | - | - | J-64 | -65.24 |
| 97 | ClCH₂ | - | - | J-63 | -65.29 |
| 118 (Ex. 4) | Me | - | - | J-63 | -65.30 |
| 130 | Cl₂CH | - | - | J-63 | -65.26 |
| 176 | EtOC=OCH₂ | - | CH₂ | J-63 | -65.33 |
| 179 | MeOC=OCH₂ | - | CH₂ | J-63 | -65.40 |
| 185 | PhCH₂ | - | CH₂ | J-63 | -65.38 |
| 226 | Me | - | CH₂ | J-63 | -65.36 |
| 228 | Et | - | CH₂ | J-63 | -65.39 |
| 230 | *n*-Pr | - | CH₂ | J-63 | -65.34 |
| 233 | *i*-Pr | - | CH₂ | J-63 | -65.33 |
| 239 | *i*-Bu | - | CH₂ | J-63 | -65.38 |
| 241 | *n*-Bu | - | CH₂ | J-63 | -65.45 |
| 244 | CH=CCH₂ | - | CH₂ | J-63 | -65.37 |
| 247 | MeOC(=O)CH₂CH₂ | - | CH₂ | J-63 | -65.36 |
| 250 | EtOC(=O)CH₂CH₂ | - | CH₂ | J-63 | -65.38 |

### INDEX TABLE D

| 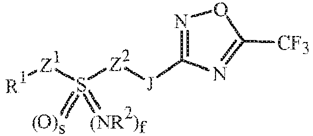 | | | | | | | | |
|---|---|---|---|---|---|---|---|---|
| Cmpd. | R¹ | Z¹ | s | f | R² | Z² | J | ¹⁹F NMR |
| 80 (Ex. 6) | Me | - | 0 | 1 | C≡N | - | J-63 | -65.26 |
| 87 (Ex. 7) | Me | - | 1 | 1 | C≡N | - | J-63 | -65.19 |

### INDEX TABLE E

| 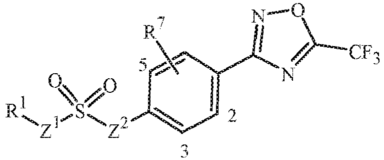 | | | | | | |
|---|---|---|---|---|---|---|
| Cmpd. | R¹ | Z¹ | Z² | R⁷ | MS | ¹⁹F NMR |
| 136 | Me | - | - | 3-NO₂ | | -65.20 |
| 137 | Me | - | - | 3-CF₃ | | -56.97, -65.22 |
| 147 | Me | - | - | 2-Cl | 327 (ES⁺) | |
| 148 | Me | - | NH | 3-Cl | 340 (ES⁺) | |
| 157 | Me | - | - | 3-Cl | | -65.28 |
| 161 | Me | - | - | 3-F | | -65.28 |
| 162 | Me | - | - | 3-MeO | | -65.28 |
| 163 | Me | - | - | 3-Me | | -65.32 |
| 169 | Me | - | - | 3-MeSO₂ | 369 (ES⁻) | |
| 193 | Me | NH | - | 3-Cl | 340 (ES⁻) | |
| 194 | Me | - | NH | 2-Cl | 342 (ES⁻) | |
| 195 | Me | - | - | 2-Br | 370 (ES⁻) | |
| 202 | Me | - | - | 2-Me | 307 (ES⁻) | |
| 212 | Me | - | - | 2-MeO | | -65.21 |
| 215 | Me | - | - | 2-NO₂ | | -63.20 |

### BIOLOGICAL EXAMPLES OF THE INVENTION

General protocol for preparing test suspensions for Tests A-E: the test compounds were first dissolved in acetone in an amount equal to 3% of the final volume and then suspended at the desired concentration (in ppm) in acetone and purified water (50/50 mix by volume) containing 250 ppm of the surfactant PEG400 (polyhydric alcohol esters). The resulting test suspensions were then used in Tests A-E. Spraying a 200 ppm test suspension to the point of run-off on the test plants was the equivalent of a rate of 800 g/ha.

### TEST A

The test solution was sprayed to the point of run-off on soybean seedlings. The following day the seedlings were inoculated with a spore suspension of *Phakopsora pachyrhizi* (the causal agent of Asian soybean rust) and incubated in a saturated atmosphere at 22 °C for 24 h and then moved to a growth chamber at 22 °C for 8 days, after which time visual disease ratings were made.

### TEST B

The test solution was sprayed to the point of run-off on grape seedlings. The following day the seedlings were inoculated with a spore suspension of *Plasmopara viticola* (the causal agent of grape downy mildew) and incubated in a saturated atmosphere at 20 °C for 24 h, and then moved to a growth chamber at 20 °C for 6 days, and again incubated in a saturated atmosphere at 20 °C for 24 h, after which time disease ratings were made.

### TEST C

The test solution was sprayed to the point of run-off on wheat seedlings. The following day the seedlings were inoculated with a spore suspension of *Zymoseptoria tritici* (the causal agent of wheat leaf blotch) and incubated in a saturated atmosphere at 24 °C for 48 h, and then moved to a growth chamber at 20 °C for 17 days, after which time disease ratings were made.

### TEST D

The test solution was sprayed to the point of run-off on wheat seedlings. The following day the seedlings were inoculated with a spore suspension of *Puccinia recondita* f. sp. *tritici* (the causal agent of wheat leaf rust) and incubated in a saturated atmosphere at 20 °C for 24 h, and then moved to a growth chamber at 20 °C for 6 days, after which time disease ratings were made.

### TEST E

The test solution was sprayed to the point of run-off on wheat seedlings. The following day the seedlings were inoculated with a spore suspension of *Erysiphe graminis* f. sp. *tritici,* (the causal agent of wheat powdery mildew) and incubated in growth chamber a saturated atmosphere at 20 °C for 8 days, after which time disease ratings were made.

Results for Tests A-E are given in Table A. In the Table, a rating of 100 indicates 100% disease control and a rating of 0 indicates no disease control (relative to the controls). Unless otherwise indicated, the rating at which the test suspension was used is shown in the column titled "Rate in ppm". An asterisk "*" next to the rating value indicates a 50 ppm test suspension was used (e.g., Test A for compound numbers 7, 8, 10, 11, 12, 15, 43, 53, 54, 61 and 72). In Table A, compounds 25, 26, 32, 43, 46, 56, 60, 63, 66, 70-73, 75, 76, 78 90, 93, 102, 103, 108, 111, 114, 115, 117, 118, 124 and 130 are the compounds of the invention. All the other compounds are not.

**TABLE A**

| Cmpd No. | Rate in ppm | Test A | Test B | Test C | Test D | Test E |
|---|---|---|---|---|---|---|
| 1 | 200 | - | 15 | 0 | 38 | 0 |
| 2 | 120 | - | 3 | 0 | 0 | 0 |
| 3 | 50 | - | 0 | 4 | 80 | 0 |
| 4 | 250 | 99 | 8 | 0 | 100 | 0 |
| 5 | 200 | - | 0 | 0 | 0 | 0 |
| 6 | 200 | - | 0 | 0 | 0 | 0 |
| 7 | 250 | 100* | 38 | 4 | 99 | 0 |
| 8 | 250 | 99* | 19 | 20 | 99 | 0 |
| 9 | 250 | 95 | 52 | 25 | 86 | 0 |
| 10 | 250 | 99* | 71 | 81 | 99 | 13 |
| 11 | 250 | 80* | 0 | 7 | 95 | 0 |
| 12 | 250 | 92* | 2 | 0 | 86 | 0 |
| 13 | 250 | 94 | 1 | 0 | 92 | 0 |
| 14 | 250 | 100 | 52 | 35 | 99 | 13 |
| 15 | 250 | 80* | 45 | 35 | 92 | 0 |
| 16 | 250 | 96 | - | 33 | 95 | 0 |
| 17 | 250 | 94 | - | 0 | 89 | 0 |
| 18 | 250 | 100 | - | 62 | 91 | 0 |
| 19 | 250 | 0 | - | 0 | 55 | 0 |
| 20 | 250 | 100 | - | 0 | 100 | 48 |
| 21 | 250 | 100 | - | 0 | 100 | 0 |
| 22 | 250 | 100 | - | 0 | 55 | 0 |
| 23 | 50 | 0 | - | - | 9 | - |
| 24 | 250 | 19 | - | 0 | 0 | 0 |
| 25 | 50 | 100 | - | - | 100 | - |
| 26 | 250 | 100 | - | 97 | 100 | 0 |
| 27 | 25 | 92 | - | - | - | - |
| 28 | 250 | 100 | 0 | 0 | 0 | 0 |
| 29 | 250 | - | 52 | 10 | 0 | 0 |
| 30 | 250 | 0 | 9 | 2 | 28 | 0 |
| 31 | 250 | - | 5 | 2 | 0 | 0 |
| 32 | 250 | 100 | - | 86 | 98 | 0 |
| 33 | 250 | 86 | - | 0 | 0 | 0 |
| 34 | 250 | 96 | - | 0 | 94 | 0 |
| 35 | 250 | 99 | - | 0 | 98 | 0 |
| 36 | 250 | 100 | - | 58 | 99 | 0 |
| 37 | 250 | 100 | - | 0 | 99 | 0 |
| 38 | 250 | 100 | - | 0 | 99 | 0 |
| 39 | 250 | 95 | - | 72 | 95 | 0 |
| 40 | 250 | 100 | - | 85 | 100 | 0 |
| 41 | 250 | 100 | - | 0 | 100 | 0 |
| 42 | 250 | 100 | - | 8 | 68 | 0 |
| 43 | 250 | 100* | 86 | 61 | 100 | 0 |
| 44 | 250 | 13 | - | 0 | 0 | 0 |
| 45 | 250 | 67 | - | 66 | 19 | 0 |
| 46 | 50 | 100 | - | - | 100 | - |
| 47 | 250 | 100 | - | 0 | 92 | 0 |
| 48 | 250 | 100 | - | 86 | 94 | 0 |
| 49 | 250 | - | 13 | 0 | 0 | 0 |
| 50 | 250 | - | 2 | 0 | 74 | 0 |
| 51 | 250 | - | 1 | 0 | 0 | 0 |
| 52 | 250 | 100 | 6 | 0 | 100 | 0 |
| 53 | 250 | 100* | 33 | 51 | 100 | 0 |
| 54 | 250 | 100* | 14 | 0 | 100 | 0 |
| 55 | 250 | 100 | - | 94 | 98 | 56 |
| 56 | 250 | 93 | - | 84 | 86 | 0 |
| 57 | 250 | - | 2 | 0 | 0 | 0 |
| 58 | 250 | - | 2 | 0 | 0 | 0 |
| 59 | 250 | 100 | 3 | 3 | 100 | 27 |
| 60 | 250 | 100 | 2 | 0 | 100 | 0 |
| 61 | 250 | 100* | 27 | 83 | 100 | 0 |
| 62 | 250 | 99 | 16 | 98 | 74 | 0 |
| 63 | 250 | 99 | 4 | 78 | 99 | 0 |
| 64 | 250 | - | 0 | 28 | 0 | 0 |
| 65 | 250 | - | 12 | 0 | 0 | 0 |
| 66 | 250 | 99 | - | 71 | 94 | 0 |
| 67 | 250 | - | 11 | 0 | 0 | 0 |
| 68 | 250 | 100 | 10 | 98 | 100 | 0 |
| 69 | 250 | - | 0 | 0 | 0 | 0 |
| 70 | 250 | 100 | 15 | 93 | 100 | 0 |
| 71 | 250 | 97 | 7 | 0 | 98 | 0 |
| 72 | 250 | 94* | 6 | 0 | 95 | 0 |
| 73 | 250 | 100* | 13 | 0 | 99 | 0 |
| 74 | 250 | 98* | 27 | 0 | 98 | 0 |
| 75 | 250 | 95* | 3 | 0 | 95 | 0 |
| 76 | 250 | 98* | 14 | 0 | 94 | 0 |
| 77 | 250 | - | 2 | 0 | 95 | 0 |
| 78 | 250 | 100 | - | 96 | 100 | 0 |
| 79 | 250 | 93 | - | 0 | 80 | 81 |
| 80 | 250 | 100 | - | 84 | 100 | 0 |
| 81 | 250 | 0 | 4 | 11 | 68 | 0 |
| 82 | 250 | 96 | 0 | 23 | 74 | 0 |
| 83 | 250 | - | 9 | 0 | 90 | 0 |
| 84 | 250 | - | - | - | 0 | 0 |
| 85 | 250 | 94* | 9 | 0 | 97 | 0 |
| 86 | 250 | 100* | - | 44 | 96 | 76 |
| 87 | 250 | 100 | - | 0 | 100 | 0 |
| 88 | 250 | 100 | - | 93 | 100 | 0 |
| 89 | 250 | 37 | - | 0 | 0 | 0 |
| 90 | 250 | 100 | - | 0 | 99 | 0 |
| 91 | 250 | 100 | - | 0 | 100 | 0 |
| 92 | 250 | 100 | - | 0 | 99 | 0 |
| 93 | 208 | 100* | 52 | 90 | 99 | 0 |
| 94 | 250 | 100 | - | 0 | 80 | 0 |
| 95 | 250 | 100 | - | 0 | 98 | 0 |
| 96 | 250 | 100 | - | 0 | 100 | 0 |
| 97 | 250 | - | - | 0 | 100 | 0 |
| 99 | 250 | 100 | | 0 | 100 | 0 |
| 100 | 250 | 100* | 31 | 42 | 99 | 0 |
| 101 | 250 | - | 23 | 56 | 84 | 0 |
| 102 | 250 | 100 | - | 0 | 100 | 0 |
| 103 | 250 | 100 | - | 96 | 100 | 21 |
| 104 | 250 | 100 | - | 0 | 99 | 0 |
| 105 | 212 | - | 60 | 89 | 68 | 0 |
| 106 | 250 | 100 | - | 0 | 95 | 0 |
| 107 | 250 | 100 | - | 0 | 91 | 0 |
| 108 | 210 | 100* | 31 | 0 | 100 | 0 |
| 109 | 250 | 100 | - | 0 | 100 | 0 |
| 110 | 250 | 100 | - | 0 | 55 | 0 |
| 111 | 250 | 100 | - | 0 | 100 | 81 |
| 112 | 250 | 100 | - | 86 | 84 | 0 |
| 113 | 250 | - | 0 | 0 | 0 | 0 |
| 114 | 250 | 100 | - | 21 | 100 | 96 |
| 115 | 250 | 100 | - | 52 | 100 | 0 |
| 116 | 250 | 96 | 14 | 0 | 95 | 0 |
| 117 | 250 | 100* | 5 | 57 | 100 | 0 |
| 118 | 250 | 100* | 14 | 53 | 100 | 0 |
| 119 | 250 | 100 | - | 0 | 100 | 0 |
| 120 | 250 | 100 | - | 0 | 99 | 0 |
| 121 | 250 | 100 | - | 85 | 99 | 0 |
| 122 | 250 | 100 | - | 0 | 99 | 0 |
| 123 | 250 | 100 | - | 0 | 96 | 0 |
| 124 | 250 | 100 | - | 0 | 100 | 0 |
| 125 | 250 | 100 | - | 0 | 68 | 0 |
| 126 | 250 | 100 | - | 0 | 100 | 0 |
| 127 | 250 | 100 | - | 0 | 100 | 0 |
| 128 | 250 | 100 | - | 0 | 100 | 0 |
| 129 | - | - | - | - | | - |
| 130 | 250 | 100* | 3 | 4 | 100 | 0 |
| 131 | 250 | 99* | 1 | 15 | 95 | 0 |
| 132 | 250 | - | - | - | | - |
| 134 | 25 | 100 | - | - | | - |
| 136 | 260 | 34 | - | 29 | 67 | 0 |
| 137 | 270 | 0 | - | 14 | 67 | 0 |
| 138 | 260 | 100 | - | 2 | 100 | 0 |
| 139 | 260 | 100 | - | 2 | 100 | 0 |
| 140 | 260 | 100 | - | 0 | 100 | 84 |
| 142 | 260 | 100 | - | 0 | 100 | 0 |
| 144 | 10 | 98 | - | - | 0 | - |
| 145 | 10 | 100 | - | - | 97 | - |
| 146 | 10 | 100 | - | - | 99 | - |
| 147 | 255 | 100 | - | 3 | 97 | 0 |
| 148 | 260 | 100 | - | 59 | 99 | 0 |
| 149 | 250 | 36 | - | 0 | 68 | 0 |
| 151 | 250 | 99 | - | 16 | 100 | 0 |
| 154 | 10 | 100 | - | - | 98 | - |
| 155 | 50 | 98 | - | - | 98 | - |
| 156 | 50 | 99 | - | - | 99 | - |
| 157 | 260 | 100 | - | 42 | 100 | 0 |
| 158 | 10 | 100 | - | - | 99 | - |
| 159 | 10 | 100 | - | - | 68 | - |
| 160 | 10 | 100 | - | - | 88 | - |
| 161 | 255 | 100 | - | 2 | 100 | 0 |
| 162 | 255 | 99 | - | 88 | 95 | 0 |
| 163 | 270 | 100 | - | 79 | 99 | 27 |
| 164 | 10 | 100 | - | - | 99 | - |
| 165 | 10 | 100 | - | - | 100 | - |
| 166 | 10 | 100 | - | - | 99 | - |
| 167 | 10 | 100 | - | - | 100 | - |
| 168 | 50 | 100 | - | - | 100 | - |
| 169 | 250 | 50 | - | 15 | 55 | 0 |
| 170 | 260 | 0 | - | 17 | 28 | 0 |
| 171 | 260 | 99 | - | 34 | 99 | 0 |
| 172 | 10 | 100 | - | - | 100 | - |
| 173 | 10 | 100 | - | - | 98 | - |
| 174 | 250 | 0 | - | 0 | 92 | 0 |
| 175 | 50 | 100 | - | - | 94 | - |
| 176 | 10 | 96 | - | - | 80 | - |
| 177 | 10 | 89 | - | - | 23 | - |
| 178 | 50 | 99 | - | - | 74 | - |
| 179 | 10 | 95 | - | - | 55 | - |
| 180 | 10 | 38 | - | - | 0 | - |
| 181 | 50 | 95 | - | - | 68 | - |
| 182 | 250 | 13 | - | 51 | 0 | 0 |
| 183 | 250 | 48 | - | 67 | 0 | 0 |
| 184 | 50 | 100 | - | - | 100 | - |
| 185 | 10 | 100 | - | - | 99 | - |
| 186 | 10 | 100 | - | - | 80 | - |
| 187 | 250 | 99 | - | 0 | 95 | 0 |
| 188 | 250 | 100 | - | 4 | 99 | 0 |
| 189 | 250 | 100 | - | 0 | 100 | 0 |
| 190 | 250 | 100 | - | 0 | 100 | 0 |
| 191 | 250 | 99 | - | 81 | 86 | 0 |
| 192 | 250 | 99 | - | 0 | 99 | 0 |
| 193 | 250 | 100 | - | 72 | 100 | 64 |
| 194 | 250 | 99 | - | 91 | 41 | - |
| 195 | 250 | 0 | - | 2 | 68 | 0 |
| 196 | 250 | - | 13 | 0 | 91 | 0 |
| 197 | 50 | 100 | - | - | 93 | - |
| 198 | 50 | 100 | - | - | 94 | - |
| 200 | 10 | 100 | - | - | 84 | - |
| 201 | 10 | 100 | - | - | 45 | - |
| 202 | 250 | 99 | - | 76 | 68 | 0 |
| 203 | 250 | 100 | - | 65 | 100 | |
| 204 | 10 | 100 | - | - | 83 | - |
| 205 | 10 | 100 | - | - | 92 | - |
| 207 | 10 | 100 | - | - | 95 | - |
| 208 | 10 | 100 | - | - | 95 | - |
| 209 | 10 | 100 | - | - | 84 | - |
| 210 | 10 | 100 | - | - | 86 | - |
| 211 | 10 | 100 | - | - | 55 | - |
| 212 | 265 | 11 | - | 0 | 67 | 0 |
| 213 | 270 | 59 | - | 30 | 55 | 0 |
| 214 | 265 | 100 | - | 96 | 100 | 0 |
| 215 | 255 | 13 | - | 0 | 0 | 0 |
| 216 | 10 | 100 | - | - | 100 | - |
| 217 | 10 | 100 | - | - | 100 | - |
| 218 | 10 | 100 | - | - | 99 | - |
| 219 | 10 | 0 | - | - | 0 | - |
| 220 | 250 | 100 | - | 97 | 100 | 0 |
| 221 | 10 | 100 | - | - | 83 | - |
| 222 | 10 | 100 | - | - | 99 | - |
| 223 | 50 | 0 | - | - | 0 | - |
| 224 | 50 | 0 | - | - | 0 | - |
| 225 | 10 | 94 | - | - | 68 | - |
| 226 | 10 | 99 | - | - | 99 | - |
| 227 | 50 | 100 | - | - | 91 | - |
| 228 | 10 | 100 | - | - | 99 | - |
| 229 | 50 | 100 | - | - | 95 | - |
| 230 | 10 | 100 | - | - | 99 | - |
| 231 | 50 | 100 | - | - | 100 | - |
| 232 | 50 | 100 | - | - | 94 | - |
| 233 | 10 | 100 | - | - | 100 | - |
| 234 | 10 | 100 | - | - | 99 | - |
| 235 | 50 | 100 | - | - | 86 | - |
| 236 | 50 | 100 | - | - | 94 | - |
| 237 | 10 | 100 | - | - | 99 | - |
| 238 | 10 | 100 | - | - | 100 | - |
| 239 | 10 | 100 | - | - | 91 | - |
| 240 | 10 | 100 | - | - | 68 | - |
| 241 | 10 | 100 | - | - | 96 | - |
| 242 | 10 | 100 | - | - | 86 | - |
| 243 | 50 | 100 | - | - | 98 | - |
| 244 | 10 | 100 | - | - | 97 | - |
| 245 | 250 | 100 | - | 100 | 100 | 0 |
| 246 | 10 | 100 | - | - | 55 | - |
| 247 | 10 | 100 | - | - | 74 | - |
| 248 | 10 | 100 | - | - | 55 | - |
| 249 | 10 | 98 | - | - | 41 | - |
| 250 | 10 | 99 | - | - | 68 | - |
| 251 | 10 | 92 | - | - | 68 | - |

## Claims

1. A compound of Formula 1: 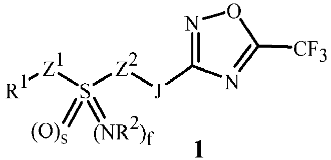
wherein R¹, Z¹, s, f, Z² and J are defined as follows, and wherein when J is a phenyl ring, the ring is connected to Z² and the oxadiazole ring in a 1,4-orientation:
| R¹ | Z¹ | s | f | Z² | J |
|---|---|---|---|---|---|
| 2-Cl-Ph | direct bond | 2 | 0 | NH | phenyl |
| Cl₂CH | direct bond | 1 | 0 | direct bond | phenyl |
| Me | direct bond | 2 | 0 | NH | phenyl |
| Ph | direct bond | 2 | 0 | NH | phenyl |
| Et | direct bond | 2 | 0 | CH₂ | phenyl |
| Me | direct bond | 2 | 0 | CH₂ | phenyl |
| (MeO)₂CHCH₂ | NH | 2 | 0 | direct bond | phenyl |
| Ph | direct bond | 2 | 0 | N(Me) | phenyl |
| CH₃C(=O) | N(Me) | 2 | 0 | direct bond | phenyl |
| 3,5-di-Cl-Ph | NH | 2 | 0 | direct bond | phenyl |
| CH₂=CHCH₂ | direct bond | 2 | 0 | direct bond | 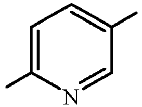 |
| Ph | direct bond | 2 | 0 | NH | 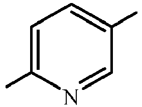 |
| 4-EtOC(=O)-Ph | NH | 2 | 0 | direct bond | phenyl |
| (Me)₂CHCH₂ | direct bond | 2 | 0 | direct bond | 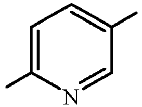 |
| 4-F-Ph | direct bond | 2 | 0 | NH | 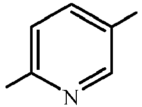 |
| 4-Me-Ph | direct bond | 2 | 0 | NH | 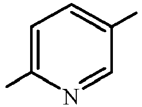 |
| 2-Cl-Ph | direct bond | 2 | 0 | NH | 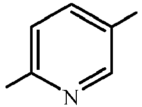 |
| 4-Cl-Ph | direct bond | 2 | 0 | NH | 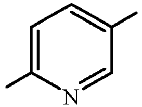 |
| 4-MeO-Ph | direct bond | 2 | 0 | NH | 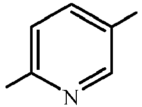 |
| CH₃ | direct bond | 2 | 0 | NHCH₂ | phenyl |
| Ph | direct bond | 2 | 0 | NHCH₂ | phenyl |
| 3-Cl-Ph | direct bond | 2 | 0 | NH | phenyl |
| 2-CF₃-Ph | direct bond | 2 | 0 | NH | phenyl |
| Me | direct bond | 2 | 0 | O | phenyl |
| CH₃C(=O) | N(OMe) | 2 | 0 | direct bond | phenyl |
| CH₃C(=O) | N(Et) | 2 | 0 | direct bond | phenyl |
| Me | direct bond | 2 | 0 | direct bond | phenyl |
| Me | direct bond | 1 | 0 | direct bond | phenyl |
wherein the bond of the pyridine J projecting to the left is bonded to Z², and the bond projecting to the right is bonded to the oxadiazole ring in Formula 1.

2. A compound of Claim 1 wherein R¹, Z¹, s, f, Z² and J are defined as follows, wherein J is connected to Z² and the oxadiazole ring in a 1,4-orientation:
| | | | | | |
|---|---|---|---|---|---|
| R¹ | Z¹ | s | f | Z² | J |
| Me | direct bond | 2 | 0 | NH | phenyl |
| Ph | direct bond | 2 | 0 | NH | phenyl |
| 2-Cl-Ph | direct bond | 2 | 0 | NH | phenyl |
| Et | direct bond | 2 | 0 | CH₂ | phenyl |
| Me | direct bond | 2 | 0 | CH₂ | Phenyl |

3. A fungicidal composition comprising (a) a compound of any of Claims 1-2; and (b) at least one other fungicide.

4. A fungicidal composition comprising (a) a compound of any of Claims 1-2; and (b) at least one additional component selected from the group consisting of surfactants, solid diluents and liquid diluents.

5. A method for controlling plant diseases caused by fungal plant pathogens comprising applying to the plant or portion thereof, or to the plant seed, a fungicidally effective amount of a compound of any of Claims 1-2.

6. A method for controlling plant diseases caused by Oomycete fungal plant pathogens comprising applying to the plant or portion thereof, or to the plant seed, a fungicidally effective amount of a compound of any of Claims 1-2.

## Patentansprüche

1. Verbindung der Formel 1: 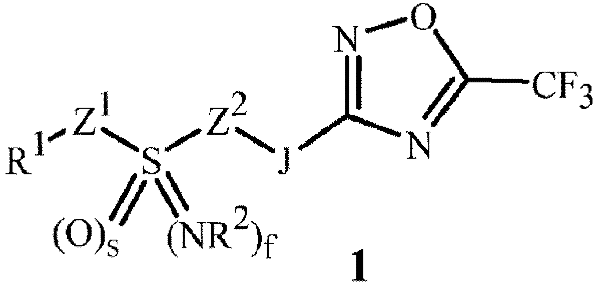
wobei R¹, Z¹, s, f, Z² und J wie folgt definiert sind und wobei J für einen Phenylring steht, der Ring an Z² gebunden ist und der Oxadiazolring in einer 1,4-Orientierung vorliegt:
| R¹ | Z¹ | s | f | Z² | J |
|---|---|---|---|---|---|
| 2-Cl-Ph | Direkte Bindung | 2 | 0 | NH | Phenyl |
| Cl₂CH | Direkte Bindung | 1 | 0 | Direkte Bindung | Phenyl |
| Me | Direkte Bindung | 2 | 0 | NH | Phenyl |
| Ph | Direkte Bindung | 2 | 0 | NH | Phenyl |
| Et | Direkte Bindung | 2 | 0 | CH₂ | Phenyl |
| Me | Direkte Bindung | 2 | 0 | CH₂ | Phenyl |
| (MeO)₂CHCH₂ | NH | 2 | 0 | Direkte Bindung | Phenyl |
| Ph | Direkte Bindung | 2 | 0 | N(Me) | Phenyl |
| CH₃C(=O) | N(Me) | 2 | 0 | Direkte Bindung | Phenyl |
| 3,5-di-Cl-Ph | NH | 2 | 0 | Direkte Bindung | Phenyl |
| CH₂=CHCH₂ | Direkte Bindung | 2 | 0 | Direkte Bindung | 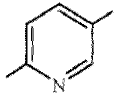 |
| Ph | Direkte Bindung | 2 | 0 | NH | 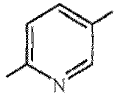 |
| 4-EtOC(=O)-Ph | NH | 2 | 0 | Direkte Bindung | Phenyl |
| (Me)₂CHCH₂ | Direkte Bindung | 2 | 0 | Direkte Bindung | 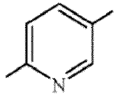 |
| 4-F-Ph | Direkte Bindung | 2 | 0 | NH | 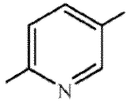 |
| 4-Me-Ph | Direkte Bindung | 2 | 0 | NH | 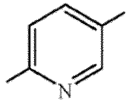 |
| 2-Cl-Ph | Direkte Bindung | 2 | 0 | NH | 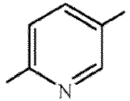 |
| 4-Cl-Ph | Direkte Bindung | 2 | 0 | NH | 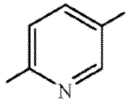 |
| 4-MeO-Ph | Direkte Bindung | 2 | 0 | NH | 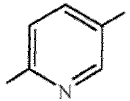 |
| CH₃ | Direkte Bindung | 2 | 0 | NHCH₂ | Phenyl |
| Ph | Direkte Bindung | 2 | 0 | NHCH₂ | Phenyl |
| 3-Cl-Ph | Direkte Bindung | 2 | 0 | NH | Phenyl |
| 2-CF₃-Ph | Direkte Bindung | 2 | 0 | NH | Phenyl |
| Me | Direkte Bindung | 2 | 0 | O | Phenyl |
| CH₃C(=O) | N(OMe) | 2 | 0 | Direkte Bindung | Phenyl |
| CH₃C(=O) | N(Et) | 2 | 0 | Direkte Bindung | Phenyl |
| Me | Direkte Bindung | 2 | 0 | Direkte Bindung | Phenyl |
| Me | Direkte Bindung | 1 | 0 | Direkte Bindung | Phenyl |
wobei die Bindung des Pyridins J, die nach links zeigt, an Z² gebunden ist und die Bindung, die nach rechts zeigt, an den Oxadiazolring in Formel 1 gebunden ist.

2. Verbindung nach Anspruch 1, wobei R¹, Z¹, s, f, Z² und J wie folgt definiert sind, wobei J an Z² gebunden ist und der Oxadiazolring in einer 1,4-Orientierung vorliegt:
| R¹ | Z¹ | s | f | Z² | J |
|---|---|---|---|---|---|
| Me | Direkte Bindung | 2 | 0 | NH | Phenyl |
| Ph | Direkte Bindung | 2 | 0 | NH | Phenyl |
| 2-Cl-Ph | Direkte Bindung | 2 | 0 | NH | Phenyl |
| Et | Direkte Bindung | 2 | 0 | CH₂ | Phenyl |
| Me | Direkte Bindung | 2 | 0 | CH₂ | Phenyl |

3. Fungizide Zusammensetzung, umfassend (a) eine Verbindung nach Anspruch 1 oder 2 und (b) mindestens ein anderes Fungizid.

4. Fungizide Zusammensetzung, umfassend (a) eine Verbindung nach Anspruch 1 oder 2 und (b) mindestens eine zusätzliche Komponente ausgewählt aus der aus Tensiden, festen Verdünnungsmitteln und flüssigen Verdünnungsmitteln bestehenden Gruppe.

5. Verfahren zur Bekämpfung von durch pilzliche Pflanzenpathogene verursachte Pflanzenkrankheiten, umfassend die Anwendung einer fungizid wirksamen Menge einer Verbindung nach Anspruch 1 oder 2 auf die Pflanze oder einen Teil davon oder auf das Pflanzensaatgut.

6. Verfahren zur Bekämpfung von durch pilzliche Pflanzenpathogene aus der Klasse der Oomyceten verursachte Pflanzenkrankheiten, umfassend die Anwendung einer fungizid wirksamen Menge einer Verbindung nach Anspruch 1 oder 2 auf die Pflanze oder einen Teil davon oder auf das Pflanzensaatgut.

## Revendications

1. Composé de formule I : 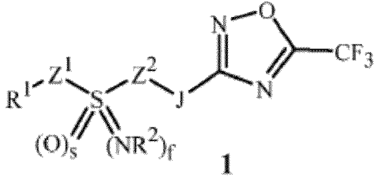
R¹, Z¹, s, f, Z² et J étant définis comme suit, et, lorsque J est un cycle phényle, le cycle est relié à Z² et au cycle oxadiazole en une orientation 1,4 :
| R¹ | Z¹ | s | f | Z² | J |
|---|---|---|---|---|---|
| 2-Cl-Ph | liaison directe | 2 | 0 | NH | phényle |
| Cl₂CH | liaison directe | 1 | 0 | liaison directe | phényle |
| Me | liaison directe | 2 | 0 | NH | phényle |
| Ph | liaison directe | 2 | 0 | NH | phényle |
| Et | liaison directe | 2 | 0 | CH₂ | phényle |
| Me | liaison directe | 2 | 0 | CH₂ | phényle |
| (MeO)₂CHCH₂ | NH | 2 | 0 | liaison directe | phényle |
| Ph | liaison directe | 2 | 0 | N(Me) | phényle |
| CH₃C(=O) | N(Me) | 2 | 0 | liaison directe | phényle |
| 3,5-di-Cl-Ph | NH | 2 | 0 | liaison directe | phényle |
| CH₂=CHCH₂ | liaison directe | 2 | 0 | liaison directe | 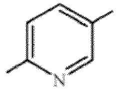 |
| Ph | liaison directe | 2 | 0 | NH | 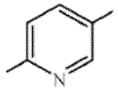 |
| 4-EtOC(=O)-Ph | NH | 2 | 0 | liaison directe | phényle |
| (Me)₂CHCH₂ | liaison directe | 2 | 0 | liaison directe | 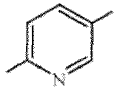 |
| 4-F-Ph | liaison directe | 2 | 0 | NH | 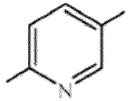 |
| 4-Me-Ph | liaison directe | 2 | 0 | NH | 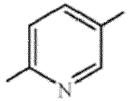 |
| 2- Cl-Ph | liaison directe | 2 | 0 | NH | 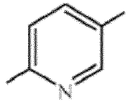 |
| 4-Cl-Ph | liaison directe | 2 | 0 | NH | 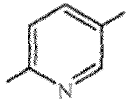 |
| 4-MeO-Ph | liaison directe | 2 | 0 | NH | 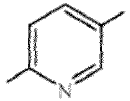 |
| CH₃ | liaison directe | 2 | 0 | NHCH₂ | phényle |
| Ph | liaison directe | 2 | 0 | NHCH₂ | phényle |
| 3-Cl-Ph | liaison directe | 2 | 0 | NH | phényle |
| 2-CF₃-Ph | liaison directe | 2 | 0 | NH | phényle |
| Me | liaison directe | 2 | 0 | O | phényle |
| CH₃C(=O) | N(OMe) | 2 | 0 | liaison directe | phényle |
| CH₃C(=O) | N(Et) | 2 | 0 | liaison directe | phényle |
| Me | liaison directe | 2 | 0 | liaison directe | phényle |
| Me | liaison directe | 1 | 0 | liaison directe | phényle |
la liaison de la pyridine J projetant vers la gauche étant liée à Z², et la liaison projetant vers la droite étant liée au cycle oxadiazole dans la formule 1.

2. Composé selon la revendication 1, R¹, Z¹, s, f, Z² et J étant définis comme suit, J étant connecté au cycle oxadiazole en une orientation 1,4 :
| R¹ | Z¹ | s | f | Z² | J |
|---|---|---|---|---|---|
| Me | liaison directe | 2 | 0 | NIl | phényle |
| Ph | liaison directe | 2 | 0 | NH | phényle |
| 2-Cl-Ph | liaison directe | 2 | 0 | NH | phényle |
| Et | liaison directe | 2 | 0 | CH₂ | phényle |
| Me | liaison directe | 2 | 0 | CH₂ | phényle |

3. Composition fongicide comprenant (a) un composé selon l'une quelconque des revendications 1 et 2 ; et (b) au moins un autre fongicide.

4. Composition fongicide comprenant (a) un composé selon l'une quelconque des revendications 1 et 2 ; et (b) au moins un composant supplémentaire choisi dans le groupe constitué par des tensioactifs, des diluants solides et des diluants liquides.

5. Procédé pour la lutte contre des maladies de végétaux causées par des pathogènes fongiques de végétaux comprenant une application au végétal ou à une partie correspondante, ou à la graine de végétal, d'une quantité efficace sur le plan fongicide d'un composé selon l'une quelconque des revendications 1 et 2.

6. Procédé pour la lutte contre des maladies de végétaux causées par des pathogènes fongiques de végétaux de type oomycètes comprenant une application au végétal ou à une partie correspondante, ou à la graine de végétal, d'une quantité efficace sur le plan fongicide d'un composé selon l'une quelconque des revendications 1 et 2.
